(19) Europäisches Patentamt
European Patent Office
Office européen des brevets

(11) Veröffentlichungsnummer : **0 517 065 A1**

(12) **EUROPÄISCHE PATENTANMELDUNG**

(21) Anmeldenummer : **92108792.0**

(22) Anmeldetag : **25.05.92**

(51) Int. Cl.[5] : **C07D 477/00,** C07D 491/14, C07D 495/14, C07D 471/14, // A61K31/40 , (C07D491/14, 311:00, 209:00, 205:00), (C07D495/14, 335:00, 209:00, 205:00), (C07D471/14, 221:00, 209:00, 205:00)

(30) Priorität : **29.05.91 DE 4117564**
**13.08.91 DE 4126653**

(43) Veröffentlichungstag der Anmeldung :
**09.12.92 Patentblatt 92/50**

(84) Benannte Vertragsstaaten :
**AT BE CH DE DK ES FR GB GR IT LI LU NL PT SE**

(71) Anmelder : **HOECHST AKTIENGESELLSCHAFT**
**Postfach 80 03 20**
**W-6230 Frankfurt am Main 80 (DE)**

(72) Erfinder : **Gerlach, Uwe, Dr.**
**Emmerich-Josef-Strasse 59**
**W-6230 Frankfurt am Main 80 (DE)**
Erfinder : **Hörlein, Rolf, Dr.**
**Assmannshäuser Weg 21**
**W-6000 Frankfurt am Main 71 (DE)**
Erfinder : **Krass, Norbert, Dr.**
**Georg-Speyer-Strasse 60**
**W-6000 Frankfurt am Main 90 (DE)**
Erfinder : **Lattrell, Rudolf, Dr.**
**Heuhohlweg 6h**
**W-6240 Königstein/Taunus (DE)**
Erfinder : **Wollmann, Theodor, Dr.**
**Gartenstrasse 25**
**W-6238 Hofheim am Taunus (DE)**
Erfinder : **Limbert, Michael, Dr.**
**Am alten Birnbaum 21**
**W-6238 Hofheim am Taunus (DE)**
Erfinder : **Markus, Astrid, Dr.**
**Sulzbacher Strasse 6**
**W-6237 Liederbach (DE)**

(54) **Tetrazyklische beta-Lactam Antibiotika und Verfahren zur ihrer Darstellung.**

(57) β-Lactam-Antibiotika der Formel I und deren pharmazeutisch verträgliche Salze,

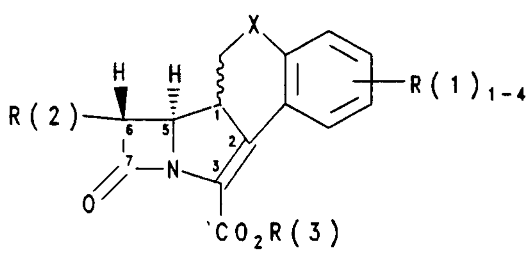

I

mit
X gleich $(CH_2)_{o-2}$, CR(a)R(b), O, $SO_{o-2}$, NR(c)
R(1), R(2) und R(3) einer Vielzahl von Substituenten sind hervorragende Antibiotika mit bemerkenswert guter antibakterieller Wirksamkeit sowohl gegen Gram-positive als auch gegen Gran-nevative Keime. Sie weisen gegenüber der renalen Dehydropeptidase eine hohe Stabilität auf.
Sie werden durch Zyklisierung der Verbindungen II

EP 0 517 065 A1

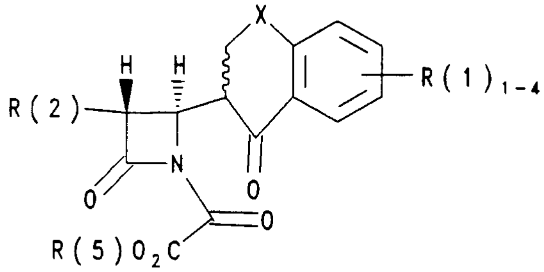

II

oder III

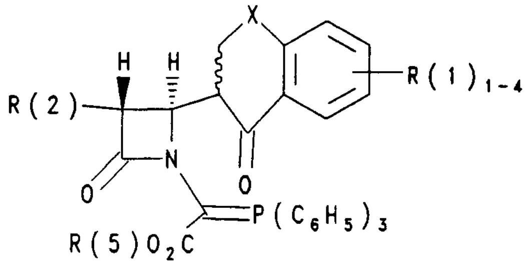

III

erhalten.

Die Erfindung betritt β-Lactamderivate mit einer tetrazyklischen Grundstruktur, die eine sehr hohe antimikrobielle Wirksamkeit gegen Gram-positive und Gram-negative Bakterien besitzen und deshalb als Arzneimittel zur Behandlung von mikrobiellen Infektionen geeignet sind sowie Verfahren zu ihrer Herstellung.

β-Lactame, wie z. B. Penicilline, Cephalosporine und Carbapeneme, stellen wertvolle Therapeutika zur Behandlung bakterieller Infektionen dar. Ein Nachteil der meisten bekannten Antibiotika ist, daß sie nicht gegen alle Erreger wirksam sind. Zudem führt ihr häufiger Einsatz zum Auftreten von Resistenzen. Dies macht die Suche nach neuen, hochwirksamen β-Lactamstrukturen notwendig.

Die Erfindung betritt daher eine neue Klasse von β-Lactam-Antibiotika der Formel I, deren Herstellung und pharmazeutisch verträglichen Salze,

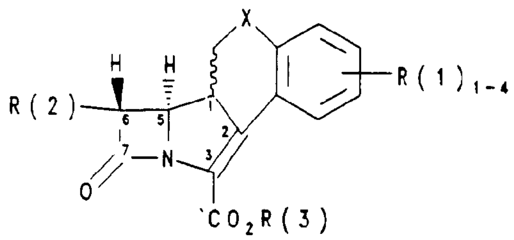

I

worin bedeuten:

X: $(CH_2)_n$ mit n = 0, 1, 2;

CR(a)R(b), wobei R(a) und R(b) voneinander unabhängig aus den folgenden Gruppen ausgewählt werden können: H; $(C_1$-$C_6)$-Alkyl; Aryl und Heteroaryl; [Aryl bedeutet z. B. unsubstituiertes und durch $(C_1$-$C_4)$-Alkyl, F, Cl, Br, $O(C_1$-$C_4)$-Alkyl, OH, $OCO(C_1$-$C_4)$-Alkyl, $NH_2$, $NH(C_1$-$C_4)$-Alkyl, $OCOC_6H_5$, $NHC_6H_5$ substituiertes Phenyl und Naphthyl, Heteroaryl steht für einen unsubstituierten oder durch $(C_1$-$C_4)$-Alkyl, F, Cl, Br, $O(C_1$-$C_4)$-Alkyl, OH, $OCO(C_1$-$C_4)$-Alkyl, $NH_2$, $NH(C_1$-$C_4)$-Alkyl, $OCOC_6H_5$, $NHC_6H_5$ substituierten 5- bis 6-gliedrigen Ring mit 1 bis 4 Heteroatomen (z. B. N, O, S) wie z. B. Furan, Pyrrol, Thiophen, Thiazol, Isothiazol, Oxazol, Isooxazol, Pyrazol, Imidazol, Thiadiazol, Triazol, Tetrazol, Pyridin, Pyrimidin, Pyridazin];

O;

$SO_n$ mit n = 0, 1, 2;

NR(c), wobei R(c) aus der Gruppe bestehend aus H, $(C_1$-$C_6)$-Alkyl, Aryl, $CO(C_1$-$C_6)$-Alkyl, CO-Aryl, CO-Heteroaryl, $(C_1$-$C_6)$-Alkoxycarbonyl, $(C_1$-$C_6)$-Alkylsulfonyl und Arylsulfonyl ausgewählt ist,

R(1): bis zu vier Substituenten, die gleich oder verschieden sind, ausgewählt aus der Gruppe, bestehend aus H, $(C_1$-$C_6)$-Alkyl,

ein Substituent aus der Gruppe bestehend aus Aryl, Heteroaryl, OH, SH, $SO_n(C_1$-$C_6)$-Alkyl (mit n = 0, 1, 2), NR(b)R(c), (wobei R(b) und R(c) wie oben definiert sind), CN, $NO_2$, C(R(a)) = NOR(b), (wobei R(a) und R(b) wie oben definiert sind),

bis zu zwei Substituenten aus der Gruppe $CF_3$, F, Cl, Br, I, $O(C_1$-$C_6)$-Alkyl, $OCO(C_1$-$C_6)$-Alkyl, OCONR(d)R(e), (wobei R(d) und R(e) voneinander unabhängig aus den folgenden Gruppen ausgewählt werden: Wasserstoff, $(C_1$-$C_6)$-Alkyl, NR(d)R(e) kann auch einem 5- oder 6-gliedrigen Ringsystem entsprechen), $SO_2N$ R(d) R(e), (wobei R(d) und R(e) wie oben definiert sind),

$CO(C_1$-$C_6)$-Alkyl, COAryl, $CO_2H$, $CO_2(C_1$-$C_6)$-Alkyl, CONR(d)R(e), (wobei R(d) und R(e) wie oben definiert sind), $CH_2R(f)$, (wobei R(f) aus den folgenden Gruppen ausgewählt ist: Hydroxy, $(C_1$-$C_6)$-Alkoxy, Acyloxy, Aryloxy, Heteroaryloxy, $(C_1$-$C_6)$-Alkylthio, Arylthio, Heteroarylthio und die daraus ableitbaren Sulfinyl- und Sulfonylverbindungen, NR(b)R(c), wobei R(b) und R(c) wie oben definiert sind. NR(b)R(c) kann zusätzlich Teil eines zyklischen und heterozyklischen Systems sein);

$NHCO(C_1$-$C_6)$-Alkyl; $NHCOC_6H_5$, NHCO-Naphthyl;

R(2): H, $(C_1$-$C_4)$-Alkyl, $CH_2OH$, $CH_2OCOR(a)$, $CH(OH)CH_3$, $CH(OCOR(a))CH_3$, $CH_2NR(b)R(c)$, $CH(NR(b)R(c))CH_3$, $C(CH_3)$ = NR(a), $CH[^{\oplus}NR(g)R(h)R(i)]CH_3$, wobei R(a), R(b) und R(c) wie unter R(1) definiert sind und R(g), R(h) und R(i) voneinander unabhängig sind und $(C_1$-$C_6)$-Alkylgruppen darstellen; $NH_2$; NHR(a)R(b); $NHCO(C_1$-$C_6)$-Alkyl; $NHCOC_6H_5$, NHCO-Napthyl;

R(3): H, $(C_1$-$C_3)$-Alkyl-$OCO(C_1$-$C_6)$-Alkyl, $(C_1$-$C_3)$-Alkyl-$OCO_2$ $(C_1$-$C_6)$-Alkyl, (5-Methyl-1,3-dioxolen-2-on-4-yl)-methyl,

und in denen die bevorzugte Stereochemie in Position 5 gleich R und in Position 6 gleich S ist.

Bevorzugt sind Verbindungen I, in denen R(1) nicht viermal Wasserstoff ist.

Bevorzugt sind Verbindungen I, in denen die Substituenten die folgenden Bedeutungen haben:

X: $CH_2$; $C(CH_3)_2$; CH-Phenyl; O; $SO_n$ mit n = O, 1, 2; $NSO_2CH_3$; $NSO_2$-$C_6H_4$-$CH_3$; wovon $CH_2$ und $SO_n$ mit n = 0, 1, 2 besonders bevorzugt sind.

R(1): H, $CH_3$, $C_6H_5$,

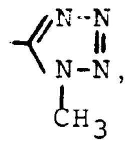

F, Cl, Br, $OCH_3$, OH, $OCOCH_3$, OCONH-$C_6H_5$, $NH_2$, $NHCOCH_3$, $NHSO_2CH_3$, $NHSO_2$-$C_6H_4$-$CH_3$, $NHCOCH_2NH_2$, CN, $COCH_3$, $C(CH_3) = NOH$, $CO_2H$, $CO_2CH_3$, $CONH_2$, $CON(CH_3)_2$,

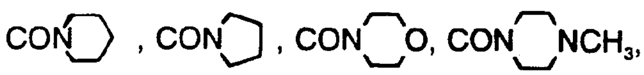

$CONHCH_2CO_2H$, $CH_2OCOCH_3$, $CH_2SCH_2CH_3$, $CH_2SCH_2CH_2NH_2$, $CH_2SCH_2CH_2NHCH(=NH)$, $CH_2SC_6H_5$,

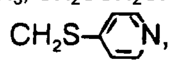

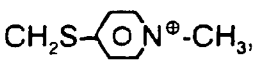

$CH_2N(CH_3)_2$, $CH_2NHCOCH_2NH_2$),

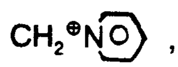

$CH_2OH$,

R(2): H, $CH_3$, $CH_2CH_3$, $CH_2OH$, CH $(OH)CH_3$, $CH(OCOCH_3)CH_3$, $CH(OCOCH_2$-$C_6H_5)CH_3$, CH-$(OCOCH_2O$-$C_6H_5)CH_3$, $CH(NH_2)CH_3$, $CH[N^{\oplus}(CH_3)_3]CH_3$, $CH(NHCOCH_3)CH_3$, $CH(NHSO_2CH_3)CH_3$, CH-$(NHSO_2C_6H_5)CH_3$, $CH(NHCOCH_2NH_2)CH_3$, wovon $CH(OH)CH_3$ mit der R-Konfiguration besonders bevorzugt ist,

R(3): H, $CH_2OCOCH_3$, $CH_2OCOCH_2CH_3$, $CH_2OCOCH_2CH_2CH_3$, $CH_2OCOCH\ (CH_3)_2$, $CH_2OCOC(CH_3)_3$, $CH_2OCOC(CH_3)_2CH_2CH_3$, $CH(CH_3)OCOCH_3$, $CH(CH_2CH_3)OCOCH_3$, $CH(CH_3)OCOC(CH_3)_3$, $CH(CH_3)OCO_2$ $CH_3$, $CH(CH_3)OCO_2CH_2CH_3$, $CH(CH_3)OCO_2CH\ (CH_3)_2$, $CH_2OCO_2CH_3$.

Besonders bevorzugt sind Verbindungen, in denen die $CH_2X$-Gruppe β-ständig ist, d. h. trans-ständig zu dem C(5) Wasserstoff.

Die Erfindung umfaßt weiterhin Verfahren zur Herstellung von Verbindungen der Formel I und ihrer pharmazeutisch verträglichen Salze, die dadurch gekennzeichnet sind, daß man

a) eine Verbindung der Formel II herstellt, wobei R(1), R(2) und X wie oben definiert sind, falls erforderlich NH- und OH-Gruppen mit Schutzgruppen substituiert sind, und R(5) eine Carboxyschutzgruppe darstellt,

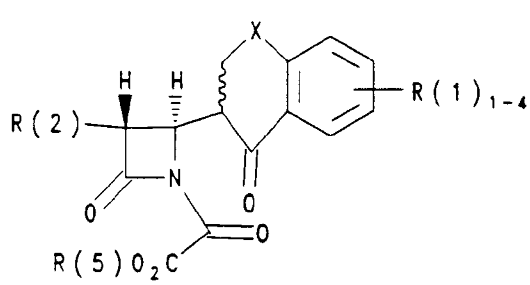

II

die Verbindungen der Formel II mit Alkanphosphonigsäureestern oder Trialkylphosphiten zyklisiert,
die Schutzgruppen abspaltet und,
falls erforderlich, die erhaltenen Produkte in pharmazeutisch verträgliche Salze überführt,
oder daß man

b) eine Verbindung der Formel III herstellt, wobei R(1), R(2) und X wie oben definiert sind, falls erforderlich NH- und OH-Gruppen mit Schutzgruppen substituiert sind, und R(5) eine Carboxyschutzgruppe oder eine der unter R(3) aufgeführten Gruppen darstellt,

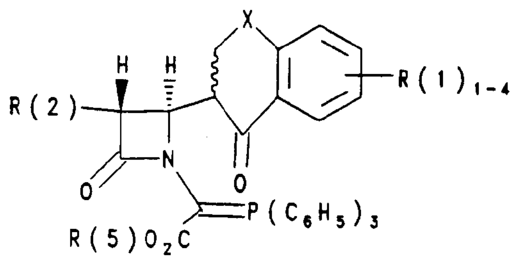

III

die Verbindungen der Formel III durch Erhitzen zyklisiert,

falls erforderlich, die Schutzgruppen abspaltet und,

falls erforderlich, die erhaltenen Produkte I in pharmazeutisch verträgliche Salze überführt,

oder daß man

c) die nach Verfahrensvariante a) und b) erhaltenen Verbindungen der Formel I, in der R(3) für ein Wasserstoff steht, in die Ester mit den unter R(3) angegebenen Gruppen überführt.

Die Herstellung von Verbindungen der Formel I nach dem Verfahren a) ist im Schema I dargestellt.

Schema I

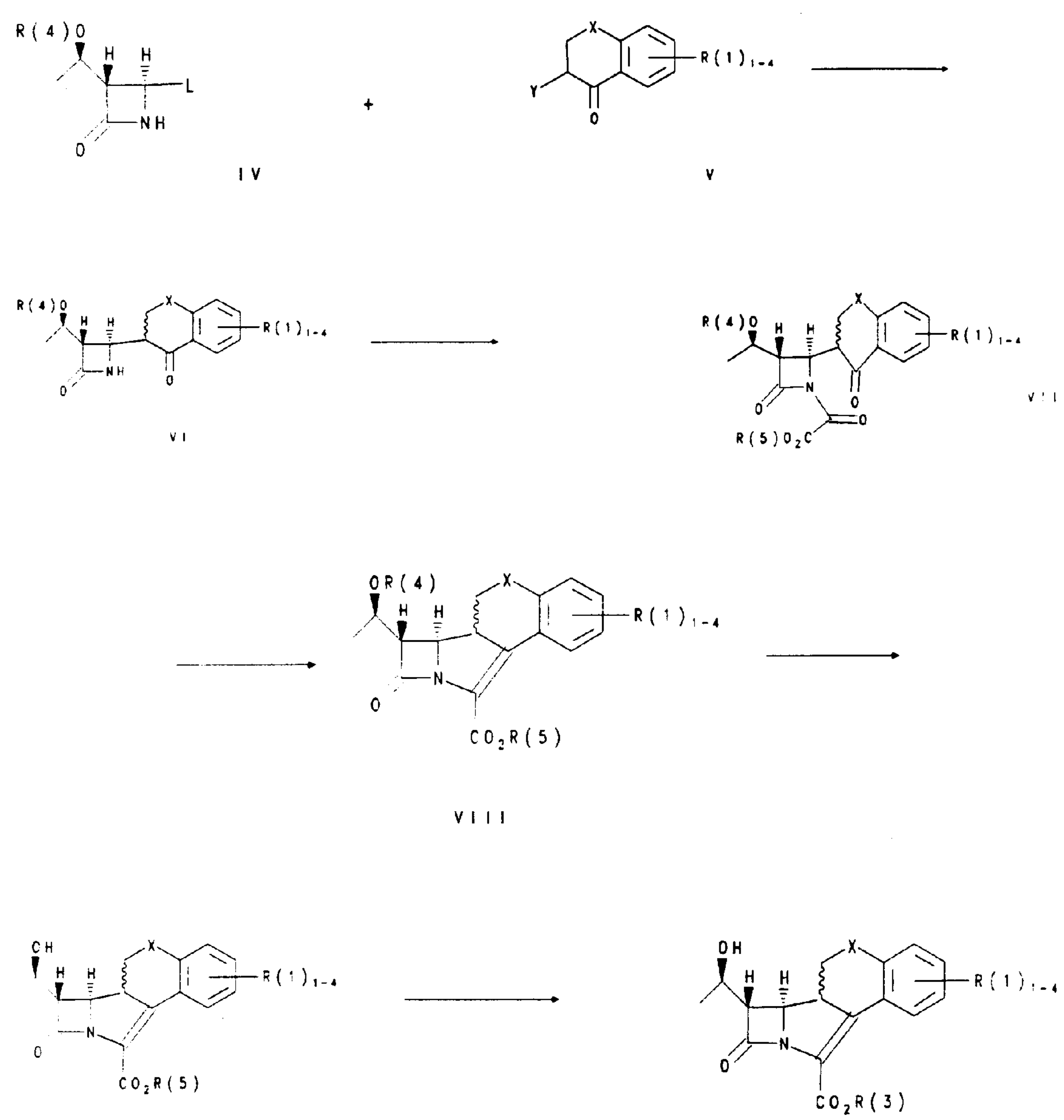

IV

V

VI

VII

VIII

IX

X

Ausgehend von Verbindungen der Formel IV und V stellt man zunächst Verbindungen der Formel VI dar. Dabei sind X und R(1) wie oben definiert, L stellt eine Abgangsgruppe, z. B. -Cl oder $-OCOCH_3$ dar. R(4) steht für eine Alkoholschutzgruppe wie die Trimethylsilylgruppe und die Tetrahydropyranylgruppe, die z.B. durch Säurehydrolyse gespalten werden können, oder die tert. Butyldimethylsilylgruppe und die Triethylsilylgruppe, die z. B. durch Tetrabutylammoniumfluorid gespalten werden können, oder die Benzyloxycarbonylgruppe und die 4-Nitrobenzyloxycarbonylgruppe, die hydrogenolytisch gespalten werden können, oder auch die Allyloxycarbonylgruppe, die durch $Pd[P(C_6H_5)_3]_4$ gespalten werden kann. Y kann Wasserstoff oder auch Brom bedeuten.

Die Umsetzungen werden durchgeführt, indem man Verbindungen der Formel V analog zu Literaturverfahren in die Metallenolate überführt, wobei unter anderem die folgenden Metalle in den angegebenen Oxidationsstufen eingesetzt werden können: Li(+1), Na(+1), K(+1), Mg(+2), B(+3), Si(+4), Sn(+2), Zn(+2), Ti(+4). Die Metallenolate können dann mit Verbindungen der Formel IV umgesetzt werden oder auch in deren Gegenwart erzeugt werden. Gegebenenfalls müssen Lewis-Säuren wie z. B. Trimethylsilyltriflat, Zink(II)chlorid oder auch Titan(IV)chlorid der Reaktion zugesetzt werden. Es werden Reaktionsbedingungen gewählt, wie sie in der Literatur beschrieben wurden, z. B. US Patentschrift 4.841.043, US Patentschrift 4.772.683, Deutsche Offenlegungsschrift DE 3509 769 A1 und Deziel et al, Tetrahedron Letters 30 (11) (1989), 1345 - 1348.

Die Verbindungen der Formel VI können als Mischungen des α- und β-Isomeren entstehen. Je nach Bedingungen und benutztem Ausgangsmaterial liegt das Verhältnis α/β zwischen 9/1 und 1/9. Die Diastereomeren können durch Chromatographie oder auch Kristallisation getrennt werden. Es können aber auch Mischungen weiter umgesetzt und auf späteren Stufen getrennt werden.

Die Acylierung zu Verbindungen der Formel VII erfolgt analog zu Literaturverfahren in an sich bekannter Weise. R(5) stellt eine Carboxyschutzgruppe wie z. B. die Benzylgruppe, die 4-Nitrobenzylgruppe oder auch die Allyl- bzw. die 2-Chlorallylgruppe dar. Diese können später hydrogenolytisch oder mit $Pd[P(C_6H_5)_3]_4$ gespalten werden.

Die Zyklisierung zu Verbindungen der Formel VIII erfolgt mit 2 - 10 Äquivalenten Alkanphosphonigsäureestern oder Trialkylphosphiten, bevorzugt mit 3 - 5 Äquivalenten Methanphosphonigsäuredimethylester oder Methanphosphonigsäurediethylester. Die Reaktion wird ausgeführt bei Temperaturen von 60°C bis 200°C, vorzugsweise von 110°C bis 170°C, in einem inerten aprotischen Lösungsmittel. Geeignete Lösungsmittel sind z. B. Toluol, Xylol oder auch Mesitylen. Die Reaktionsdauer hängt von den Reaktanden, der Temperatur und dem Lösungsmittel ab und liegt zwischen 5 Minuten und 48 Stunden. Die Produkte werden nach dem Entfernen des Lösungsmittels durch Chromatographie oder Kristallisation gereinigt.

Die Abspaltung der Schutzgruppen zu Verbindungen der Formel IX und weiter zu Verbindungen der Formel X, wobei R(3) Wasserstoff bedeutet, ist abhängig von den gewählten Schutzgruppen und erfolgt, wie weiter oben beschrieben in Analogie zu allgemeinen Literaturmethoden. Die Verbindungen der Formel X können durch Chromatographie an RP-18-Kieselgel oder durch Kristallisation gereinigt werden.

Schema II

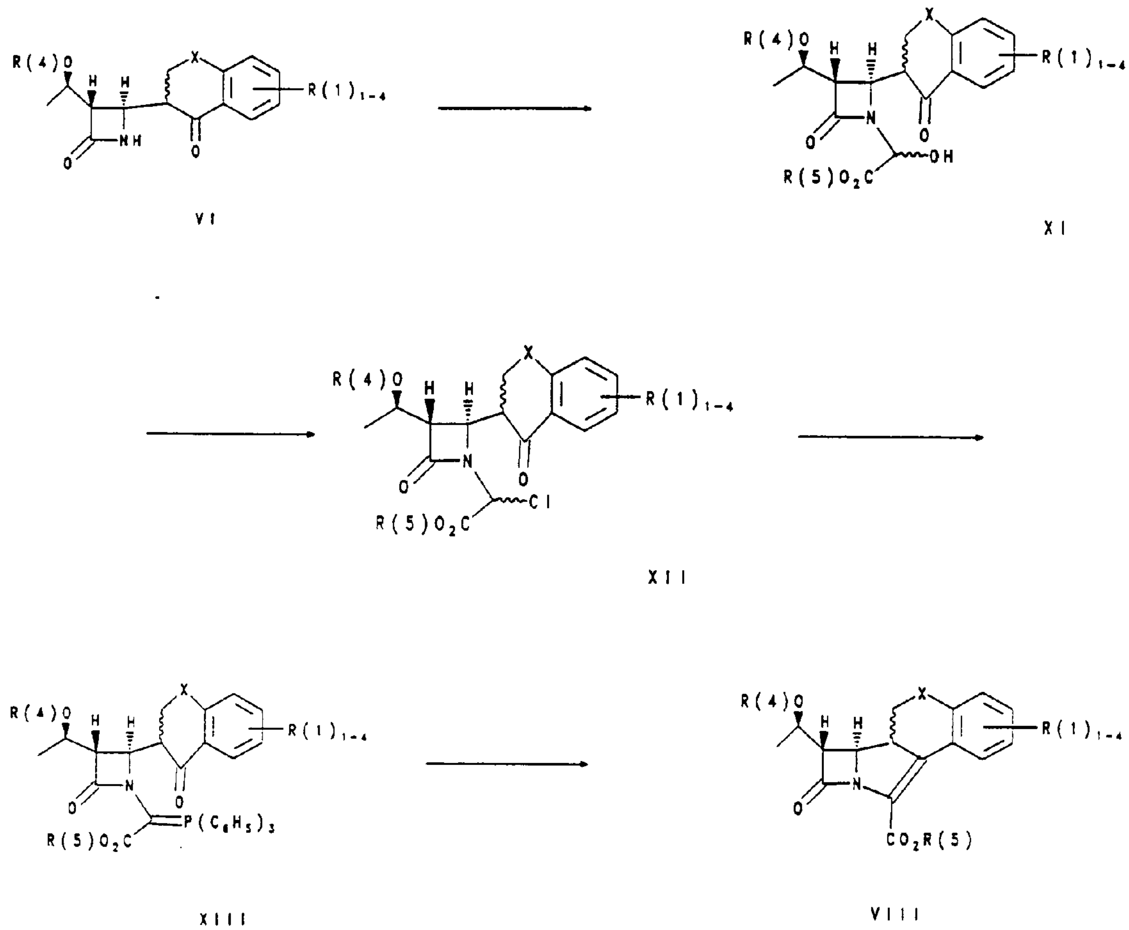

VI XI XII XIII VIII

Ebenso sind die Verbindungen der Formel I nach dem Verfahren b), das in Schema II dargestellt ist, zugänglich.

Die Herstellung von Verbindungen der Formel XIII erfolgt ausgehend von Verbindungen der Formel VI analog zu beschriebenen Literaturmethoden, wie R. N. Guthikonda et al., J. Med. Chem. 30 (1987), 871 - 880.

Die Zyklisierung zu Verbindungen der Formel XIII erfolgt in inerten aprotischen Lösungsmitteln, wie z. B. Toluol, Xylol oder Mesitylen. Die Reaktion wird bei Temperaturen von 60°C bis 200°C, vorzugsweise von 110°C bis 170°C, durchgeführt. Die Reaktionsdauer hängt von den Reaktanden, der Temperatur und dem Lösungsmittel ab und liegt zwischen 15 Minuten und 48 Stunden. Die Produkte werden nach dem Entfernen des Lösungsmittels durch Chromatographie und Kristallisation gereinigt und dann werden, wie bei Verfahren a) beschrieben, die Schutzgruppen entfernt und, falls erforderlich, pharmazeutisch verträgliche Salze hergestellt.

R(5) kann in diesem Fall auch eine der unter R(3) aufgeführten Esterkomponenten sein, so daß man nach Abspaltung der Alkoholschutzgruppe direkt zu in vivo spaltbaren Estern der Formel I kommt (R(3) nicht gleich H).

Die Herstellung von Verbindungen mit der Formel XV, wobei R(6) für Alkyl, Aryl oder Heteroaryl steht, kann durch Acylierung von Verbindungen der Formel IX nach Literaturvorschriften und anschließender Abspaltung der Carboxyschutzgruppe erfolgen.

Schema III

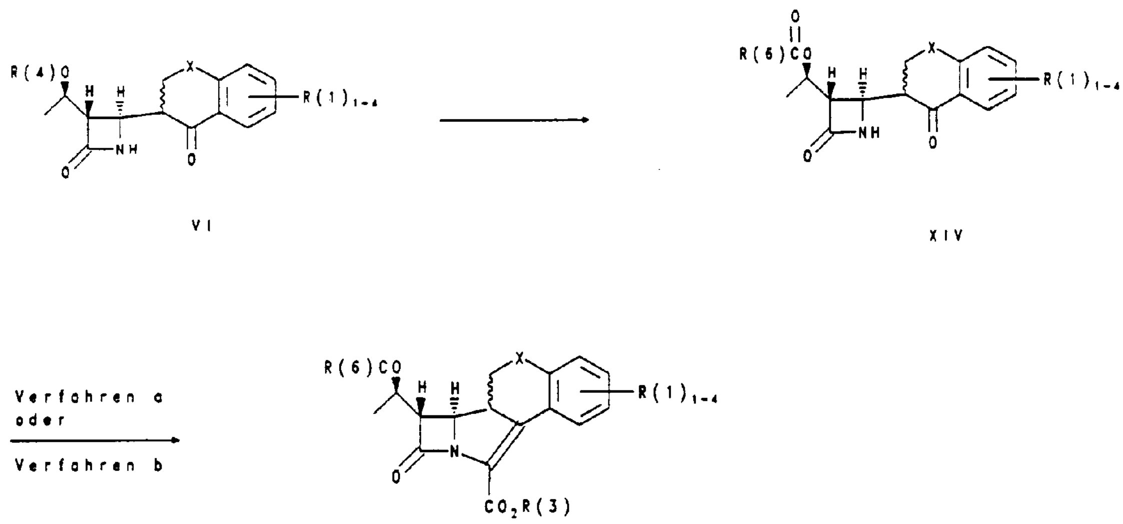


VI

XIV

XV

Weiterhin können, wie in Schema III dargestellt, die Verbindungen der Formel VI, wobei R(4) für die Trimethylsilylgruppe und die tert.-Butyldimethylsilylgruppe steht, nach an sich bekannten Verfahren mit Lewis-Säuren wie Eisen(III)chlorid und Säureanhydriden in Verbindungen der Formel XIV umgewandelt werden und diese anschließend nach Verfahren a) oder b) in das Produkt mit der Formel XV überführt werden.

Die Aminoethylderivate der Formel XVIII in Schema IV, wobei R(7) eine Aminoschutzgruppe, wie z. B. Allyloxycarbonyl darstellt, werden wie folgt erhalten: Ausgehend von den oben beschriebenen Verbindungen der Formel VI stellt man den Alkohol der Formel XVI her. Wenn z. B. R(4) eine tert.-Butyldimethylsilylgruppe darstellt, kommt man zu Verbindungen der Formel XVI durch Umsetzungen mit Mineralsäuren, wie z. B. Salzsäure in Methanol, oder Lewis-Säuren wie z. B. Bortrifluorid in Acetonitril.

Schema IV

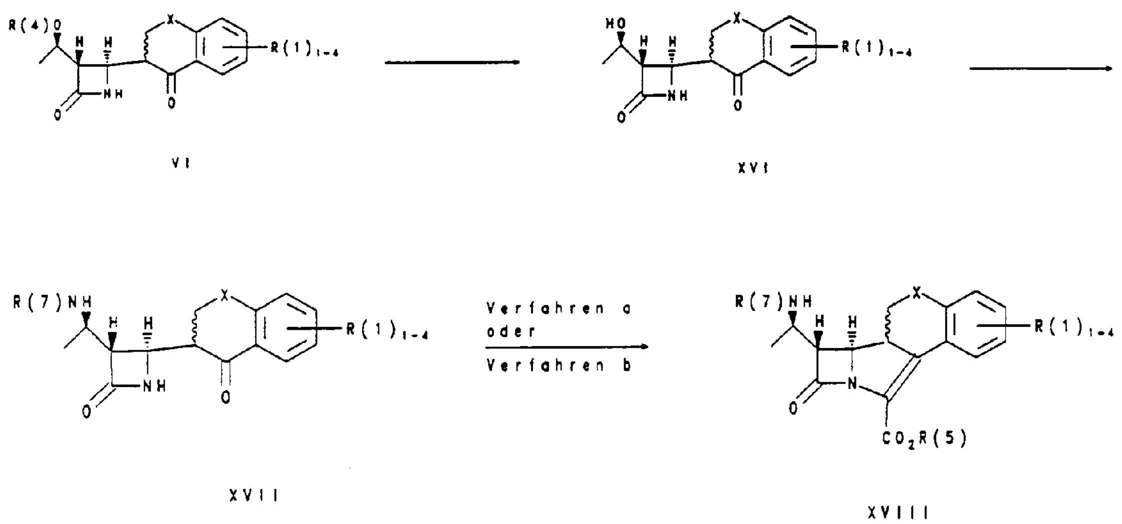


VI XVI XVII XVIII

Durch Methoden, wie sie z. B. in der Europäischen Offenlegungsschrift 89122711.8 beschrieben sind und anschließendem Schutz der Aminogruppe, z. B. mit der Allyloxycarbonylgruppe, erhält man Verbindungen der Formel XVII, die nach Verfahren a) oder b) in Verbindungen der Formel XVIII übergeführt werden.

Nach Abspaltung der Aminoschutzgruppe können Derivatisierungen, z. B. Acylierung oder Alkylierung, nach Literaturmethoden durchgeführt werden, bevor anschließend die Carboxyschutzgruppe gespalten wird.

Die Substituenten R(1) werden über die Vorstufen der Formel V in die Verbindungen der Formel I eingeführt. In einigen Fällen ist es jedoch vorteilhaft, die Substituenten R(I) durch an sich bekannte Reaktionen bei Verbindungen mit der Formel VIII einzuführen, z. B. durch die folgenden Umwandlungen:

R(1) = $CH_2OH$ in R(1) = $CH_2^{\oplus}$ 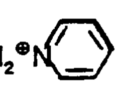

oder R(1) = $CO_2C_6F_5$ in R(1) = $CON(CH_3)_2$. Anschließend erfolgt wie oben beschrieben die Abspaltung der Schutzgruppen.

Sollen nach Verfahren c) Verbindungen der Formel I erhalten werden, in der R(3) für ($C_1$-$C_6$)-Alkanoyloxy-($C_1$-$C_3$)-alkyl, ($C_1$-$C_6$)-Alkoxycarbonyloxy-($C_1$-$C_3$)-alkyl oder (2-Oxo-5-methyl-1,3-dioxolen-4-yl)-methyl steht, so setzt man in an sich bekannter Weise die Verbindungen der Formel I, in denen R(3) = Wasserstoff bedeutet, mit einer Verbindung der Formel XIX oder XX um.

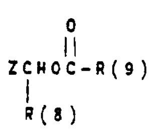

XIX

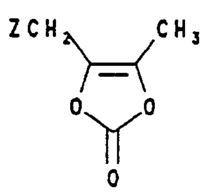

XX

Dabei bedeuten R(8) Wasserstoff oder eine ($C_1$-$C_2$)-Alkylgruppe, R(9) eine ($C_1$-$C_6$)-Alkylgruppe oder eine ($C_1$-$C_6$)-Alkoxygruppe und Z bedeutet Halogen, vorzugsweise Chlor, Brom oder Jod. Dabei werden Verfahren angewendet, wie sie für Veresterungsreaktionen bekannt sind.

Als pharmazeutisch verträgliche Salze der Verbindungen der Formel I seien beispielsweise erwähnt Lithium-, Natrium-, Kalium-, Calcium-, Magnesiumsalze oder Salze mit organischen Aminen wie Diethylamin, Benethamin, Piperazin, Tromethamin.

Die erfindungsgemäßen Verbindungen der Formel I und ihre pharmazeutisch verträglichen Salze zeigen bemerkenswert gute antibakterielle Wirksamkeit sowohl gegen Gram-positive als auch Gram-negative Keime. Die Verbindungen weisen gegenüber der renalen Dehydropeptidase eine hohe Stabilität auf.

Auch gegen Penicillinase- und Cephalosporinase-bildende Bakterien sind die Verbindungen der Formel I unerwartet gut wirksam. Da sie zudem günstige toxikologische und pharmakologische Eigenschaften zeigen, stellen sie wertvolle Chemotherapeutika dar.

Die Erfindung betrifft auch Arzneipräparate zur Behandlung von mikrobiellen Infektionen, die durch einen Gehalt an einer oder mehreren der erfindungsgemäßen Verbindungen charakterisiert sind.

Die erfindungsgemäßen Produkte können auch in Kombinationen mit anderen Wirkstoffen, beispielsweise aus der Reihe der Penicilline, Cephalosporine, Quinolone, Glykopeptide oder Aminoglykoside zur Anwendung kommen.

Die Verbindungen der Formel I und ihre pharmazeutisch verträglichen Salze können oral, intramuskulär oder intravenös verabfolgt werden.

Arzneipräparate, die eine oder mehrere Verbindungen der Formel I als Wirkstoff enthalten, können hergestellt werden, indem man die Verbindungen der Formel I mit mehreren pharmakologisch verträglichen Trägerstoffen oder Verdünnungsmitteln, wie Füllstoffen, Emulgatoren, Gleitstoffen, Geschmackskorrigentien, Farbstoffen oder Puffersubstanzen, vermischt und in eine geeignete galenische Zubereitungsform bringt, wie Tabletten, Dragees, Kapseln oder eine zur parenteralen Applikation geeignete Suspension oder Lösung.

Als Träger- oder Verdünnungsmittel seien beispielsweise erwähnt: Traganth, Milchzucker, Talkum, Agar-Agar, Polyglykole, Ethanol und Wasser. Puffersubstanzen sind beispielsweise organische Verbindungen, wie z. B. N,N′-Dibenzylethylendiamin, Diethanolamin, Ethylendiamin, N-Methylglucamin, N-Benzylphenethylamin, Diethylamin und Tris-(hydroxymethyl)aminomethan (Tromethamin), oder anorganische Verbindungen, wie Phosphatpuffer, Natriumbicarbonat und Natriumcarbonat. Für die parenterale Applikation kommen vorzugsweise Suspensionen oder Lösungen in Wasser mit oder ohne Puffersubstanzen in Betracht. Es ist auch möglich, die Wirkstoffe als solche ohne Träger- oder Verdünnungsmittel in geeigneter Form, beispielsweise in Kapseln, zu applizieren.

Geeignete Dosen der Verbindungen der Formel I oder ihrer pharmazeutisch verträglichen Salze liegen bei etwa 0,4 g bis maximal etwa 20 g pro Tag, vorzugsweise von 1 bis 10 g, insbesondere bei 2 bis 6 g/Tag für einen Erwachsenen von etwa 75 kg Körpergewicht.

Es können Einzel- oder im allgemeinen Mehrfachdosen verabreicht werden, wobei die Einzeldosis den Wirkstoff in einer Menge von etwa 50 bis 1000 mg, vorzugsweise von etwa 100 bis 500 mg, enthalten kann.

Die folgenden Ausführungsbeispiele für erfindungsgemäß herstellbare 5R, 6S-Verbindungen dienen zur weiteren Erläuterung der Erfindung.

In den Beispielen wurden die folgenden Abkürzungen benutzt: THF = Tetrahydrofuran, DMF = Dimethylformamid, s = Singulett, d = Dublett, t = Triplett, q = Quartett, m = Multiplett, mc = zentriertes Multiplett, bs = breites Singulett.

Beispiel 1:

Kalium-(1S,5R,6S)-6-[(1R)-1-hydroxyethyl]-(1,2,3,4-tetrahydronaphtho)[2,1-a]carbapen-2-em-3-carboxylat

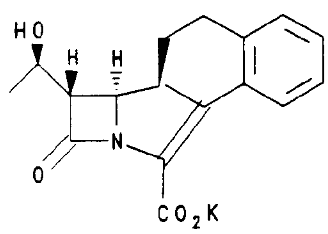

Stufe 1:

(3S,4R)-3-[(1R)-1-tert.-Butyldimethylsilyloxyethyl]-4-[(2R)-1-oxo-1,2,3,4-tetrahydronaphth-2-yl]-azetidin-2-on.

Eine Suspension von 126 g (1.06 mol) Zinnpulver in 260 ml DMF und 130 ml Methylenchlorid wurde bei Zimmertemperatur mit 102 g (0.36 mol) (3S,4R)4-Acetoxy-3-[(1R)-1-tert.-butyldimethylsiloxyethyl]-azetidin-2-on und 3.0 g (11.8 mmol) Jod versetzt. Nach vollständiger Entfärbung der Lösung wurden 4.2 g (21.6 mmol) Silbertetrafluoroborat zugegeben und die Mischung 15 min bei Zimmertemperatur gerührt. Die Mischung wurde auf 10°C gekühlt und eine Lösung von 120 g (0.53 mol) 2-Bromtetralon in 60 ml DMF und 30 ml Methylenchlorid innerhalb von 1.5 h zugetropft, wobei die Innentemperatur in einem Intervall von ± 2°C bei 10°C gehalten wurde. Nach weiteren 60 min bei Zimmertemperatur wurde die Reaktionslösung in 1.5l eines Gemisches von Cyclohexan/Ethylacetat (1:1) gegossen und über Kieselgur (®Celite) filtriert. Die organische Phase wurde mit 300 ml 1n HCl, 600 ml 5 %iger Natronlauge und 300 ml Wasser extrahiert und über $MgSO_4$ getrocknet. Das Rohprodukt enthielt nach HPLC die beiden Isomeren im Verhältnis $\alpha/\beta$ = 2:3. Durch Umkristallisieren aus n-Heptan konnten 26.3 g (20 %) des β-Isomeren (Reinheit > 97 %) isoliert werden. - $^1$H-NMR (270 MHz, $CDCl_3$): δ = 0.10 (s, 6H, $SiCH_3$); 0.87 (s, 9H, $SiC(CH_3)_3$); 1.28 (d, 3H, $CH$-$CH_3$); 2.08 und 2.30 (2 x mc, 2 x 1H, $CH_2$-$CH_2$-CH); 3.07-3.14 (m, 3H, $CH_2$-$CH_2$-CH und H-3); 4.25 (mc, 1H, CH-$CH_3$); 4.45 (dd, 1H, H-4); 5.77 (bs, 1H, NH); 7.25-7.38 (m, 2H, Aromaten-H); 7.52 (mc, 1H, Aromaten-H); 8.02 (d, 1H, Aromaten-H).

Stufe 2:

[(3S,4R)-3-[(1R)-1-tert.-Butyldimethylsilyloxyethyl]-4-[(2R)-1-oxo-1,2,3,4-tetrahydronaphth-2-yl]-2-oxoazetidin-1-yl]-2-oxoessigsäureallylester.

Eine Lösung von 2.0 g (5.35 mmol) (3S,4R)-3-[(1R)-1-tert.-Butyldimethylsilyloxyethyl]-4-[(2R)-1-oxo-1,2,3,4-tetrahydronaphth-2-yl]-azetidin-2-on in 45 ml wasserfreiem Methylenchlorid wurde unter Argon als Schutzgas bei 0°C mit 1.07 g (10.7 mmol) $CaCO_3$ und 1.59 g (10.7 mmol) Oxalsäureallylesterchlorid versetzt. Zu der Mischung tropfte man innerhalb von 1 h 1.81 ml (1.34 g, 10.4 mmol) Ethyldiisopropylamin in 10 ml Methylenchlorid und rührte weitere 2 h bei der angegebenen Temperatur. Der Feststoff wurde abgesaugt und die organische Phase zweimal mit je 20 ml Wasser extrahiert. Nach Trocknen über $MgSO_4$ wurde das Lösungsmittel am Rotationsverdampfer entfernt und der Rückstand an Kieselgel (desaktiviert mit 10 % $H_2O$) chromatographiert (Laufmittel: Toluol/Ethylacetat = 30:1). Ausbeute: 1.95 g (75 %), weiße Kristalle. - $^1$H-NMR (270 MHz, $CDCl_3$): δ = 0.06 und 0.08 (2 x s, 2 x 3H, $SiCH_3$); 0.85 (s, 9H, $SiC(CH_3)_3$); 1.19 (d, 3H, CH-$CH_3$, J = 6 Hz), 2.03 und 2.27 (2 x mc, 2 x 1H, $CH_2$-$CH_2$-CH); 3.12 (dd, 2H, $CH_2$-$CH_2$-CH, J = 4, 9 Hz); 3.21 (mc, 1H, $CH_2$-$CH_2$-CH, J = 4, 5, 10 Hz); 3.32 (dd, 1H, H-3, J = 4 Hz); 4.33 (mc, 1H, CH-$CH_3$); 4.67 (dd, 1H, H-4, J = 4 Hz); 4.81 (mc, 2H, $CH_2$-CH = $CH_2$); 5.27-5.45 (m, 2H, $CH_2$-CH=$CH_2$, J = 10, 17 Hz, J(Allyl) = 1 Hz); 5.97 (mc, 1H, $CH_2$-CH=$CH_2$); 7.23-7.37 (m, 2H, Aromaten-H); 7.50 (m, 1H, Aromaten-H); 8.04 (d, 1H, Aromaten-H).

Stufe 3:

(1S,5R,6S)-6-[(1R)-1-tert.-Butyldimethylsilyloxyethyl]-(1,2,3,4-tetrahydronaphtho)[2,1-a]carbapen-2-em-3-carbonsäureallylester.

300 mg (0.62 mmol) [(3S,4R)-3-[(1R)-1-tert.-Butyldimethylsilyloxyethyl]-4-[(2R)-1-oxo-1,2,3,4-tetrahydronaphth-2-yl]-2-oxoazetidin-1-yl]-2-oxoessigsäureallylester und 255 mg (2.12 mmol) Methanphosphonigsäurediethylester wurden in 10 ml wasserfreiem Mesitylen unter Argon 3 h bei 160°C gerührt. Nach Entfernen des Lösungsmittels im Hochvakuum erhielt man nach Säulenchromatographie (Kieselgel, Laufmittel: Toluol/Ethylacetat = 30:1) 176 mg (63 %) des zyklisierten Produktes, Schmp. 126°C. - $^1$H-NMR (270 M Hz, $CDCl_3$): δ = 0.11 (s, 6H, $SiCH_3$); 0.92 (s, 9H, $SiC(CH_3)_3$); 1.27 (d, 3H, CH-$CH_3$); 1.94 und 2.08 (2 x mc, 2 x 1H, $CH_2$-$CH_2$-CH); 3.07 (dd, 2H, $CH_2$-$CH_2$-CH); 3.18 (mc, 1H, $CH_2$-$CH_2$-CH); 3.28 (dd, 1H, H-6); 4.20-4.35 (m, 2H, CH-$CH_3$ und H-5); 4.78 (mc, 2H, $CH_2$-CH = $CH_2$); 5.23-5.47 (m, 2H, $CH_2$-CH = $CH_2$); 5.98 (mc, 1H, $CH_2$-CH = $CH_2$); 7.10-7.25 (m, 3H, Aromaten-H); 7.77 (d, 1H, Aromaten-H).

Stufe 4:

(1S,5R,6S)-6-[(1R)-1-Hydroxyethyl]-(1,2,3,4-tetrahydronaphtho)[2,1-a]carbapen-2-em-3-carbonsäureallylester.

Bei 0°C gab man zu einer Lösung von 586 mg (1.29 mmol) (1S,5R,6S)-6-[(1R)-1-tert.-Butyldimethylsiloxyethyl](1,2,3,4-tetrahydronaphtho)[2,1-a]carbapen-2-em-3-carbonsäure-allylester in 6 ml wasserfreiem THF 465 mg (7.75 mmol) Essigsäure (10 %ige Lösung in THF) und tropfte anschließend eine Lösung von 1.22 g (3.87 mmol) Tetrabutylammoniumfluorid-Trihydrat in 11 ml THF innerhalb von 15 min zu. Die Eiskühlung wurde entfernt und das Reaktionsgemisch weitere 54 h bei Zimmertemperatur gerührt. Das Lösungsmittel wurde im Vakuum entfernt, der Rückstand in Ethylacetat aufgenommen und die organische Phase mit je 25 ml einer ges. $NaHCO_3$-Lösung und Wasser extrahiert. Nach Trocknen über $MgSO_4$ und Einengen der Lösung wurde das Produkt durch Chromatographie über Kieselgel (Laufmittel: Toluol/Ethylacetat = 3:1) isoliert. Ausbeute: 216 mg (49 %). - $^1$H-NMR (270 MHz. $CDCl_3$): δ= 1.37 (d, 3H, $CH$-$CH_3$); 1.85-1.99 (m, 2H, $CH_2$-$CH_2CH$ und OH); 2.08-2.20 (m, 1H, $CH_2$-$CH_2$-CH); 3.06 (dd, 2H, $CH_2$-$CH_2$-CH); 3.22 (mc, 1H, $CH_2$-$CH_2$-CH); 3.32 (dd, 1H, H-6); 4.23-4.38 (m, 1H, CH-$CH_3$); 4.35 (dd, 1H, H-5); 4.78 (mc, 1H, $CH_2$-CH=$CH_2$); 5.24-5.48 (m, 2H, $CH_2$-CH=$CH_2$); 5.98 (mc, 1H, $CH_2$-CH=$CH_2$); 7.10-7.29 (m, 3H, Aromaten-H), 7.78 (d, 1H, Aromaten-H).

Stufe 5:

Kalium-(1S,5R,6S)-6-[(1R)-1-hydroxyethyl]-(1,2,3,4-tetrahydronaphtho)[2,1-a]carbapen-2-em-3-carboxylat.

Unter Ausschluß von Sauerstoff gab man zu 309 mg (0.92 mmol) (1S,5R,6S)-6-[(1R)-1-Hydroxyethyl]-(1,2,3,4-tetrahydronaphtho)[2,1-a]carbapen-2-em-3-carbonsäureallylester, gelöst in 3 ml Methylenchlorid, eine Lösung von 185 mg (1.0 mmol) Kalium-2-ethylhexanoat in 2 ml Ethylacetat. Nach Zusatz von 12 mg (0.05 mmol) Triphenylphosphin und 30 mg (0.03 mmol) $Pd[P(C_6H_5)_3]_4$ rührte man 30 min bei Zimmertemperatur, verdünnte mit 15 ml Methylenchorid und setzte 20 ml Wasser zu. Die Wasserphase wurde abgetrennt und das Methylenchlorid nochmals mit 20 ml Wasser extrahiert. Anschließend wurden die vereinigten Wasserphasen mit 15 ml Methylenchlorid extrahiert und gefriergetrocknet. Der Rückstand wurde mit Wasser über (R)LiChroprep RP18 chromatographiert. Nach dem Gefriertrocknen der produkthaltigen Fraktionen erhielt man 155 mg (50 %) des gewünschten Produktes. - $^1$H-NMR (270 MHz, DMSO): δ = 1.13 (d, 3H,CH-$CH_3$); 1.65 und 1.92 (2 x mc, 2 x 1H, $CH_2$-$CH_2$-CH); 2.84-3.05 (m, 3H, $CH_2$-$CH_2$-CH); 3.20 (dd, 1H, H-6); 3.95 (mc, 1H, C-$CH_3$); 4.10 (dd, 1H, H-5); 4.95 (d, 1H, OH); 6.91-7.04 (m, 3H, Aromaten-H); 7.65 (d, 1H, Aromaten-H).

Beispiel 2:

Kalium-(1S,5R,6S)-6-[(1R)-1-hydroxyethyl]-(1,2,3,4-tetrahydronaphtho)[2,1-a]carbapen-2-em -3-carboxylat.

Stufe 1:

(3S,4R)-1-[(Allyloxycarbonyl)-hydroxymethyl]-3-[(1R)-1-tert.-butyldimethylsilyloxyethyl]-4-[(2R)-1-oxo-1,2,3,4-tetrahydronaphth-2-yl]-azetidin-2-on.

Zu einer Lösung von 2.0 g (5.35 mmol) des Azetidinons (siehe Stufe 1 von Beispiel 1) in 6 ml wasserfreiem THF wurden unter Argon 1.23 g (10.8 mmol) Glyoxylsäureallylester gegeben und anschließend über eine Spritze 0.21 ml (1.5 mmol) Triethylamin langsam zugetropft. Nach 4 h bei Zimmertemperatur wurde das Reaktionsgemisch auf Eiswasser gegossen, die organische Phase abgetrennt und die wässrige Phase mehrmals mit Ethylacetat extrahiert. Das Lösungsmittel wurde am Rotationsverdampfer entfernt und das Rohprodukt direkt weiter in Stufe 2 umgesetzt.

Stufe 2:

(3S,4R)-1-[(Allyloxycarbonyl)-chlormethyl]-3-[(1R)-1-tert.-butyldimethylsilyloxyethyl]-4-[(2R)-1-oxo-1,2,3,4-tetrahydronaphth-2-yl]-azetidin-2-on.

Die Lösung von 2.0 g der Hydroxyverbindung in 40 ml THF wurde bei -30°C mit 1.55 ml (1.43 g, 13.3 mmol) 2,6-Lutidin versetzt. Man tropfte 0.85 ml (1.39 g, 11.6 mmol) Thionylchlorid in 5 ml THF zu und rührte die Mischung 1 h bei der angegebenen Temperatur. Der Reaktionsansatz wurde im Ölpumpenvakuum eingeengt,

der Rückstand in Ethylacetat aufgenommen und das Produkt durch Filtration von unlöslichen Bestandteilen abgetrennt. Nach Entfernen des Lösungsmittels am Rotationsverdampfer wurde die Rohsubstanz sofort weiter in Stufe 3 eingesetzt.

Stufe 3:

(3S,4R)-1-[(Allyloxycarbonyl)-triphenylphosphoranylidenmethyl]-3-[(1R)-1-tert.-butyldimethylsilyloxyethyl]-4-[(2R)-1-oxo-1,2,3,4-tetrahydronaphth-2-yl]-azetidin-2-on.

Zu einer Lösung der Chlorverbindung in 6 ml DMF gab man 1.3 g (4.96 mmol) Triphenylphosphin und rührte die Mischung 60 min bei Zimmertemperatur. Anschließend wurde der Reaktionsansatz mit 30 ml Ethylacetat versetzt und dreimal mit je 20 ml einer verd. $NaHCO_3$-Lösung gewaschen. Die organische Phase wurde über $MgSO_4$ getrocknet und das Lösungsmittel im Vakuum entfernt. Die Chromatographie an Kieselgel (Laufmittel: Toluol/Ethylacetat = 5/1 bis 2/1) ergab 905 mg (23 %, über drei Stufen) des Phosphorans. - MS (FAB): m/e = 738 (M + Li), 732 (M + H+).

Stufe 4:

(1S,5R,6S)-6-[(1R)-1-tert.-Butyldimethylsilyloxyethyl]-(1,2,3,4-tetrahydronaphtho)[2,1-a]carbapen-2-em-3-carbonsäureallylester.

Eine Lösung von 893 mg (1.22 mmol) Phosphoran in 20 ml Mesitylen wurde 3 h auf 160°C erhitzt. Das Lösungsmittel wurde im Vakuum entfernt und das Rohprodukt über Kieselgel (Laufmittel: Toluol/Ethylacetat = 30/1) chromatographiert. Ausbeute: 141 mg (25 %). Die Verbindung ist laut $^1H$-NMR-Daten mit dem Produkt aus Beispiel 1/Stufe 3 identisch.

Beispiel 3:

Kalium-(1S,5R,6S)-6-[(1R)-1-hydroxyethyl]-(1,2,3,4-tetrahydronaphtho)[2,1-a]carbapen-2-em-3-carboxylat.

Stufe 1:

[(3S,4R)-3-[(1R)-1-Hydroxyethyl]-4-[(2R)-1-oxo-1,2,3,4-tetrahydronaphth-2-yl)]-2-oxoazetidin-1-yl]-2-oxoessigsäureallylester.

Zu einer Lösung von 250 mg (0.52 mmol) der N-Oxalylverbindung (Produkt aus Stufe 2 von Beispiel 1) in 5 ml Acetonitril tropfte man bei -40°C innerhalb von 20 min 109 mg (0.78 mmol) Bortrifluorid-Etherat. Nach weiteren 2 h bei dieser Temperatur versetzte man das Reaktionsgemisch mit 10 ml verd. $NaHCO_3$-Lsg. und extrahierte die Mischung mit Ethylacetat. Die organischen Phasen wurden über $MgSO_4$ getrocknet und das Lösungsmittel im Vakuum entfernt. Das Rohprodukt wurde direkt weiter zum Silylether umgesetzt.

Stufe 2:

[(3S,4R)-3-[(1R)-1-Trimethylsilyloxyethyl]-4-[(2R)-1-oxo-1,2,3,4-tetrahydronaphth-2-yl)]-2-oxoazetidin -1-yl]-2-oxoessigsäureallylester.

Eine Mischung von 67 mg (0.66 mmol) Triethylamin und 72 mg (0.66 mmol) Chlortrimethylsilan in 2 ml Methylenchlorid wurde langsam mit 177 mg (0.44 mmol) der Hydroxyverbindung, gelöst in 1 ml Methylenchlorid, versetzt und 2.5 h bei Zimmertemperatur gerührt. Das Lösungsmittel wurde im Ölpumpenvakuum entfernt und der Rückstand an Kieselgel (Laufmittel: Toluol/Ethylacetat = 30: 1) chromatographiert. Ausbeute: 64 mg (28 %, über beide Stufen) Silylether. - $^1H$-NMR (270 MHz, $CDCl_3$): $\delta$ = 0.10 (s, 9H, $SiCH_3$); 1.20 (d, 3H, CH-$CH_3$); 1.98-2.30 (m, 2H, CH-$CH_2$-$CH_2$); 3.06-3.24 (m, 3H, CH-$CH_2$-$CH_2$); 3.32 (dd, 1H, H-3); 4.29 (mc, 1H, CH-$CH_3$); 4.58 (dd, 1H, H-4); 4.82 (d, 2H, $CH_2$-CH=$CH_2$); 5.29-5.47 (m, 2H, $CH_2$-CH=$CH_2$); 5.98 (mc, 1H, $CH_2$-CH=$CH_2$); 7.22-7.35 (m, 2H, Aromaten-H); 7.50 (mc, 1H, Aromaten-H); 8.03 (d, 1H, Aromaten-H).

Stufe 3:

(1S,5R,6S)-6-[(1R)-1-Trimethylsilyloxyethyl]-(1,2,3,4-tetrahydronaphtho)[2,1-a]carbapen-2-em -3-carbonsäureallylester.

131 mg (0.30 mmol) Trimethylsilylether wurden wie im Beispiel 1 unter Stufe 3 beschrieben mit Methanphosphonigsäurediethylester umgesetzt. Nach chromatographischer Reinigung (Kieselgel, Laufmittel: Toluol/Ethylacetat = 30/1) erhielt man 33 mg (27 %) Zyklisierungsprodukt. - $^1$H-NMR (270 MHz, $CDCl_3$): $\delta$ = 0.16 (s, 9H, $SiCH_3$); 1.28 (d, 3H, $CH\text{-}CH_3$); 1.53-2.15 (m, 2H, $CH\text{-}CH_2\text{-}CH_2$); 3.07 (mc, 2H, $CH\text{-}CH_2\text{-}CH_2$); 3.19 (mc, 1H, $CH\text{-}CH_2\text{-}CH_2$); 3.29 (dd, 1H, H-6); 4.18-4.32 (m, 2H, H-5 und $CH\text{-}CH_3$); 4.79 (mc, 2H, $CH_2\text{-}CH{=}CH_2$); 5.22-5.47 (m, 2H, $CH_2\text{-}CH{=}CH_2$); 5.99 (mc, 1H, $CH_2\text{-}CH{=}CH_2$); 7.10-7.28 (m, 3H, Aromaten-H); 7.76 (d, 1H, Aromaten-H).

Stufe 4:

(1S,5R,6S)-6-[(1R)-1-Hydroxyethyl]-(1,2,3,4-tetrahydronaphtho)-[2,1-a]-carbapen-2-em-3-carbonsäureallylester.

Die Silylschutzgruppe wurde analog zu Stufe 4 in Beispiel 1 mit Tetrabutylammoniumfluorid Trihydrat gespalten. Die Reaktion war jedoch bereits nach 30 min bei Zimmertemperatur vollständig. Ausgehend von 30 mg (0.07 mmol) Substrat erhielt man nach Chromatographie an Kieselgel (Laufmittel: Toluol/Ethylacetat = 3/1) 12 mg (49 %) der Hydroxyverbindung. Das Produkt ist nach dem 1H-NMR Vergleichsspektrum mit dem Produkt von Beispiel 1/Stufe 4 identisch.

Die Spaltung des Allylesters zum Kaliumsalz wurde bereits in Beispiel 1 unter Stufe 5 beschrieben.

Beispiel 4:

Kalium-(1R,5R,6S)-6-[(1R)-1-hydroxyethyl]-(1,2,3,4-tetrahydronaphtho)[2,1-a]carbapen-2-em-3-carboxylat.

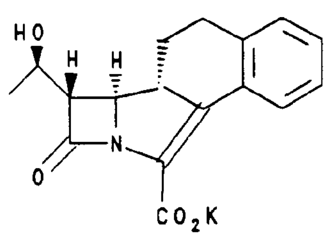

Stufe 1:

(3S,4R)-3-[(1R)-1-tert.-Butyldimethylsilyloxyethyl]-4-[(2S)-1-oxo-1,2,3,4-tetrahydronaphth-2-yl]-azetidin-2-on.

Das unter Beispiel 1 in Stufe 1 erhaltene Rohprodukt wurde an Kieselgel chromatographiert (Laufmittel: Toluol/Ethylacetat = 4/1). Nach Kristallisation aus n-Heptan erhielt man das weniger polare o-Diastereomere in 14 % Ausbeute. Schmp. (Heptan) 152°C. -$^1$H-NMR (270 M Hz, $CDCl_3$): $\delta$ = 0.06 und 0.09 (2 x s, 2 x 3H, $SiCH_3$); 0.89 (s, 9H, $SiC(CH_3)_3$); 1.28 (d, 3H, $CH\text{-}CH_3$); 1.79-1.98 und 2.24-2.46 (2 x m, 2 x 1H, $CH\text{-}CH_2\text{-}CH_2$); 2.55 (mc, 1H, $CH\text{-}CH_2\text{-}CH_2$); 2.86 (mc, 1H, H-3); 2.96-3.19 (m, 2H, $CH\text{-}CH_2\text{-}CH_2$); 3.73 (dd, 1H, H-4); 4.20 (mc, 1H, $CH\text{-}CH_3$); 6.45 (br, 1H, NH); 7.24-7.39 (m, 2H, Aromaten-H); 7.51 (t, 1H, Aromaten-H); 8.01 (d, 1H, Aromaten-H).

Stufe 2:

[(3S,4R)-3-[(1R)-1-tert.-Butyldimethylsilyloxyethyl]-4[(2S)-1-oxo-1,2,3,4-tetrahydronaphth-2-yl]-2-oxoazetidin-1-yl]-2-oxoessigsäureallylester.

Wie im Beispiel 1 unter Stufe 2 beschrieben wurden 5 g (13.4 mmol) Azetidin-2-on acyliert. Nach Chromatographie an Kieselgel (Laufmittel: Heptan/Ethylacetat = 4: 1) erhielt man 6.06 g (93 %) öliges Produkt.-$^1$H-

NMR (270 MHz, $CDCl_3$): δ = 0.05 und 0.10 (2 x s, 2 x 3H, $SiCH_3$); 0.88 (s, 9H, $SiC(CH_3)_3$); 1.30 (d, 3H, CH-$CH_3$); 1.95-2.12 und 2.20-2.33 (2 x m, 2 x 1H, CH-$CH_2$-$CH_2$); 3.04-3.11 (m, 2H, CH-$CH_2$-$CH_2$); 3.18 (m, 1H, H-3); 3.51 (mc, 1H, CH-$CH_2$-$CH_2$); 4.37 (mc, 1H, CH-$CH_3$); 4.81 (2H, d, $CH_2$-CH=$CH_2$); 5.13 (mc, 1H, H-4); 5.33 und 5.42 (2 x d, 2 x 1H, $CH_2$-CH = $CH_2$); 5.98 (mc, 1H, $CH_2$-CH=$CH_2$); 7.22-7.38 (m, 2H, Aromaten-H); 7.51 (t, 1H, Aromaten-H); 8.03 (d, 1H, Aromaten-H).

Stufe 3:

(1R,5R,6S)-6-[(1R)-1-tert.-Butyldimethylsilyloxyethyl]-(1,2,3,4-tetrahydronaphtho)[2,1-a]carbapen-2-em-3-carbonsäureallylester.

6.0 g (12.4 mmol) Allylester in 150 ml wasserfreiem Xylol wurden bei 140°C mit 10.2 g (37.2 mmol) Methanphosphonigsäurediethylester umgesetzt. Nach dem Einengen im Hochvakuum und Chromatographie an Kieselgel (Laufmittel: Toluol/Ethylacetat = 98:2) erhielt man 346 mg (6 %) Produkt.-$^1$H-NMR (270 MHz, $CDCl_3$): δ = 0.09 und 0.10 (2 x s, 2 x 3H, $SiCH_3$); 0.90 (s, 9H, $SiC(CH_3)_3$); 1.28 (d, 3H, CH-$CH_3$); 1.85-2.05 und 2.18-2.30 (2 x m, 2 x 1H, CH-$CH_2$-$CH_2$); 2.95-3.03 (m, 2H, CH-$CH_2$-$CH_2$); 3.18 (dd, 1H, H-6); 3.29 (mc, 1H, CH-$CH_2$-$CH_2$); 3.78 (dd, 1H, H-5); 4.24 (mc, 1H, CH-$CH_3$); 4.77 (mc, 2H, $CH_2$-CH=$CH_2$); 5.25 und 5.44 (2 x d, 2 x 1H, $CH_2$-CH=$CH_2$); 5.99 (mc, 1H, $CH_2$-CH-$CH_2$); 6.95-7.30 (m, 3H, Aromaten-H); 8.48 (d, 1H, Aromaten-H).

Stufe 4:

(1R,5R,6S)-6-[(1R)-1-Hydroxyethyl]-(1,2,3,4-tetrahydronaphtho)[2,1-a]carbapen-2-em-3-carbonsäureallylester.

Die Spaltung des Silylethers wurde wie unter Beispiel 1 als Stufe 4 beschrieben durchgeführt. Aus 330 mg (0.73 mmol) Silylether wurden 145 mg (59 %) Produkt erhalten, das direkt in Stufe 5 eingesetzt wurde.

Stufe 5:

Kalium-(1R,5R,6S)-6-[(1R)-1-hydroxyethyl]-(1,2,3,4-tetrahydronaphtho)[2,1-a]carbapen-2-em-3-carboxylat.

Ausgehend von 145 mg (0.43 mmol) Allylester erhielt man analog zur Stufe 5 in Beispiel 1 nach dem Gefriertrocknen 104 mg (72 %) Kaliumsalz.- $^1$H-NMR (270 MHz, $D_2O$): δ = 1.32 (d, 3H, CH-$CH_3$); 1.68-1.86 und 2.21-2.33 (2 x m, 2 x 1H, CH-$CH_2$-$CH_2$); 2.89-2.98 (m, 2H, CH-$CH_2$-$CH_2$); 3.36 (mc, 1H, CH-$CH_2$-$CH_2$); 3.47 (dd, 1H, H-6); 3.88 (dd, 1H, H-5); 4.25 (mc, 1H, CH-$CH_2$); 7.16-7.30 (m, 3H, Aromaten-H); 7.95 (d, 1H, Aromaten-H).

Beispiel 5:

Kalium-(1S,5R,6S)-6-[(1R)-1-hydroxyethyl]-(7-fluor-1,2,3,4-tetrahydronaphtho)[2,1-a]carbapen-2-em-3-carboxylat.

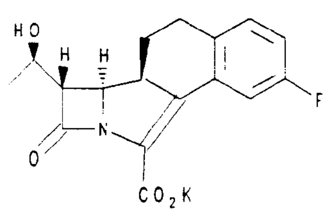

Stufe 1:

(3S,4R)-3-[(1R)-1-tert.-Butyldimethylsilyloxyethyl]-4-[(2R)-7-fluor-1-oxo-1,2,3,4-tetrahydronaphth-2-yl]-azetidin-2-on.

Wie für die Stufe 1 im Beispiel 1 beschrieben wurde, setzte man 9.9 g (40 mmol) 2-Brom-7-fluor-tetralon um. Nach der Chromatographie an Kieselgel (Laufmittel: Toluol/Ethylacetat = 4/1) erhielt man 810 mg (8 %)

Produkt.-$^1$H-NMR (270 MHz; $CDCl_3$): δ = 0.09 (s, 6H, $SiCH_3$); 0.88 (s, 9H, $SiC(CH_3)_3$); 1.27 (d, 3H, $CH\text{-}CH_3$); 1.96-2.15 und 2.22-2.35 (2 x m, 2 x 1H, $CH\text{-}CH_2\text{-}CH_2$); 2.73 (mc, 1H, $CH\text{-}CH_2\text{-}CH_2$); 3.02-3.13 (m, 3H, H-3, $CH\text{-}CH_2\text{-}CH_2$); 4.27 (mc, 1H, $CH\text{-}CH_3$); 4.43 (dd, 1H, H-4); 5.82 (br, 1H, NH); 7.16-7.30 (m, 2H, Aromaten-H); 7.67 (dd, 1H, Aromaten-H).

Stufe 2:

[(3S,4R)-3-[(1R)-1-tert.-Butyldimethylsilyloxyethyl]-4-[(2R)-7-fluor-1-oxo-1,2,3,4-tetrahydronaphth -2-yl]-2-oxoazetidin-1-yl]-2-oxoessigsäureallylester.

Analog zur Stufe 2 im Beispiel 1 synthetisierte man aus 756 mg (1.93 mmol) Azetidin-2-on 890 mg (92 %) Allylester. Schmp. 100-101°C-$^1$H-NMR (270 MHz, $CDCl_3$): δ = 0.07 und 0.09 (2 x s, 2 x 3H, $SiCH_3$); 0.86 (s, 9H, $SiC(CH_3)_3$); 1.20 (d, 3H, $CH\text{-}CH_3$); 1.92-2.10 und 2.20-2.32 (2 x m, 2 x 1H, $CH\text{-}CH_2\text{-}CH_2$); 3.01-3.12 (m, 2H, $CH\text{-}CH_2\text{-}CH_2$); 3.17 (mc, 1H, $CH\text{-}CH_2\text{-}CH_2$); 3.29 (mc, 1H, H-3); 4.33 (mc, 1H, $CH\text{-}CH_3$); 4.69 (mc, 1H, H-4); 4.80 (d, 2H, $CH_2\text{-}CH{=}CH_2$); 5.31 und 5.41 (2 x d, 2 x 1H, $CH_2\text{-}CH{=}CH_2$); 5.97 (mc, 1H, $CH_2\text{-}CH{=}CH_2$); 7.15-7.28 (m, 2H, Aromaten-H); 7.70 (dd, 1H, Aromaten-H).

Stufe 3:

(1S,5R,6S)-6-[(1R)-1-tert.-Butyldimethylsilyloxyethyl]-(7-fluor-1,2,3,4-tetrahydronaphtho)[2,1-a]carbapen-2-em-3-carbonsäureallylester.

Die Zyklisierung von 870 mg (1.73 mmol) Allylester wurde wie im Beispiel 1 beschrieben durchgeführt. Nach 45 Minuten bei 160°C wurde aufgearbeitet. Nach der Chromatographie erhielt man 326 mg (40 %) Produkt.-$^1$H-NMR (270 MHz, $CDCl_3$): δ = 0.10 (s, 6H, $SiCH_3$); 0.90 (s, 9H, $SiC(CH_3)_3$); 1.27 (d, 3H, $CH\text{-}CH_3$); 1.82-2.14 (m, 2H, $CH\text{-}CH_2\text{-}CH_2$); 2.97-3.06 (m, 2H, $CH\text{-}CH_2\text{-}CH_2$); 3.17 (mc, 1H, $CH\text{-}CH_2\text{-}CH_2$); 3.28 (dd, 1H, H-6); 4.27 (mc, 1H, $CH\text{-}CH_3$); 4.32 (dd, 1H, H-5); 4.78 (mc, 2H, $CH_2\text{-}CH{=}CH_2$); 5.27 und 5.42 (2 x d, 2 x 1H, $CH_2\text{-}CH{=}CH_2$); 5.98 (mc, 1H, $CH_2\text{-}CH\text{-}CH_2$); 6.94 (dt, 1H, Aromaten-H); 7.08 (dd, 1H, Aromaten-H); 7.54 (dd, 1H, Aromaten-H).

Stufe 4:

(1S,5R,6S)-6-[(1R)-1-Hydroxyethyl]-(7-fluor-1,2,3,4-tetrahydronaphtho)[2,1-a]carbapen-2-em -3-carbonsäureallylester.

Aus 310 mg (0.66 mmol) Silylether stellte man analog zur Stufe 4 im Beispiel 1 die Hydroxyethylverbindung (118 mg, 50 %) dar. Diese wurde sofort weiter umgesetzt.

Stufe 5

Kalium-(1S,5R,6S)-6-[(1R)-1-hydroxyethyl]-(7-fluor-1,2,3,4-tetrahydronaphtho)[2,1-a]carbapen-2-em-3-carboxylat.

Aus 117 mg (0.33 mmol) Allylester erhielt man analog zu Stufe 5 im Beispiel 147 mg (40 %) Kaliumsalz.-$^1$H-NMR (270 MHz, $D_2O$): δ = 1.30 (d, 3H, $CH\text{-}CH_3$); 1.72-1.93 und 2.05-2.20 (2 x m, 2 x 1H, $CH\text{-}CH_2\text{-}CH_2$); 2.90-3.05 (m, 2H, $CH\text{-}CH_2\text{-}CH_2$); 3.25 (mc, 1H, $CH\text{-}CH_2\text{-}CH_2$); 3.58 (dd, 1H, H-6); 4.28 (mc, 1H, $CH\text{-}CH_3$); 4.38 (dd, 1H, H-5); 6.98 (dt, 1H, Aromaten-H); 7.15-7.28 (m, 2H, Aromaten-H).

Beispiel 6:

Natrium-(1S,5R,6S)-6-[(1R)-1-Hydroxyethyl]-(4,4-dimethyl-1,2,3,4-tetrahydronaphtho)[2,1-a]carbapen-2-em-3-carboxylat.

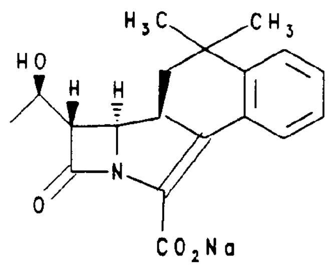

Stufe 1:

(3S,4R)-3-[(1R)-1-tert.-Butyldimethylsilyl-oxyethyl]-4-[(2RS)-4,4-dimethyl-1-oxo-1,2,3,4-tetrahydronaphth-2-yl]-azetidin-2-on.

7.5 g (30 mmol) 2-Brom-4,4-dimethyltetra1on wurden in Analogie zu Beispiel 1/Stufe 1 umgesetzt. Das Rohprodukt enthielt nach $^1$H-NMR die beiden Isomeren im Verhältnis $\alpha/\beta$ = 1/3. Das Isomerengemisch wurde nach Chromatographie an Kieselgel (desaktiviert mit 10 % $H_2O$, Laufmittel: Toluol/Ethylacetat = 5/1) erhalten. Ausbeute: 5.4 g (59 %).-$^1$H-NMR (270 MHz, $CDCl_3$): $\delta$ = 0.10 (s, 6H, $SiCH_3$); 0.89 (s, 9H, $SiC(CH_3)_3$; 1.32 (d, 3H, $CH$-$CH_3$): 1.39 (s, 3H, $CH_3$, $\beta$-Verbindung); 1.41 (s, 3H, $CH_3$, $\alpha$-Verbindung); 1.45 (s, 3H, $CH_3$, $\alpha$-Verbindung); 1.47 (s, 3H, $CH_3$, $\beta$-Verbindung); 1.85-2.20 (m, 2H, Sechsring-H); 2.90-3.03 (m, 1H, Sechsring-H); 3.05 (dd, 1H, H-3); 3.68 (dd, 1H, H-4, $\alpha$-Verbindung); 4.20-4.35 (m, 1H, $CH$-$CH_3$); 4.46 (dd, 1H, H-4, $\beta$-Verbindung); 5.73 (s, 1H, NH, $\beta$-Verbindung); 6.50 (s, 1H, NH, $\alpha$-Verbindung); 7.25-7.62 und 7.90-8.05 (m, 4H, Aromaten-H).

Stufe 2:

[(3S,4R)-3-[(1R)-1-tert.-Butyldimethylsilyloxyethyl]-4-[(2R,S)-4,4-dimethyl-1-oxo-1,2,3,4-tetrahydronaphth-2-yl]-2-oxoazetidin-1-yl]-2-oxoessigsäureallylester.

3.0 g (7.5 mmol) Azetidinon wurden in Analogie zu Beispiel 1/Stufe 2 umgesetzt. Das Produkt enthielt nach $^1$H-NMR die beiden Isomeren im Verhältnis $\alpha/\beta$ = 1:4. Ausbeute: 3.1 g (80 %).-$^1$H-NMR (270 MHz, $CDCl_3$): $\delta$ = 0.08 und 0.11 (2 x s, 2 x 3H, $SiCH_3$); 0.88 (s, 9H, $SiC(CH_3)_3$); 1.25 (d, 3H, $CH$-$CH_3$); 1.41 (s, 3H, $CH_3$); 1.48 (s, 3H, $CH_3$); 1.80-2.00 (m, 2H, Sechsring-H); 3.20 (mc, 1H, H-3, $\alpha$-Verbindung); 3.26 (mc, 1H, H-3, $\beta$-Verbindung); 3.45-3.65 (m, 1H, Sechsring-H, $\beta$-Verbindung); 3.54 -2.75 (m, 1H, Sechsring-H, $\alpha$-Verbindung); 4.20-4.40 (m, 1H, $CH$-$CH_3$); 4.57 (mc, H-4, $\beta$-Verbindung); 4.70-4.90 (m, 2H, -$CH_2$-$CH$=$CH_2$); 5.10 (mc, 1H, H-4, $\alpha$-Verbindung); 5.10-5.50 (m, 2H, -$CH_2$-$CH$ = $CH_2$); 5.85-6.10 (m, 1H, -$CH_2$-$CH$ = $CH_2$); 7.10-7.60 und 7.90-8.05 (m, 4H, Aromaten-H).

Stufe 3

(1S,5R,6S)-6-[(1R)-1-tert.-Butyldimethylsilyloxymethyl]-(4,4-dimethyl-1,2,3,4-tetrahydronaphtho)[2,1-a]carbapen-2-em-3-carbonsäureallylester.

3.8 g (7.5 mmol) Allylester wurden in Analogie zu Beispiel 1/Stufe 3 zyklisiert. Das Produkt enthielt nach $^1$H-NMR ausschließlich das $\beta$-Isomere. Ausbeute: 2.4 g (68 %).-$^1$H-NMR (270 MHz, $CDCl_3$): $\delta$ = 0.10 (s, 6H, $SiCH_3$); 0.90 (s, 9H, $SiC(CH_3)_3$); 1.28 (d, 3H, $CH$-$CH_3$); 1.40 (s, 3H, $CH_3$); 1.49 (s, 3H, $CH_3$); 1.75-1.93 (m, 2H, Sechsring-H); 3.15-3.40 (m, 2H, Sechsring-H und H-6); 4.15-4.40 (m, 2H, $CH$-$CH_3$ und H-5); 4.60-4.90 (m, 2H, $CH_2$-$CH$=$CH_2$); 5.20-5.50 (m, 2H, $CH_2$-$CH$=$CH_2$); 5.85-6.10 (m, 1H, $CH_2$-$CH$ = $CH_2$); 7.10-7.40 und 7.70-7.80 (m, 4H, Aromaten-H).

Stufe 4

(1S,5R,6S)-6-[(1R)-1-Hydroxyethyl]-(4,4-dimethyl-1,2,3,4-tetrahydronaphtho)[2,1-a]carbapen-2-em-3-carbonsäure-allylester.

400 mg (0.83 mmol) Silylether wurden in Analogie zu Beispiel 1/Stufe 4 umgesetzt. Ausbeute: 60 mg (20 %).-$^1$H-NMR (270 MHz, $CDCl_3$): δ = 1.34 (s, 3H, $CH_3$); 1.36 (d, 3H, CH-$CH_3$); 1.43 (s, 3H, $CH_3$); 1.70-1.90 (m, 2H, Sechsring-H); 2.75-3.05 (m, 1H, Sechsring-H); 3.18 (dd, 1H, H-6); 3.92 (dd, 1H, H-5); 4.15-4.30 (m, 1H, CH-$CH_3$); 4.60-4.75 (m, 2H, $CH_2$-CH=$CH_2$); 5.10-5.25 (m, 2H, $CH_2$-CH=$CH_2$); 5.70-5.90 (m, 1H, $CH_2$-$CH_2$ = $CH_2$); 7.10-7.40 (m, 4H, Aromaten-H).

Stufe 5:

Natrium-(1S,5R,6S)-6-[(1R)-1-Hydroxyethyl]-(4,4-dimethyl-1,2,3,4-tetrahydronaphtho)[2,1-a]carbapen-2-em-3-carboxylat.

50 mg (0.14 mmol) Allylester wurden in Analogie zu Beispiel 1/Stufe 5 umgesetzt. Ausbeute: 45 mg (92 %).-[1]-NMR (270 MHz, $D_2O$): δ = 1.28 (d, 3H, CH-$CH_3$); 1.32 und 1.42 (2 x s, 2 x 3H, $CH_3$); 1.82 (s, 1H, Sechsring-H); 1.86 (d, 1H, Sechsring-H); 3.08 (mc, 1H, Sechsring-H); 3.42 (dd, 1H, H-6); 3.96 (dd, 1H, H-5); 4.23 (mc, 1H, CH-$CH_3$); 7.14-7.30 (m, 2H, Aromaten-H); 7.39 (dt, 1H, Aromaten-H); 7.52 (d, 1H, Aromaten-H).

Beispiel 7:

Kalium-(1R,5R,6S)-6-[(1R)-1-hydroxyethyl]-(chromano)[3,4-a]carbapen-2-em-3-carboxylat

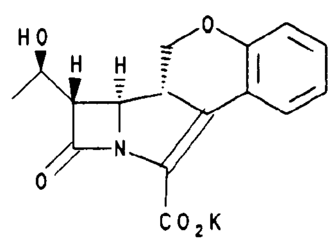

Stufe 1:

(3S,4R)-3-[(1R)-1-tert.-Butyldimethylsilyloxyethyl]-4-[(3R)-4-oxochroman-3-yl]azetidin-2-on.

Analog zu Stufe 1 in Beispiel 1 wurden 3.40 g (15 mmol) 3-Bromchroman-4-on umgesetzt. Das Rohprodukt enthielt nach HPLC die beiden Isomeren im Verhältnis α/β = 1:3. Durch Säulenchromatographie (Kieselgel, Laufmittel: Toluol/Ethylacetat = 3/1, konnten beide Isomere getrennt werden. Man isolierte 0.50 g (13 %) des α-Isomeren.-$^1$H-NMR (270 MHz, $CDCl_3$): δ = 0.09 (s, 6H, $SiCH_3$); 0.89 (s, 9H, Si-$C(CH_3)_3$); 1.28 (d, 3H, CH-$CH_3$); 2.86 (mc, 1H, O-$CH_2$-CH); 2.94 (mc, 1H, H-3); 3.71 (dd, 1H, H-4); 4.18 (mc, 1H, CH-$CH_3$); 4.29 und 4.61 (2 x 1H, O-$CH_2$-CH); 6.78 (bs, 1H, NH); 7.02 (mc, 2H, Aromaten-H); 7.50 (mc, 1H, Aromaten-H); 7.88 (mc, 1H, Aromaten-H).

Stufe 2:

[(3S,4R)-3-[(1R)-1-tert.-Butyldimethylsilyloxyethyl]-4-[(3R)-4-oxochroman-3-yl]-2-oxoazetidin-1-yl]-2-oxoessigsäureallylester.

Analog zu Stufe 2 in Beispiel 1 wurden 1.00 g (2.66 mmol) des Azetidinons mit Oxalsäurallylesterchlorid umgesetzt. Nach Säulenchromatographie an Kieselgel (desaktiviert mit 10 % $H_2O$, Laufmittel: Toluol/Ethylacetat= 30/1) erhielt man 1.25 g (96 %) Öl.-$^1$H-NMR (270 MHz, $CDCl_3$): δ = 0.05 (s, 6H, $SiCH_3$); 0.84 (s, 9H, $SiC(CH_3)_3$); 1.02 (d, 3H, CH-$CH_3$); 3.50 (m, 1H, O-$CH_2$-CH und H-3); 4.24 (mc, 1H, CH-$CH_3$); 4.60-4.80 (m, 3H, O-$CH_2$-CH und H-4); 4.82 (mc, 2H, $CH_2$-CH =$CH_2$); 5.37 (mc, 2H, $CH_2$-CH=$CH_2$); 5.96 (mc, 1H, $CH_2$-CH=$CH_2$); 7.02 (mc, 2H, Aromaten-H); 7.50 (mc, 1H, Aromaten-H); 7.79 (d, 1H, Aromaten-H).

Stufe 3:

(1S,5R,6S)-6-[(1R)-1-tert.-Butyldimethylsilyloxyethyl]-(chromano)[3,4a]carbapen-2-em-3-carbonsäureallylester.

1.30 g (2.66 mmol) Allylester wurden, wie in Beispiel 1 unter Stufe 3 beschrieben, in wasserfreiem Xylol umgesetzt. Nach Chromatographie an Kieselgel (Laufmittel: Toluol/Ethylacetat= 50/1) wurden 250 mg (20 %) als Öl isoliert.-$^1$H-NMR (270 MHz, $CDCl_3$): δ = 0.10 (s, 6H, $SiCH_3$); 0.89 (s, 9H, $SiC(CH_3)_3$); 1.28 (d, 3H, $CH\text{-}CH_3$); 3.24 (dd, 1H, H-6); 3.60-3.71 (m, 1H, $OCH_2\text{-}CH$); 3.77 (dd, 1H, H-5); 4.11 (dd, 1H, $O\text{-}CH_2\text{-}CH$); 4.22 (mc, 1H, $CH\text{-}CH_3$); 4.59 (dd, 1H, $O\text{-}CH_2\text{-}CH$); 4.70-4.90 (m, 2H, $CH_2\text{-}CH{=}CH_2$); 5.25-5.52 (m, 2H, $CH_2\text{-}CH{=}CH_2$); 5.90-6.10 (m, 1H, $CH_2\text{-}CH{=}CH_2$); 6.85-7.00 (m, 2H, Aromaten-H); 7.31 (mc, 1H, Aromaten-H); 8.60 (d, 1H, Aromaten-H).

Stufe 4:

(1S,5R,6S)-3-[(1R)-1-Hydroxyethyl]-(chromano)[3,4-a]carbapen-2-em-3-carbonsäureallylester.

Analog zu Stufe 4 in Beispiel 1 wurden 250 mg (0.55 mmol) des Silylethers umgesetzt. Nach Chromatographie (Kieselgel, Laufmittel: Toluol/Ethylacetat = 1/2) wurden 300 mg gelbes Öl erhalten und sofort weiter umgesetzt.

Stufe 5:

Kalium-(1R,5R,6S)-6-[(1R)-1-hydroxyethyl]-(chromano)[3,4-a]carbapen-2-em-3-carboxylat.

Analog zu Stufe 5 in Beispiel 1 wurden 300 mg des rohen Allylesters umgesetzt. Nach Chromatographie ((R)Lichroprep RP 18, Laufmittel: Wasser) isolierte man 100 mg (54 % bezogen auf Stufe 3) als gelben Feststoff.-$^1$H-NMR (270 MHz, $D_2O$): δ = 1.31 (d, 3H, $CH\text{-}CH_3$); 3.57 (dd, 1H, $OCH_2\text{-}CH$); 3.67 (mc, 1H, H-1); 3.89 (dd, 1H, H-5); 4.05 (dd, 1H, $O\text{-}CH_2\text{-}CH$); 4.24 (mc, 1H, $CH\text{-}CH_3$); 4.65 (dd, 1H, $O\text{-}CH_2\text{-}CH$); 6.90-7.00 (m, 2H, Aromaten-H); 7.20-7.30 (m, 1H, Aromaten-H); 8.13 (m, 1H, Aromaten-H).

Beispiel 8:

Kalium-(1S,5R,6S)-6-[(1R)-1-hydroxyethyl]-(thiochromano)[3,4-a]carbapen-2-em-3-carboxylat.

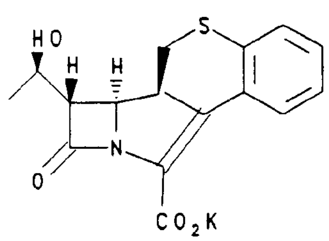

Stufe 1:

(3S,4R)-3-[(1R)-1-tert.-Butyldimethylsilyloxyethyl]-4-[(3R)-4-oxothiochroman-3-yl]azetidin-2-on.

Analog zu Stufe 1 in Beispiel 1 wurden 4.86 g (20 mmol) 3-Bromthiochroman-4-on umgesetzt. Durch Säulenchromatographie (Kieselgel, Laufmittel: Toluol/Ethylacetat = 6/1) konnten beide Isomere getrennt werden. Man isolierte 1.36 g (17 %) des β-Isomeren.-$^1$H-NMR (270 MHz, $CDCl_3$): δ = 0.09 (s, 6H, $SiCH_3$); 0.88 (s, 9H, $SiC(CH_3)_3$); 1.14 (d, 3H, $CH\text{-}CH_3$); 3.01 (mc, 1H, $S\text{-}CH_2\text{-}CH$); 3.12 (dd, 1H, H-3); 3.25-3.31 (m, 2H, $S\text{-}CH_2\text{-}CH$); 4.25 (mc, 1H, $CH\text{-}CH_3$); 4.48 (dd, 1H, H-4); 5.89 (bs, 1H, NH); 7.13-7.45 (m, 3H, Aromaten-H); 8.06 (d, 1H, Aromaten-H).

Stufe 2:

[(3S,4R)-3-[(1R)-1-tert.-Butyldimethylsilyloxyethyl]-4-[(3R)-4-oxothiochroman-3-yl]-2-oxoazetidin-1-yl]-2-oxoessigsäureallylester.

Analog zu Stufe 2 in Beispiel 1 wurden 1.35 g (3.45 mmol) des Azetidinons mit Oxalsäureallylesterchlorid umgesetzt. Nach Säulenchromatographie an Kieselgel (desaktiviert mit 10 % $H_2O$, Laufmittel: Toluol/Ethylacetat= 3/1) erhielt man 1.43 g (82 %) gelben Feststoff.-$^1$H-NMR (270 MHz, $CDCl_3$): $\delta$ = 0.07 (s, 6H, $SiCH_3$); 0.82 (s, 9H, $SiC(CH_3)_3$); 1.04 (d, 3H, $CH$-$CH_3$); 3.31 -3.38 (m, 3H, S-$CH_2$-CH und S-$CH_2$-CH); 3.41 (dd, 1H, H-3); 4.31 (mc, 1H, CH-$CH_3$); 4.81 (mc, 2H, $CH_2$-CH=$CH_2$); 4.92 (mc, 1H, H-4); 5.30-5.47 (m, 2H, $CH_2$-CH=$CH_2$); 5.96 (mc, 1H, $CH_2$-CH=$CH_2$); 7.12-7.42 (m, 3H, Aromaten-H); 8.08 (d, 1H, Aromaten-H).

Stufe 3:

(1S,5R,6S)-3-[(1R)-1-tert.-Butyldimethylsilyloxyethyl]-(thiochromano)[3,4-a]carbapen-2-em-3-carbonsäureallylester.

2.02 g (4.0 mmol) Allylester wurden, wie in Beispiel 1 unter Stufe 3 beschrieben, umgesetzt. Nach Chromatographie an Kieselgel (Laufmittel: Toluol/Ethylacetat = 50/1) wurden 930 mg (49 %) als gelber Feststoff isoliert.-$^1$H-NMR (270 MHz, $CDCl_3$): $\delta$ = 0.10 (s, 6H, $SiCH_3$); 0.90 (s, 9H, $SiC(CH_3)_3$); 1.28 (d, 3H, CH-$CH_3$); 2.98 (dd, 1H, H-6); 3.19-3.38 (m, 3H, S-$CH_2$-CH und S-$CH_2$-CH); 4.28 (mc, 1H, CH-$CH_3$); 4.39 (dd, 1H, H-5); 4.76 (mc, 2H, $CH_2$-CH =$CH_2$); 5.31 (mc, 2H, $CH_2$-CH=$CH_2$); 5.91 (mc, 1H, $CH_2$-CH=$CH_2$); 6.97-7.22 (m, 3H, Aromaten-H); 7.53 (mc, 1H, Aromaten-H).

Stufe 4:

(1S,5R,6S)-6-[(1R)-1-Hydroxyethyl]-(thiochromano)[3,4-a]carbapen-2-em-3-carbonsäureallylester.

Analog zu Stufe 4 in Beispiel 1 wurden 326 mg (0.69 mmol) des Silylethers umgesetzt. Nach Chromatographie (Kieselgel, Laufmittel: Toluol/Ethylacetat= 1/1) wurden 161 mg (65 %) weißer Feststoff erhalten.-$^1$H-NMR (270 MHz, $CDCl_3$): $\delta$ = 1.39 (d, 3H, CH-$CH_3$); 3.02 (dd, 1H, H-6); 3.18-3.47 (m, 3H, S-$CH_2$-CH und S-$CH_2$-CH); 4.29 (mc, 1H, CH-$CH_3$); 4.41 (dd, 1H, H-5); 4.68-4.79 (m, 2H, $CH_2$-CH = $CH_2$); 5.23-5.42 (m, 2H, $CH_2$-CH = $CH_2$); 5.93 (m, 1H, $CH_2$-CH=$CH_2$); 6.98-7.21 (m, 3H, Aromaten-H); 7.53 (mc, 1H, Aromaten-H).

Stufe 5:

Kalium-(1S,5R,6S)-6-[(1R)-1-Hydroxyethyl]-(thiochromano)[3,4-a]carbapen-2-em-3-carboxylat.

Analog zu Stufe 5 in Beispiel 1 wurden 161 mg (0.45 mmol) des Allylesters umgesetzt. Nach Chromatographie (R)Lichroprep RP 18, Laufmittel: Wasser) isolierte man 64 mg (40 %) weißen Feststoff.-$^1$H-NMR (270 MHz, $D_2O$): $\delta$ = 1.31 (d, 3H, CH-$CH_3$); 3.10-3.42 (m, 3H, S-$CH_2$-CH und S-$CH_2$-CH) 3.56 (dd, 1H, H-6); 4.28 (mc, 1H, CH-$CH_3$); 4.43 (dd, 1H, H-5); 7.04-7.10 (m, 1H, Aromaten-H); 7.17-7.24 (m, 2H, Aromaten-H); 7.39 (mc, 1H, Aromaten-H).

Beispiel 9:

Kalium-(1S,5R,6S)-6-[(1R)-1-Acetoxyethyl]-(1,2,3,4-tetrahydronaphtho)[2,1-a]carbapen-2-em-3-carboxylat.

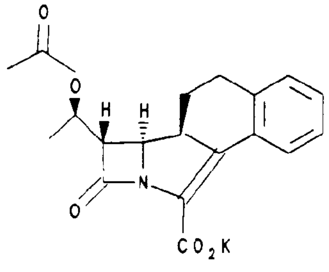

Stufe 1:

(3S,4R)-3-[(1R)-1-Acetoxyethyl]-4-[(R)-1-oxo-1,2,3,4-tetrahydronaphth-2-yl]-azetidin-2-on

Eine Lösung von 2.0 g (5.35 mmol) Silylether aus Beispiel 1/Stufe 1 in 10 ml (10.82 g, 106 mmol) Essigsäureanhydrid wurde bei 0°C portionsweise mit insgesamt 100 mg (0.62 mmol) $FeCl_3$ versetzt und 2.5 h unter Eiskühlung gerührt. Man gab 25 ml Methylenchlorid und 25 ml Wasser zu dieser Mischung, trennte die organische Phase ab und extrahierte die wässrige Phase zweimal mit je 10 ml Methylenchlorid. Nach Waschen der organischen Phase mit ges. $NaHCO_3$-Lösung und Trocknen über $MgSO_4$ wurde das Rohprodukt an Kieselgel (Laufmittel: Toluol/Ethylacetat = 2/1) chromatographiert. Ausbeute: 1.56 g (97 %). $^1$H-NMR (270 MHz, $CDCl_3$): $\delta$ = 1.42 (d, 3H, CH-$CH_3$); 1.94-2.11 (m, 1H, $CH_2$-$CH_2$-CH); 2.03 (s, 3H, $COCH_3$); 2.22-2.34 (m, 1H, $CH_2$-$CH_2$-CH); 2.73 (mc, 1H, $CH_2$-$CH_2$-CH); 3.11 (dd, 2H, $CH_2$-$CH_2$-CH); 3.20 (dd, 1H, H-3); 4.19 (dd, 1H, H-4); 5.33 (mc, 1H, CH-$CH_3$); 6.08 (bs, 1H, NH); 7.23-7.37 (m, 2H, Aromaten-H); 7.52 (mc, 1H, Aromaten-H); 8.01 (d, 1H, Aromaten-H).

Stufe 2:

[(3S,4R)-3-[(1R)-1-Acetoxyethyl]-4-[(2R)-1-oxo-1,2,3,4-tetrahydronaphth-2-yl]-2-oxoazetidin -1-yl]-2-oxoessigsäureallylester.

Analog zu Stufe 2 in Beispiel 1 wurden 1.45 g (4.8 mmol) des Azetidinons mit Oxalsäureallylesterchlorid umgesetzt. Nach Säulenchromatographie an Kieselgel (Laufmittel: Toluol/Ethylacetat = 30/1) wurden 1.62 g (81 %) Produkt isoliert.-$^1$H-NMR (270 MHz, $CDCl_3$): $\delta$ = 1.37 (d, 3H, CH-$CH_3$); 2.02-2.15 (m, 1H, $CH_2$-$CH_2$-CH); 2.20-2.30 (m, 1H, $CH_2$-$CH_2$-CH); 3.09-3.47 (m, 3H, $CH_2$-$CH_2$-CH); 3.51 (dd, 1H, H-3); 4.49 (dd, 1H, H-4); 4.85 (mc, 2H, $CH_2$-CH =$CH_2$); 5.30-5.48 (m, 2H, $CH_2$-CH =$CH_2$); 5.89-6.06 (m, 1H, $CH_2$-CH=$CH_2$ und CH-$CH_3$); 7.32-7.37 (m, 2H, Aromaten-H); 7.47-7.54 (m, 1H, Aromaten-H); 8.05 (d, 1H, Aromaten-H).

Stufe 3:

(1S,5R,6S)-6-[(1R)-1-Acetoxyethyl]-(1,2,3,4-tetrahydronaphtho)[2,1-a]carbapen-2-em-3-carbonsäureallylester.

245 mg (0.59 mmol) Allylester wurden wie in Beispiel 1 unter Stufe 3 beschrieben umgesetzt. Nach Chromatographie an Kieselgel (Laufmittel: Toluol/Ethylacetat = 30/ 1 ) wurden 55 mg (24 %) Produkt als Öl isoliert.-$^1$H-NMR (270 MHz, $CDCl_3$): $\delta$ = 1.41 (d, 3H, CH-$CH_3$); 1.85-2.16 (m, 2H, $CH_2$-$CH_2$-CH); 2.07 (s, 3H, $COCH_3$); 3.04-3.11 (m, 2H, $CH_2$-$CH_2$-CH); 3.20 (mc, 1H, $CH_2$-$CH_2$-CH); 3.47 (dd, 1H, H-6); 4.30 (dd, 1H, H-5); 4.79 (mc, 2H, $CH_2$-CH =$CH_2$); 5.21-5.46 (m, 3H, $CH_2$-CH =$CH_2$ und CH-$CH_3$), 5.91-6.08 (m, 1H, $CH_2$-CH=$CH_2$); 7.11-7.29 (m, 3H, Aromaten-H); 7.78 (d, 1H, Aromaten-H).

Stufe 4:

Kalium-(1S,5R,6S)-6-[(1R)-1-Acetoxyethyl]-(1,2,3,4-tetrahydronaphtho)[2,1-a]carbapen-2-em-3-carboxylat.

Wie im Beispiel 1 unter Stufe 5 beschrieben wurden 118 mg (0.31 mmol) des Allylesters umgesetzt. Das Kaliumsalz wurde über $^{(R)}$LiChroprep RP18 (Laufmittel: $H_2O$, anschließend Gradient bis 15 % $CH_3CN$) gereinigt. Ausbeute: 49 mg (41 %).-$^1$H-NMR (270 MHz, $D_2O$): $\delta$ = 1.37 (d, 3H, CH-$CH_3$); 1.74-1.97 (m, 1H, CH-$CH_2$-$CH_2$); 2.05-2.23 (m, 1H, CH-$CH_2$-$CH_2$); 2.14 (s, 3H, $COCH_3$); 2.98-3.06 (m, 2H, CH-$CH_2$-$CH_2$); 3.23 (mc, 1H, CH-$CH_2$-$CH_2$); 3.55 (dd, 1H, H-6); 4.41 (dd, 1H, H-5); 5.30 (mc, 1H, CH-$CH_3$); 7.13-7.28 (m, 3H, Aromaten-H); 7.47 (d, 1H, Aromaten-H).

Beispiel 10

Natrium-(1R,5R,6S)-6-[(1R)-1-hydroxyethyl]-(7-brom-1,2,3,4-tetrahydronaphtho)[2,1-a]carbapen-2-em-3-carboxylat.

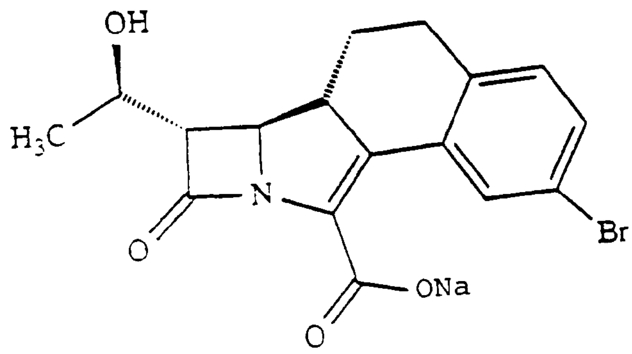

Vorstufe:

2,7-Dibrom-1-tetralon.

34.8 g (155 mmol) 7-Brom-1-tetralon wurden in 775 ml trockenem Diethylether gelöst. Man tropfte 8.0 ml (24.8 g, 155 mmol) Brom innerhalb von 20 Minuten zu. Bereits bei der Zugabe des ersten Tropfens erfolgte sofortige Entfärbung. Es wurde 1 h bei Zimmertemperatur nachgerührt und anschließend mit 9 %iger Natriumbicarbonat-Lösung und gesättigter Kochsalzlösung ausgeschüttelt. Nach Trocknen über Magnesiumsulfat wurde das Lösungsmittel am Rotationsverdampfer abgezogen und der ölige Rückstand an der Ölpumpe bis zur Gewichtskonstanz getrocknet. Übers Wochenende erhielt man im Kühlschrank Kristalle, die in Ethanol verrieben und abgesaugt wurden. Nach Trocknen im Vakuumexsikkator über Phosphorpentoxid erhielt man 30.6 g (65 %) braune Kristalle. Schmp. 76-77°C.-$^1$H-NMR (60 MHz, $CDCl_3$): δ= 2.30-2.60 und 2.80-3.30 (2 x m, 2 x 2H, $CH_2$-$CH_2$); 4.80 (mc, 1H, CHBr); 7.20 (d, 1H, Aromaten-H); 7.70 (dd, 1H, Aromaten-H); 8.31 (d, 1H, Aromaten-H).

Stufe 1:

(3S,4R)-3-[(1R)-1-tert.-Butyldimethylsilyloxyethyl]-4-[(2S)-7-brom-1-oxo-1,2,3,4-tetrahydronaphth-2-yl]-azetidin-2-on.

Wie für die Stufe 1 im Beispiel 1 beschrieben wurde, setzte man 5.0 g (16.4 mmol) 2,7-Dibrom-1-tetralon um. Nach Chromatographie an Kieselgel (Laufmittel: Toluol/Ethylacetat = 5/1) erhielt man 1.37 g (25 %) Produkt.-$^1$H-NMR (270 MHz, $CDCl_3$): δ= 0.07 und 0.09 (2 x s, 6H, $SiCH_3$); 0.89 (s, 9H, $SiC(CH_3)_3$); 1.28 (d, 3H, CH-$CH_3$); 1.70-2.20 (m, 1H, $CH_2$-$CH_2$-CH); 2.30 (mc, 1H, $CH_2$-$CH_2$-CH); 2.53 (mc, 1H, $CH_2$-$CH_2$-CH); 2.84 (mc, 1H, $CH_2$-$CH_2$-CH); 2.95-3.10 (m, 2H, $CH_2$-$CH_2$-CH und H-3); 3.71 (dd, 1H, H-4); 4.20 (mc, 1H, CH-$CH_3$); 6.42 (s, 1H, NH); 7.16 (d, 1H, Aromaten-H); 7.60 (dd, 1H, Aromaten-H); 8.12 (d, 1H, Aromaten-H).

Stufe 2:

[(3S,4R)-3-[(1R)-1-tert.-Butyldimethylsilyloxyethyl]-4-[(2S)-7-brom-1-oxo-1,2,3,4-tetrahydronaphth-2-yl]-2-oxoazetidin-1-yl]-2-oxoessigsäureallylester.

Analog zur Stufe 2 im Beispiel 1 synthetisierte man aus 790 mg (1.75 mmol) Azetidin-2-on 800 mg (81 %) Allylester.-$^1$H-NMR (270 MHz, $CDCl_3$): δ= 0.04 und 0.10 (2 x s, 6H, $SiCH_3$); 0.88 (s, 9H, $SiC(CH_3)_3$); 1.29 (d, 3H, CH-$CH_3$); 1.90-2.10 (m, 1H, CH-$CH_2$-CH); 2.20-2.35 (m, 1H, $CH_2$-$CH_2$-CH); 2.95-3.10 (m, 2H, $CH_2$-$CH_2$-CH); 3.17 (mc, 1H, H-3); 3.49 (mc, 1H, $CH_2$-$CH_2$-CH); 4.35 (mc, 1H, CH-$CH_3$); 4.82 (mc, 2H, $CH_2$-CH =$CH_2$); 5.30-5.50 (m, 2H, $CH_2$-CH=$CH_2$); 5.85-6.10 (m, 1H, $CH_2$-CH=$CH_2$); 7.18 (d, 1H, Aromaten-H); 7.60 (dd, 1H, Aromaten-H); 8.15 (d, 1H, Aromaten-H).

Stufe 3:

(1R,5R,6S)-6-[(1R)-1-tert.-Butyldimethylsilyloxyethyl]-(7-brom-1,2,3,4-tetrahydronaphtho)[2,1-a]carbapen-2-em-3-carbonsäureallylester.

Die Zyklisierung von 800 mg (1.42 mmol) Allylester wurde wie im Beispiel 1 beschrieben durchgeführt. Nach 45 Minuten bei 160°C wurde aufgearbeitet. Nach der Chromatographie erhielt man 290 mg (38 %) Produkt.-$^1$H-NMR (270 MHz, $CDCl_3$): δ= 0.10 und 0.11 (2 x s, 6H, $SiCH_3$); 0.90 (s, 9H, $SiC(CH_3)_3$); 1.28 (d, 3H, $CH\text{-}CH_3$); 1.80-2.00 (m, 1H, $CH_2\text{-}CH_2\text{-}CH$); 2.15-2.30 (m, 1H, $CH_2\text{-}CH_2\text{-}CH$); 2.87-3.00 (m, 2H, $CH_2\text{-}CH_2\text{-}CH$); 3.19 (dd, 1H, H-6); 3.26 (mc, 1H, $CH_2\text{-}CH_2\text{-}CH$); 3.79 (dd, 1H, H-5); 4.22 (mc, 1H, $CH\text{-}CH_3$); 4.70-4.90 (m, 2H, $CH_2\text{-}CH{=}CH_2$);
5.22-5.55 (m, 2H, $CH_2\text{-}CH{=}CH_2$); 5.90-6.10 (m, 1H, $CH_2\text{-}CH{=}CH_2$); 7.02 (d, 1H, Aromaten-H); 7.38 (dd, 1H, Aromaten-H); 8.70 (d, 1H, Aromaten-H).

Stufe 4:

(1R,5R,6S)-6-[(1R)-1-Hydroxyethyl]-(7-brom-1,2,3,4-tetrahydronaphtho)[2,1-a]carbapen-2-em-3-carbonsäureallylester.

Aus 280 mg (0.53 mmol) Silylether stellte man analog zur Stufe 4 im Beispiel 1 die Hydroxyethylverbindung dar. Nach Säulenchromatographie an Kieselgel erhielt man 160 mg (73 %).-$^1$H-NMR (270 MHz, $CDCl_3$): δ= 1.39 (d, 3H, $CH\text{-}CH_3$); 1.84-2.00 (m, 1H, $CH_2\text{-}CH_2\text{-}CH$); 2.20-2.40 (m, 1H, $CH_2\text{-}CH_2\text{-}CH$); 2.88-3.00 (m, 2H, $CH_2\text{-}CH_2\text{-}CH$); 3.20-3.35 (m, 2H, $CH_2\text{-}CH_2\text{-}CH$ und H-6); 3.83 (dd, 1H, H5); 4.26 (mc, 1H, $CH\text{-}CH_3$); 4.70-4.95 (m, 2H, $CH_2\text{-}CH{=}CH_2$); 5.23-5.55 (m, 2H, $CH_2\text{-}CH{=}CH_2$); 5.90-6.10 (m, 1H, $CH_2\text{-}CH{=}CH_2$); 7.04 (d, 1H, Aromaten-H); 7.40 (dd, 1H, Aromaten-H); 8.66 (d, 1H, Aromaten-H).

Stufe 5:

Natrium-(1R,5R,6S)-6-[(1R)-1-hydroxyethyl]-(7-brom-1,2,3,4-tetrahydronaphtho)[2,1-a]carbapen-2-em-3-carboxylat.

Aus 160 mg (0.38 mmol) Allylester erhielt man 40 mg (26 %) Natriumsalz.-$^1$H-NMR (270 MHz, $D_2O$): δ= 1.33 (d, 3H, $CH\text{-}CH_3$); 1.60-1.85 (m, 1H, $CH_2\text{-}CH_2\text{-}CH$); 2.20-2.35 (m, 1H, $CH_2\text{-}CH_2\text{-}CH$); 2.70-3.00 (m, 2H, $CH_2\text{-}CH_2\text{-}CH$); 3.25-3.40 (m, 1H, $CH_2\text{-}CH_2\text{-}CH$); 3.48 (dd, 1H, H-6); 3.87 (dd, 1H, H-5); 4.25 (mc, 1H, $CH\text{-}CH_33$); 7.13 (d, 1H, Aromaten-H); 7.38 (dd, 1H, Aromaten-H); 8.28 (d, 1H, Aromaten-H).

Beispiel 11

Natrium-(1S,5R,6S)-6-[(1R)-1-hydroxyethyl]-(7-chlor-1,2,3,4-tetrahydronaphtho)[2,1-a]carbapen-2-em-3-carboxylat.

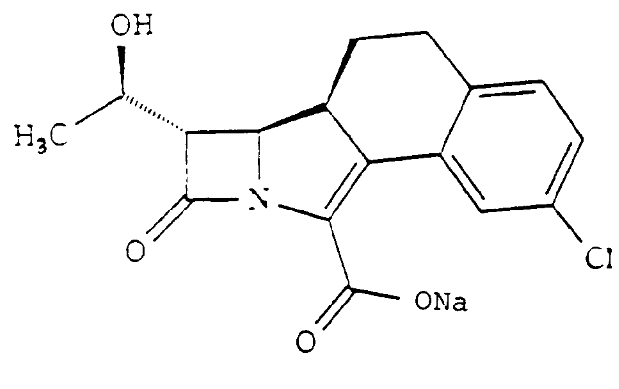

Vorstufe:

2-Brom-7-chlor-1-tetralon

Wie für die Vorstufe im Beispiel 10 beschrieben wurde, setzte man 10 g (60mmol) 7-Chlor-1-tetralon um. Man erhielt das Produkt in Form wachsartiger Kristalle. Ausbeute: 15.4 g (98 %).-$^1$H-NMR (270 MHz, $CDCl_3$): δ= 2.30-2.60 (m, 2H, $CH_2$-$CH_2$); 2.80-3.00 (m, 1H, $CH_2$-$CH_2$); 3.20-3.40 (m, 1H, $CH_2$-$CH_2$); 4.72 (t, 1H, CHBr); 7.25 (d, 1H, Aromaten-H); 7.49 (dd, 1H, Aromaten-H); 8.06 (d, 1H, Aromaten-H).

Stufe 1:

(3S,4R)-3-[(1R)-1-tert.-Butyldimethylsilyloxyethyl]-4-[(2R)-7-chlor-1-oxo-1,2,3,4-tetrahydronaphth-2-yl]-azetidin-2-on.

Wie für die Stufe 1 im Beispiel 1 beschrieben wurde, setzte man 15.4 g (59 mmol) 2-Brom-7-chlor-1-tetralon um. Nach Chromatographie an Kieselgel (Laufmittel: Toluol/Ethylacetat = 5/1) erhielt man 5.50 g (30 %) Produkt.-$^1$H-NMR (270 MHz, $CDCl_3$): δ= 0.08 und 0.09 (2 x s, 6H, $SiCH_3$); 0.86 (s, 9H, $SiC(CH_3)_3$); 1.28 (d, 3H, CH-$CH_3$); 1.90-2.15 (m, 1H, $CH_2$-$CH_2$-CH); 2.20-2.38 (m, 1H, $CH_2$-$CH_2$-CH); 2.65-2.80 (m, 1H, $CH_2$-$CH_2$-CH); 3.00-3.18 (m, 3H, $CH_2$-$CH_2$-CH und H-3); 4.26 (mc, 1H, CH-$CH_3$); 4.42 (dd, 1H, H-4); 5.74 (bs, 1H, NH); 7.23 (d, 1H, Aromaten-H); 7.46 (dd, 1H, Aromaten-H); 7.98 (d, 1H, Aromaten-H).

Stufe 2:

[(3S,4R)-3-[(1R)-1-tert.-Butyldimethylsilyloxyethyl]-4-[(2R)-7-chlor-1-oxo-1,2,3,4-tetrahydronaphth-2-yl]-2-oxoazetidin-1-yl]-2-oxoessigsäureallylester.

Analog zur Stufe 2 im Beispiel 1 synthetisierte man aus 3.10 g (7.60 mmol) Azetidin-2-on 3.8 g (96 %) Allylester.-$^1$H-NMR (270 MHz, $CDCl_3$): δ= 0.06 und 0.08 (2 x s, 6H, $SiCH_3$); 0.87 (s, 9H, $SiC(CH_3)_3$); 1.20 (d, 3H, CH-$CH_3$); 1.90-2.10 (m, 1H, $CH_2$-$CH_2$-CH); 2.20-2.33 (m, 1H, $CH_2$-$CH_2$-CH); 3.00-3.23 (m, 3H, $CH_2$-$CH_2$-CH); 3.29 (t, 1H, H-3); 4.33 (mc, 1H, CH-$CH_3$); 4.79 (t, 1H, H-4); 4.80 (mc, 2H, $CH_2$-CH=$CH_2$); 5.28-5.50 (m, 2H, $CH_2$-CH=$CH_2$); 5.90-6.10 (m, 1H, $CH_2$-CH=$CH_2$); 7.22 (d, 1H, Aromaten-H); 7.46 (dd, 1H, Aromaten-H); 8.01 (d, 1H, Aromaten-H).

Stufe 3:

(1S,5R,6S)-6-[(1R)-1-tert.-Butyldimethylsilyloxyethyl]-(7-chlor-1,2,3,4-tetrahydronaphtho)[2,1-a]carbapen-2-em-3-carbonsäureallylester.

Die Zyklisierung von 3.80 g (7.3 mmol) Allylester wurde wie in Beispiel 1 beschrieben durchgeführt. Nach 45 Minuten bei 160°C wurde aufgearbeitet. Nach der Chromatographie erhielt man 1.50 g (42 %) Produkt.-$^1$H-NMR (270 MHz, $CDCl_3$): δ= 0.10 (s, 6H, $SiCH_3$); 0.90 (s, 9H, $SiC(CH_3)_3$); 1.28 (d, 3H, CH-$CH_3$); 1.80-2.15 (m, 2H, $CH_2$-$CH_2$-CH); 2.95-3.08 (m, 2H, $CH_2$-$CH_2$-CH); 3.17 (mc, 1H, $CH_2$-$CH_2$-CH); 3.30 (dd, 1H, H-6); 4.20-4.38 (m, 2H, CH-$CH_3$ und H-5); 4.65-4.95 (m, 2H, $CH_2$-CH=$CH_2$); 5.20-5.50 (m, 2H, $CH_2$-CH=$CH_2$); 5.90-6.10 (m, 1H, $CH_2$-CH=$CH_2$); 7.06 (d, 1H, Aromaten-H); 7.19 (dd, 1H, Aromaten-H); 7.78 (d, 1H, Aromaten-H).

Stufe 4:

(1S,5R,6S)-6-[(1R)-1-Hydroxyethyl]-(7-chlor-1,2,3,4-tetrahydronaphtho)[2,1-a]carbapen-2-em-3-carbonsäureallylester.

Aus 1.5 g (3.07 mmol) Silylether stellte man analog zur Stufe 4 im Beispiel 1 die Hydroxyethylverbindung dar. Nach Säulenchromatographie an Kieselgel erhielt man 200 mg (17 %).-$^1$H-NMR (270 MHz, $CDCl_3$): δ= 1.37 (d, 3H, CH-$CH_3$); 1.80-2.00 (m, 1H, $CH_2$-$CH_2$-CH); 2.05-2.20 (m, 1H, $CH_2$-$CH_2$-CH); 2.90-3.10 (m, 2H, $CH_2$-$CH_2$-CH); 3.10-3.28 (m, 1H, $CH_2$-$CH_2$-CH); 3.32 (dd, 1H, H-6); 4.28 (mc, 1H, CH-$CH_3$); 4.35 (dd, 1H, H-5); 4.60-4.95 (m, 2H, $CH_2$-CH=$CH_2$); 5.20-5.50 (m, 2H, $CH_2$-CH=$CH_2$); 5.90-6.10 (m, 1H, $CH_2$-CH=$CH_2$); 7.07 (d, 1H, Aromaten-H); 7.21 (dd, 1H, Aromaten-H); 7.77 (d, 1H, Aromaten-H).

Stufe 5:

Natrium-(1S,5R,6S)-6-[(1R)-1-hydroxyethyl]-(7-chlor-1,2,3,4-tetrahydronaphto)[2,1-a]carbapen-2-em-3-carboxylat.

Aus 35 mg (0.094 mmol) Allylester erhielt man 30 mg Natriumsalz. -$^1$H-NMR (270 MHz, $D_2O$): δ= 1.31 (d, 3H, $CH-CH_3$); 1.70-1.95 (m, 1H, $CH_2-CH_2-CH$); 2.50-2.70 (m, 1H, $CH_2-CH_2-CH$); 2.90-3.10 (m, 2H, $CH_2-CH_2-CH$); 3.27 (mc, 1H, $CH_2-CH_2-CH$); 3.60 (dd, 1H, H-6); 4.29 (mc, 1H, $CH-CH_3$); 4.40 (dd, 1H, H-5); 7.20 (d, 1H, Aromaten-H); 7.12 (dd, 1H, Aromaten-H); 7.50 (d, 1H, Aromaten-H).

Beispiel 12

Natrium-(1R,5R,6S)-6-[(1R)-1-hydroxyethyl]-(7-chlor-1,2,3,4-tetrahydronaphtho)[2,1-a]carbapen-2-em-3-carboxylat.

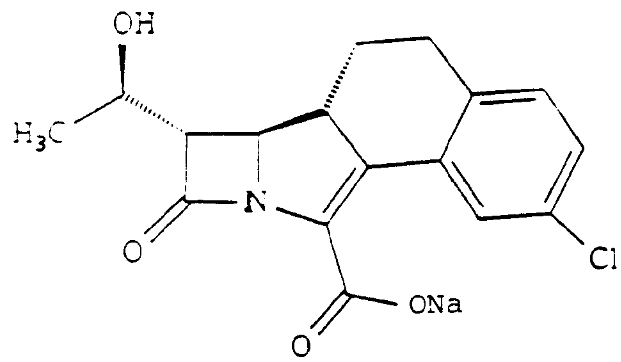

Stufe 1:

(3S,4R)-3-[(1R)-1-tert.-Butyldimethylsilyloxyethyl]-4-[(2S)-7-chlor-1-oxo-1,2,3,4-tetrahydronaphth-2-yl]-azetidin-2-on.

Wie für die Stufe 1 im Beispiel 1 beschrieben wurde, setzte man 15.4 g (59 mmol) 2-Brom-7-chlor-1-tetralon um. Nach Chromatographie an Kieselgel (Laufmittel: Toluol/Ethylacetat = 5/1) erhielt man 6.15g (34%) Produkt.-$^1$H-NMR (270 MHz, $CDCl_3$): δ= 0.08 und 0.10 (2 x s, 6H, $SiCH_3$); 1.29 (d, 3H, $CH-CH_3$); 1.77-2.00 (m, 1H, $CH_2-CH_2-CH$); 2.20-2.40 (m, 1H, $CH_2-CH_2-CH$); 2.47-2.60 (m, 1H, $CH_2-CH_2-CH$); 2.85 (mc, 2H, $CH_2-CH_2-CH$); 2.98-3.15 (m, 2H, $CH_2-CH_2-CH$ und H-3); 3.71 (dd, 1H, H-4); 4.20 (mc, 1H, $CH-CH_3$); 6.42 (bs, 1H, NH); 7.23 (d, 1H, Aromaten-H); 7.47 (dd, 1H, Aromaten-H); 7.96 (d, 1H, Aromaten-H).

Stufe 2:

[(3S,4R)-3-[(1R)-1-tert.-Butyldimethylsilyloxyethyl]-4-[(2S)-7-chlor-1-oxo-1 ,2,3,4-tetrahydronaphth-2-yl]-2-oxoazetidin-1-yl]-2-oxoessigsäureallylester.

Analog zur Stufe 2 im Beispiel 1 synthetisierte man aus 6.5 g (15.9 mmol) Azetidin-2-on 4.92 g (59%) Allylester.-$^1$H-NMR (270 MHz, $CDCl_3$): δ= 0.04 und 0.10 (2 x s, 6H, $SiCH_3$); 0.90 (s, 9H, $SiC(CH_3)_3$); 1.29 (d, 3H, $CH-CH_3$); 1.92-2.10 (m, 1H, $CH_2-CH_2-CH$); 2.21-2.35 (m, 1H, $CH_2-CH_2-CH$); 3.04 (mc, 2H, $CH_2-CH_2-CH$); 3.18 (mc, 1H, H-3); 3.50 (mc, 1H, $CH_2-CH_2-CH$); 4.36 (mc, $CH-CH_3$); 4.81 (mc, 2H, $CH_2-CH=CH_2$); 5.11 (mc, 1H, H-4); 5.30-5.50 (m, 2H, $CH_2-CH=CH_2$); 5.90-6.10 (m, 1H, $CH_2-CH=CH_2$); 7.22 (d, 1H, Aromaten-H); 7.47 (dd, 1H, Aromaten-H); 7.98 (d, 1H, Aromaten-H).

Stufe 3:

(1R,5R,6S)-6-[(1R)-1-tert.-Butyldimethylsilyloxyethyl]-(7-chlor-1,2,3,4-tetrahydronaphtho)[2,1-a]carbapen-2-em-3-carbonsäureallylester.

Die Zyklisierung von 4.92 g (9.46 mmol) Allylester wurde wie im Beispiel 1 beschrieben durchgeführt. Nach

45 Minuten bei 160°C wurde aufgearbeitet. Nach Chromatographie erhielt man 2.1 g (46 %) Produkt.-$^1$H-NMR (270 MHz, $CDCl_3$): δ = 0.10 und 0.11 (2 x s, 6H, $SiCH_3$); 0.92 (s, 9H, $SiC(CH_3)_3$); 1.29 (d, 3H, $CH\text{-}CH_3$); 1.80-2.00 (m, 1H, $CH_2\text{-}CH_2\text{-}CH$); 2.17-2.30 (m, 1H, $CH_2\text{-}CH_2\text{-}CH$); 2.90-3.02 (m, 2H, $CH_2\text{-}CH_2\text{-}CH$); 3.20 (dd, 1H, H-6); 3.80 (dd, 1H, H-5); 4.23 (mc, 1H, $CH\text{-}CH_3$); 4.78 (mc, 2H, $CH_2\text{-}CH{=}CH_2$); 5.23 -5.56 (m, 2H, $CH_2\text{-}CH{=}CH_2$); 5.90-6.10 (m, 1H, $CH_2\text{-}CH{=}CH_2$); 7.10 (d, 1H, Aromaten-H); 7.25 (dd, 1H, Aromaten-H); 8.58 (d, 1H, Aromaten-H).

Stufe 4:

(1R,5R,6S)-6-[(1R)-1-Hydroxyethyl]-(7-chlor-1,2,3,4-tetrahydronaphtho)[2,1-a]carbapen-2-em-3-carbonsäureallylester.

Aus 0.98 g (2 mmol) Silylether stellte man analog zur Stufe 4 im Beispiel 1 die Hydroxyethylverbindung dar. Nach Säulenchromatographie an (R)LiChrosoprep RP18 (Laufmittel/: Acetonitril/Wasser = 9/1) erhielt man 440 mg (59 %).-$^1$H-NMR (270 MHz, $CDCl_3$): δ= 1.38 (d, 3H, $CH\text{-}CH_3$); 1.82-1.98 (m, 1H, $CH_2\text{-}CH_2\text{-}CH$), 2.22-2.35 (m, 1H, $CH_2\text{-}CH_2\text{-}CH$); 2.87-3.02 (m, 2H, $CH_2\text{-}CH_2\text{-}CH$); 3.27 (dd, 1H, H-6); 3.28-3.37 (m, 1H, $CH_2\text{-}CH_2\text{-}CH$); 3.83 (dd, 1H, H-5); 4.28 (mc, 1H, $CH\text{-}CH_3$); 4.81 (mc, 2H, $CH_2\text{-}CH{=}CH_2$); 5.20-5.52 (m, 2H, $CH_2\text{-}CH{=}CH_2$); 5.90-6.12 (m, 1H, $CH_2\text{-}CH{=}CH_2$); 7.09 (d, 1H, Aromaten-H); 7.24 (dd, 1H, Aromaten-H); 8.52 (d, 1H, Aromaten-H).

Stufe 5:

Natrium-(1R,5R,6S)-6-[(1R)-1-hydroxyethyl]-(7-chlor-1,2,3,4-tetrahydronaphtho)[2,1-a]carbapen-2-em-3-carboxylat.

Aus 200 mg (0.54 mmol) Allylester erhielt man 85 mg (44 %) Natriumsalz. - $^1$H-NMR (270 MHz, $D_2O$): δ= 1.32 (d, 3H, $CH\text{-}CH_3$); 1.71 (mc, 1H, $CH_2\text{-}CH_2\text{-}CH$), 2.20-2.31 (m, 1H, $CH_2\text{-}CH_2\text{-}CH$); 2.82-2.96 (m, 2H, $CH_2\text{-}CH_2\text{-}CH$); 3.32 (mc, 1H, $CH_2\text{-}CH_2\text{-}CH$); 3.48 (dd, 1H, H-6); 3.85 (dd, 1H, H-5); 4.24 (mc, 1H, $CH\text{-}CH_3$); 7.12 - 7.28 (m, 2H, Aromaten-H); 8.08 (d, 1H, Aromaten-H).

Beispiel 13

Natrium-(1S,5R,6S)-6-[(1R)-1-hydroxyethyl]-(5,7-dimethyl-1,2,3,4-tetrahydronaphtho)[2,1-a]carbapen-2-em-3-carboxylat.

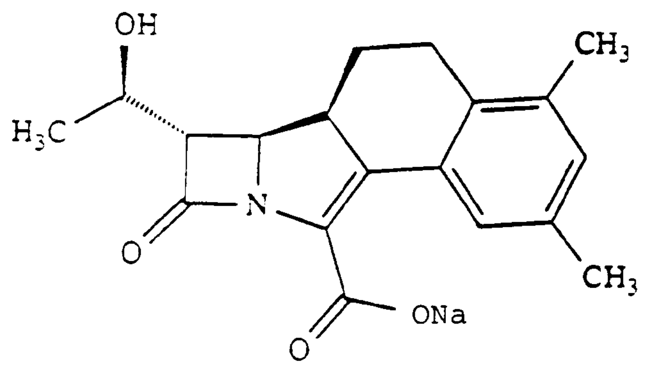

Vorstufe:

2-Brom-5,7-dimethyl-1-tetralon.

Wie für die Vorstufe im Beispiel 10 beschrieben wurde, setzte man 52 g (298 mmol) 5,7-Dimethyl-1-tetralon um. Man erhielt das Produkt nach dem Verreiben in Petrolether in kristallisierter Form. Ausbeute: 62.2 g (83%). Schmp.: 79-80°C-$^1$H-NMR (270 MHz, $CDCl_3$): δ= 2.30 (s, 3H, $CH_3$); 2.34 (s, 3H, $CH_3$); 2.43-2.58 (m, 2H, $CH_2\text{-}CH_2$); 2.83 (mc, $^1$H, $CH_2\text{-}CH_2$); 2.95-3.13 (m, 1H, $CH_2\text{-}CH_2$); 4.70 (t, 1H, CHBr); 7.23 (s, 1H, Aromaten-H); 7.79 (s, 1H, Aromaten-H).

Stufe 1:

(3S,4R)-3-[(1R)-1-tert.-Butyldimethylsilyloxyethyl]-4-[(2R)-5,7-dimethyl-1-oxo-1,2,3,4-tetrahydronaphth-2-yl]-azetidin-2-on.

Wie für die Stufe 1 im Beispiel 1 beschrieben wurde, setzte man 8.2 g (32.8 mmol) 2-Brom-5,7-dimethyl-1-tetralon um. Nach Chromatographie an Kieselgel (Laufmittel: Toluol/Ethylacetat = 5/1) erhielt man 2.53 g (26%) Produkt.-$^1$H-NMR (270 MHz, $CDCl_3$): δ= 0.90 und 0.91 (2 x s, 6H, $SiCH_3$); 0.97 (s, 9H, $SiC(CH_3)_3$); 1.28 (d, 3H, $CH$-$CH_3$); 1.90-2.15 (m, 1H, $CH_2$-$CH_2$-CH); 2.25-2.60 (m, 1H, $CH_2$-$CH_2$-CH); 2.29 (s, 3H, $CH_3$); 2.33 (s, 3H, $CH_3$); 2.60-2.90 (m, 2H, $CH_2$-$CH_2$-CH); 2.95-3.12 (m, 2H, $CH_2$-$CH_2$-CH und H-3); 4.24 (mc, 1H, CH-$CH_3$); 4.40 (dd, 1H, H-4); 5.72 (bs, 1H, NH); 7.20 (s, 1H, Aromaten-H); 7.69 (s, 1H, Aromaten-H).

Stufe 2:

[(3S,4R)-3-[(1R)-1-tert.-Butyldimethylsilyloxyethyl]-4-[(2R)-5,7-dimethyl-1-oxo-1,2,3,4-tetrahydronaphth-2-yl-]-2-oxoazetidin-1-yl]-2-oxoessigsäureallylester.

Analog zur Stufe 2 im Beispiel 1 synthetisierte man aus 1.54 g (3.84 mmol) Azetidin-2-on 1.9 g (96%) Allylester.-$^1$H-NMR (270 MHz, $CDCl_3$): δ= 0.04 und 0.06 (2 x s, 6H, $SiCH_3$); 0.87 (s, 9H, $SiC(CH_3)_3$); 1.19 (d, 3H, CH-$CH_3$); 1.90-2.10 (m, 1H, $CH_2$-$CH_2$-CH); 2.20-2.29 (m, 1H, $CH_2$-$CH_2$-CH); 2.29 (s, 3H, $CH_3$); 2.32 (s, 3H, $CH_3$); 2.70-2.90 (m, 2H, $CH_2$-$CH_2$-CH); 3.00-3.27 (m, 2H, $CH_2$-$CH_2$-CH); 3.42 (t, 1H, H-3); 4.32 (mc, 1H, CH-$CH_3$); 4.62 (dd, 1H, H-4); 4.81 (mc, 2H, $CH_2$-CH=$CH_2$); 5.25-5.50 (m, 1H, $CH_2$-CH=$CH_2$); 5.87-6.10 (m, 1H, $CH_2$-CH=$CH_2$); 7.18 (s, 1H, Aromaten-H); 7.70 (s, 1H, Aromaten-H).

Stufe 3:

(1S,5R,6S)-6-[(1R)-1-tert.-Butyldimethylsilyloxyethyl]-(5,7-dimethyl-1,2,3,4-tetrahydronaphtho)[2,1-a]carbapen-2-em-3-carbonsäureallylester.

Die Zyklisierung von 1.9 g (3.7 mmol) Allylester wurde wie in Beispiel 1 beschrieben durchgeführt. Nach 60 Minuten bei 160°C wurde aufgearbeitet. Nach der Chromatographie erhielt man 790 mg (44 %) Produkt.-$^1$H-NMR (270 MHz, $CDCl_3$): δ= 0.10 (s, 6H, $SiCH_3$); 0.90 (s, 6H, $SiC(CH_3)_3$); 1.80-2.00 (m, 1H, $CH_2$-$CH_2$-CH); 2.10-2.18 (m, 1H, $CH_2$-$CH_2$-CH); 2.19 (s, 3H, $CH_3$); 2.27 (s, 3H, $CH_3$); 2.64-2.82 (m, 1H, $CH_2$-$CH_2$-CH); 2.92 (mc, 1H, $CH_2$-$CH_2$-CH); 3.09 (mc, 1H, $CH_2$-$CH_2$-CH); 3.27 (dd, 1H, H-6); 4.15-4.35 (mc, 2H, CH-$CH_3$ und H-5); 4.77 (mc, 2H, $CH_2$-CH=$CH_2$); 5.32 (mc, 2H, $CH_2$-CH=$CH_2$); 5.88-6.09 (m, 1H, $CH_2$-CH=$CH_2$); 6.95 (s, 1H, Aromaten-H); 7.35 (s, 1H, Aromaten-H).

Stufe 4:

(1S,5R,6S)-6-[(1R)-1-Hydroxyethyl]-(5,7-dimethyl-1,2,3,4-tetrahydronaphtho)[2,1-a]carbapen-2-em-3-carbonsäureallylester.

Aus 790 mg (1.64 mmol) Silylether stellte man analog zur Stufe 4 im Beispiel 1 die Hydroxyethylverbindung dar. Nach Säulenchromatographie an Kieselgel erhielt man 300 mg (50 %).-$^1$H-NMR (270 MHz, $CDCl_3$): δ= 1.48 (d, 3H, CH-$CH_3$); 1.77-2.00 (m, 2H, $CH_2$-$CH_2$-CH); 2.20 (s, 3H, $CH_3$); 2.28 (s, 3H, $CH_3$); 2.65-2.83 (m, 2H, $CH_2$-$CH_2$-CH); 2.92 (mc, 1H, $CH_2$-$CH_2$-CH); 3.14 (mc, 1H, $CH_2$-$CH_2$-CH); 3.31 (dd, 1H, H-6); 4.20-4.40 (m, 2H, CH-$CH_3$ und H-5); 4.78 (mc, 2H, $CH_2$-CH=$CH_2$); 5.33 (mc, 2H, $CH_2$-CH=$CH_2$); 5.88-6.10 (m, 1H, $CH_2$-CH=$CH_2$); 6.97 (s, 1H, Aromaten-H).

Stufe 5:

Natrium-(1S,5R,6S)-6-[(1R)-1-hydroxyethyl]-(5,7-dimethyl-1,2,3,4-tetrahydronaphtho)[2,1-a]carbapen-2-em-3-carboxylat.

Aus 300 mg (0.41 mmol) Allylester erhielt man 60 mg (21 %) Natriumsalz.-$^1$H-NMR (270 MHz, $D_2O$): δ= 1.29 (d, 3H, CH-$CH_3$); 1.60-1.82 (m, 2H, $CH_2$-$CH_2$-CH), 2.18 (s, 3H, $CH_3$); 2.22 (s, 3H, $CH_3$); 2.58-2.83 (m, 2H, $CH_2$-$CH_2$-CH); 2.90 (mc, 1H, $CH_2$-$CH_2$-CH); 3.06 (dd, 1H, H-6); 4.27 (mc, 1H, CH-$CH_3$); 4.32 (dd, 1H, H-5); 7.00 (s, 1H, Aromaten-H); 7.12 (s, 1H, Aromaten-H).

Beispiel 14

Kalium-(1S,5R,6S)-6-[(1R)-1-hydroxyethyl]-[7-[(4-methylpiperazin-1-yl)carbonyl]-1,2,3,4-tetrahydronaphtho][2,1-a]carbapen-2-em-3-carboxylat.

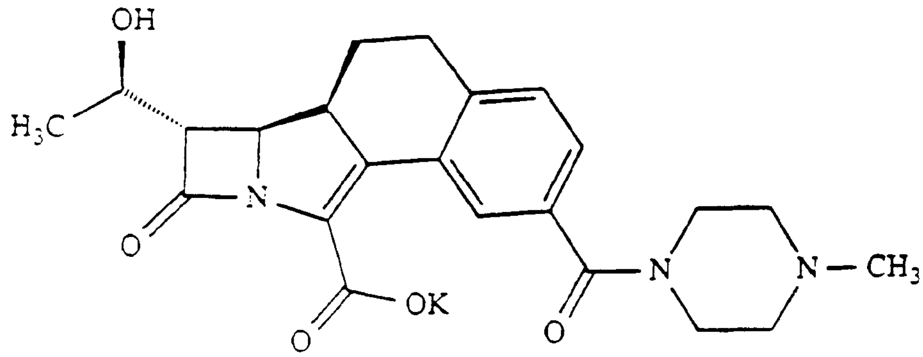

Vorstufe:

1-Tetralon-7-carbonsäurepentafluorphenylester.

Zu einer Suspension von 6.6 g (35 mmol) 1-Tetralon-7-carbonsäure in 50 ml Ethylacetat gab man bei 0°C 7.3 g (38 mmol) Pentafluorphenol und 8.5 g (41 mmol) Dicyclohexylcarbodiimid. Nach einstündigem Rühren bei Zimmertemperatur saugte man den Harnstoff ab, wusch mit Ethylacetat nach und engte die Lösung im Vakuum ein. Der Rückstand wurde mit 250 ml n-Heptan und 50 ml Ethylacetat in der Siedehitze gelöst und mit Aktivkohle versetzt. Die Aktivkohle wurde in der Siedehitze abfiltriert. Nach dem Abkühlen auf 0°C kristallisierte das Produkt aus. Durch Absaugen erhielt man 8.6 g (70 %) des gewünschten Esters. Schmp. 113°C.-$^1$H-NMR (270 MHz, $CDCl_3$): δ= 2.20 (mc, 2H, $CH_2$-$CH_2$-$CH_2$); 2.75 (t, 2H, $CH_2$-$CH_2$-$CH_2$); 3.09 (t, 2H, $CH_2$-$CH_2$-$CH_2$); 7.47 (d, 1H, Aromaten-H); 8.26 (dd, 1H, Aromaten-H); 8.88 (d, 1H, Aromaten-H).

Stufe 1:

(3S,4R)-3-[(1R)-1-tert.-Butyldimethylsilyloxyethyl]-4-[(2R)-1-oxo-7-pentafluorphenoxycarbonyl-1,2,3,4-tetrahydronaphth-2-yl]-azetidin-2-on.

Zu einer Lösung von 5.13 g (18 mmol) Azetidinon und 8.23 g (23 mmol) des Pentafluorphenylesters in 90 ml wasserfreiem Methylenchlorid gab man bei 0°C zunächst 6.0 ml (44 mmol) Triethylamin und dann 9.2 ml (51 mmol) Trimethylsilyltriflat. Man rührte weitere 4 Stunden bei Zimmertemperatur, gab die Reaktionslösung auf 220 ml gesättigte Natriumhydrogencarbonatlösung, trennte die organische Phase ab und extrahierte die wäßrige Phase einmal mit 200 ml Ethylacetat. Die vereinigten organischen Phasen wurden mit 100 ml gesättigter Kochsalzlösung gewaschen, mit 270 ml 2N Salzsäure versetzt und 30 Minuten kräftig gerührt. Die organische Phase wurde abgetrennt, über Natriumsulfat getrocknet und im Vakuum eingeengt. Nach Chromatographie an Kieselgel (Laufmittel: Toluol/Ethylacetat = 2/1) und Kristallisation aus n-Heptan erhielt man 1.21 g (12 %) des gewünschten polareren Diastereomeren. Schmp. 149°C.-$^1$H-NMR (270 MHz, $CDCl_3$): δ= 0.1 (s, 6H, $SiCH_3$); 0.89 (s, 9H, $SiC(CH_3)_3$); 1.30 (d, 3H, CH-$CH_3$); 2.05-2.20 und 2.30-2.45 (2 x m, 2 x 1H, $CH_2$-$CH_2$-CH); 2.81 (mc, 1H, $CH_2$-$CH_2$-CH); 3.11 (dd, 1H, H-3); 3.15-3.30 (m, 2H, $CH_2$-$CH_2$-CH); 4.28 (mc, 1H, CH-$CH_3$); 4.45 (mc, 1H, H-4); 5.74 (bs, 1H, NH); 7.48 (d, 1H, Aromaten-H); 8.27 (dd, 1H, Aromaten-H); 8.82 (d, 1H, Aromaten-H).

Stufe 2:

[(3S,4R)-3-[(1R)-1-tert.-Butyldimethylsilyloxyethyl]-4-[(2R)-1-oxo-7-pentafluorphenoxycarbonyl-1,2,3,4-tetrahydronaphth-2-yl]-2-oxoazetidin-1-yl]-2-oxoessigsäureallylester.

Analog zur Stufe 2 im Beispiel 1 synthetisierte man aus 2.4 g (4.1 mmol) Azetidin-2-on 2.8 g (98 %) Allylester.-$^1$H-NMR (270 MHz, $CDCl_3$): δ= 0.08 und 0.10 (2 x s, 6H, $SiCH_3$); 0.86 (s, 9H, $SiC(CH_3)_3$); 1.27 (d, 3H, CH-$CH_3$); 1.98-2.10 und 2.25-2.40 (2 x m, 2 x 1H, $CH_2$-$CH_2$-CH); 3.15-3.40 (m, 4H, $CH_2$-$CH_2$-CH und H-3);

4.36 (mc, 1H, $CH-CH_3$); 4.72 (dd, 1H, H-4); 4.81 (mc, 2H, $CH_2-CH=CH_2$); 5.30-5.50 (m, 2H, $CH_2-CH=CH_2$); 5.90-6.05 (m, 1H, $CH_2-CH=CH_2$); 7.48 (d, 1H, Aromaten-H); 8.27 (dd, 1H, Aromaten-H); 8.89 (d, 1H, Aromaten-H).

Stufe 3:

(1S,5R,6S)-6-[(1R)-1-tert.-Butyldimethylsilyloxyethyl]-(7-pentafluorphenoxycarbonyl-1,2,3,4-tetrahydronaphtho)[2,1-a]carbapen-2-em-3-carbonsäureallylester.

Die Zyklisierung von 2.4 g (3.45 mmol) Allylester wurde wie in Beispiel 1 beschrieben durchgeführt. Nach 10 Minuten bei 140°C wurde aufgearbeitet. Nach der Chromatographie erhielt man 1.04 g (46 %) Produkt.-$^1$H-NMR (270 MHz, $CDCl_3$): δ= 0.12 (s, 6H, $SiCH_3$); 0.90 (s, 6H, $SiC(CH_3)_3$); 1.28 (d, 3H,$CH-CH_3$); 1.80-2.20 (m, 2H, $CH_2-CH_2-CH$); 3.05-3.28 (m, 3H, $CH_2-CH_2-CH$); 3.31 (dd, 1H, H-6); 4.27 (mc, 1H, $CH-CH_3$); 4.38 (dd, 1H, H-5); 4.77 (mc, 2H, $CH_2-CH=CH_2$); 5.28 (mc, 2H, $CH_2-CH=CH_2$); 5.85-6.05 (m, 1H, $CH_2-CH=CH_2$); 7.30 (d, 1H, Aromaten-H); 8.01 (dd, 1H, Aromaten-H); 8.55 (d, 1H, Aromaten-H).

Stufe 4:

(1S,5R,6S)-6-[(1R)-1-tert.-Butyldimethylsilyloxyethyl]-[7-[(4-methylpiperazin-1-yl)-carbonyl]-1,2,3,4-tetrahydronaphtho][2,1-a]carbapen-2-em-3-carbonsäureallylester.

500 mg (0.75 mmol) Pentafluorphenylester wurden im 3 ml DMF gelöst und bei-78 °C mit 277 μl (2.48 mmol) N-Methylpiperazin versetzt. Nach einer weiteren Stunde bei dieser Temperatur gab man den Ansatz zu einer Mischung aus 10 ml Wasser, 0.9 ml 2N Salzsäure und 7.5 ml Methylenchlorid, trennte die organische Phase ab, trocknete sie über Natriumsulfat und engte im Vakuum ein. Nach Chromatographie des Rückstandes (Laufmittel: Methylenchlorid/Methanol = 10/1, dann 7/1) erhielt man 329 mg (75 %) des gewünschten Produktes.-$^1$H-NMR (270 MHz, $CDCl_3$): δ= 0.07 (s, 6H, $SiCH_3$); 0.89 (s, 9H, $SiC(CH_3)_3$); 1.26 (d, 3H, $CH-CH_3$); 1.6-2.15 (m, 2H, $CH_2-CH_2-CH$); 2.33 (s, 3H, $NCH_3$); 2.3-2.6 (m, 4H, Piperazin-$CH_2$); 3.0-3.23 (m, 3H, $CH_2-CH_2-CH$); 3.28 (dd, 1H, H-6); 3.4-3.9 (m, 4H, Piperazin-$CH_2$); 4.26 (mc, 1H, $CH-CH_3$); 4.33 (dd, 1H, H-5); 4.75 (mc, 2H, $CH_2-CH=CH_2$); 5.35 (mc, 2H, $CH_2-CH=CH_2$); 5.90 -6.10 (m, 1H, $CH_2-CH=CH_2$); 7.18 (d, 1H, Aromaten-H); 7.34 (dd, 1H, Aromaten-H); 7.78 (d, 1H, Aromaten-H).

Stufe 5:

(1S,5R,6S)-6-[(1R)-1-Hydroxyethyl]-[7-[(4-methylpiperazin-1-yl)-carbonyl]1,2,3,4-tetrahydronaphtho][2,1-a] carbapen-2-em-3-carbonsäureallylester.

Aus 320 mg (0.55 mmol) Silylether stellte man analog zur Stufe 4 im Beispiel 1 die Hydroxyethylverbindung dar. Nach Beendigung der Reaktion verdünnte man mit 30 ml Ethylacetat und 10 ml Wasser. Die wäßrige Phase wurde mit verdünnter Natriumhydrogencarbonatlösung neutralisiert und zweimal mit jeweils 20 ml Ethylacetat extrahiert. Nach dem Trocknen über Natriumsulfat und dem Einengen im Vakuum erhielt man 79 mg (31 %) Produkt, das sofort weiter umgesetzt wurde.

Stufe 6:

Kalium-(1S,5R,6S)-6-[(1R)-1-hydroxyethyl]-[7-[(4-methylpiperazin-1-yl)-carbonyl]-1,2,3,4-tetrahydronaphtho][2,1-a]carbapen-2-em-3-carboxylat.

Aus 79 mg (0.17 mmol) Allylester erhielt man 7.6 mg (10 %) Kaliumsalz. -$^1$H-NMR (270 MHz, $D_2O$): δ= 1.29 (d, 3H, $CH-CH_3$); 1.75-1.95 und 2.08-2.22 (2 x m, 2 x 1H, $CH_2-CH_2-CH$), 2.63 (s, 3H, $CH_3$); 2.7-3.2 und 3.5-4.0 (2 x m, 11 H, Piperazin-$CH_2$, H-6 und $CH_2-CH_2-CH$); 3.27 (mc; 1H $CH_2-CH_2-CH$); 4.27 (mc, 1H, $CH-CH_3$); 4.39 (dd, 1H, H-5); 7.30 (mc, 2H, Aromaten-H); 7.47 (d, 1H, Aromaten-H).

Beispiel 15

Kalium-(1R,5R,6S)-6-[(1R)-1-hydroxyethyl]-(6-methoxycarbonylchromano)[3,4-a]-carbapen-2-em-3-carboxylat.

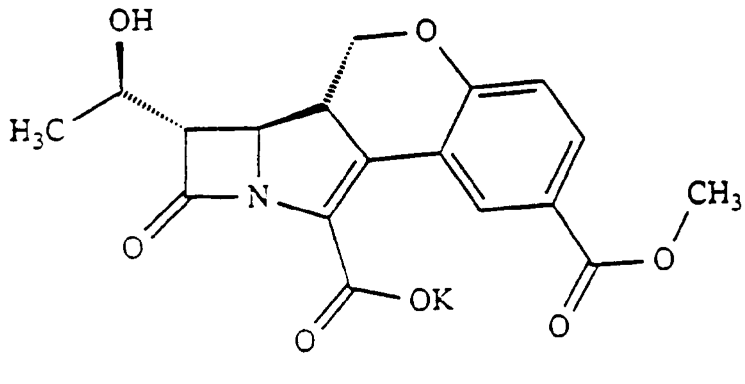

Stufe 1:

(3S,4R)-3-[(1R)-1-tert.-Butyldimethylsilyloxyethyl]-4-[(3R)-6-methoxycarbonyl-4-oxochroman-3-yl]-azetidin-2-on.

Ausgehend von 960 mg (3.35 mmol) (3S,4R)-4-Acetoxy-3-[(1R)-1-tert.-butyldimethylsilyloxyethyl]-azetidin-2-on und 1.03 g (5.0 mmol) 4-Oxochroman-6-yl-carbonsäuremethylester erhielt man wie unter Stufe 1 im Beispiel 14 beschrieben nach Chromatographie (Laufmittel: Toluol/Ethylacetat = 10/1, 1.1 l , dann 2/1) 370 mg (25 %) des weniger polaren Diastereomeren.-$^1$H-NMR (270 MHz, $CDCl_3$): δ= 0.08 und 0.10 (2 x 1s, 2 x 3H, $SiCH_3$); 0.90 (s, 9H, $SiC(CH_3)_3$); 1.28 (d, 3H, $CH$-$CH_3$); 2.85-2.98 (m, 2H, O-$CH_2$-CH und H-3); 3.72 (dd, 1H, H-4); 3.92 (s, 3H, $CO_2CH_3$); 4.18 (mc, 1H, CH-$CH_3$); 4.32 und 4.65 (2 x mc, 2 x 1H, O-$CH_2$-CH); 6.26 (bs, 1H, NH); 7.03 (d, 1H, Aromaten-H); 8.18 (dd, 1H, Aromaten-H) 8.59 (d, 1H, Aromaten-H).

Stufe 2:

[(3S,4R)-3-[(1R)-1-tert.-Butyldimethylsilyloxyethyl]-4-[(3R)-6-methoxycarbonyl-4-oxochroman-3-yl)]-2-oxoazetidin-1-yl]-2-oxoessigsäureallylester.

Analog zu Stufe 2, Beispiel 1 wurden 550 mg (1.25 mmol) des vorstehenden (3R)-Isomeren mit Oxalsäureallylesterchlorid umgesetzt. Nach dreimaligem Waschen mit Eiswasser und Trocknen mit $MgSO_4$ erhielt man die Titelverbindung als dickes Öl in quantitativer Ausbeute, das sofort zyklisiert wurde.-$^1$H-NMR (270 MHz, $CDCl_3$): δ= 0.02 und 0.08 (2 x s, 2 x 3H, SiCH3); 0.82 (s, 9H, $SiC(CH_3)_3$); 1.08 (d, 3H, CH-$CH_3$); 3.2-3.4 (m, 2H, O-$CH_2$-CH und H-3); 3.90 (s, 3H, $CO_2CH_3$); 4.2-4.5 (m, 4H, H-4, CH-$CH_3$ und O-$CH_2$-CH); 4.77 (mc, 2H, $CH_2$-CH=$CH_2$); 5.35 (mc, 2H, $CH_2$-CH=$CH_2$); 5.95 (mc, 1H, $CH_2$-CH=$CH_2$); 7.02 (mc, 1H, Aromaten-H); 7.98 (mc, 1H, Aromaten-H); 8.60 (mc, 1H, Aromaten-H).

Stufe 3:

(1R,5R,6S)-6-[(1R)-1-tert.-Butyldimethylsilyloxyethyl]-(6-methoxycarbonylchromano)[3,4-a]carbapen-2-em-3-carbonsäureallylester.

1.25 mmol Rohprodukt aus Stufe 2 wurden mit 600 mg (4.4 mmol) $MeP(OEt)_2$ in 25 ml Mesitylen 1 Stunde unter Rückfluß erhitzt. Die erkaltete Lösung wurde direkt über Kieselgel, desaktiviert mit 10% Wasser, mit Toluol/Ethylacetat (10/1) chromatographiert. Die Titelverbindung wird als Öl (160 mg, 25 %) erhalten.-$^1$H-NMR (270 MHz, $CDCl_3$): δ = 0.11 (s, 6H, $SiCH_3$); 0.91 (s, 9H, $SiC(CH_3)_3$); 1.25 (d, 3H, CH-$CH_3$); 3.25 (dd, 1H, H-6); 3.62-3.70 (m, 1H, O-$CH_2$-CH), 3.77 (dd, 1H, H-5); 3.92 (s, 3H, $CO_2CH_3$); 4.10-4.20 (m, 1H, O-$CH_2$-CH); 4.24 (mc, 1H, CH-$CH_3$); 4.61-4.68 (m, 1H, O-$CH_2$-CH); 4.72-4.80 (m, 2H, $CH_2$-CH=$CH_2$); 5.25-5.50 (m, 2H, $CH_2$-CH=$CH_2$); 5.90-6.05 (m, 1H, $CH_2$-CH=$CH_2$); 6.90 (d, 1H, Aromaten-H); 7.94 (dd, 1H, Aromaten-H); 9.29 (d, 1H, Aromaten-H).

Stufe 4:

(1R,5R,6S)-6-[(1R)-1-Hydroxyethyl]-(6-methoxycarbonylchromano)[3,4-a]carbapen-2-em-3-carbonsäureallylester.

Analog Stufe 4, Beispiel 1 wurden 150 mg (0.29 mmol) des Silylethers umgesetzt (Reaktionszeit: 20 h). Nach Chromatographie (Laufmittel: Toluol/Ethylacetat = 1/1) wurden 80 mg (69 %) kristallines Produkt erhalten.-$^1$H-NMR (270 MHz, $CDCl_3$): δ= 1.38 (d, 3H, $CH-CH_3$); 3.32 (dd, 1H, H-6); 3.62-3.73 (m, 1H, $O-CH_2-CH$); 3.82 (dd, 1H, H-5); 3.92 (s, 3H, $CO_2CH_3$); 4.15 (mc, 1H, $O-CH_2-CH$); 4.28 (mc, 1H, $CH-CH_3$); 4.66-4.95 (m, 3H, $O-CH_2-CH$ und $CH_2-CH=CH_2$); 5.25-5.50 (m, 2H, $CH_2-CH=CH_2$); 5.95-6.12 (m, 1H, $CH_2-CH=CH_2$); 6.92 (d, 1H, Aromaten-H); 7.96 (dd, 1H, Aromaten-H); 9.24 (d, 1H, Aromaten-H).

Stufe 5

Kalium-(1R,5R,6S)-6-[(1R)-1-hydroxyethyl]-(6-methoxycarbonylchromano)[3,4-a]carbapen-2-em-3-carboxylat.

Analog Stufe 5, Beispiel 1 wurden 80 mg (0.20 mmol) Allylester umgesetzt. Die Reaktionslösung wurde nach 1 h mit 3 ml Diethylether und 2 ml Wasser verrührt und die Wasserphase über (Polystyrol-Adsorberharz ®Amberlite XAD-2 (Korngröße 20-60 mesh) (1.5 x 15 cm-Säule, je 10 ml Fraktionen) mit Wasser chromatographiert. Die produkthaltigen Fraktionen wurden gefriergetrocknet und man erhielt 25 mg (32 %) amorphen Feststoff.-$^1$H-NMR (270 MHz, $D_2O$): δ= 1.29 (d, 3H, $CH-CH_3$); 3.58 (dd, 1H, H-6); 3.69 (mc, 1H, $O-CH_2-CH$); 3.75 (dd, 1H, H-5); 3.90 (s, 3H, $CO_2CH_3$); 4.12 (dd, 1H, $O-CH_2-CH$); 4.27 (mc, 1H, $CH-CH_3$); 4.65 (dd, 1H, $O-CH_2-CH$); 6.95 (d, 1H, Aromaten-H); 7.82 (dd, 1H, Aromaten-H); 8.98 (d, 1H, Aromaten-H).

Beispiel 16

Kalium-(1S,5R,6S)-6-[(1R)-1-hydroxyethyl]-(7-methoxy-1,2,3,4-tetrahydronaphtho)[2,1-a]carbapen-2-em-3-carboxylat

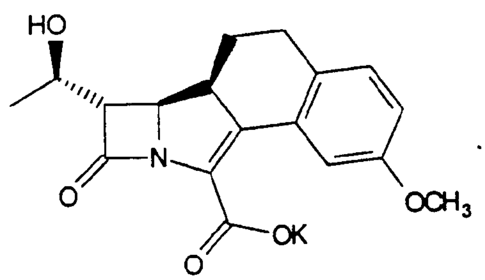

Stufe 1:

(3S,4R)-3-[(1R)-1-tert.-Butyldimethylsilyloxyethyl]-4-(2R)-7-methoxy-1-oxo-1,2,3,4-tetrahydronaphth -2-yl]-azetidin-2-on

Zu 8.1 ml mit einer 1.5-molaren Lösung von Lithiumdiisopropyl (12.2 mmol, in THF/Heptan, 6/4) und 60 ml wasserfreiem THF gab man bei-78°C 2 g (11.4 mmol) 7-Methoxytetralon in 10 ml THF. Nach 10 min bei-78°C rührt man 30 min bei 0°C. Bei -78°C wurden dann 13.3 ml einer 1-molaren Lösung von Chlortriisopropoxytitanat (13.3 mmol) in Hexan zugetropft, und es wurde 70 min bei dieser Temperatur gerührt. Nach Zugabe von 2.87 g (10 mmol) (3S,4R)-4-Acetoxy-3-[(1R)-1-tert.-butyldimethylsilyloxyethyl]-azetidin-2-on, gelöst in 5 ml THF, ließ man die Reaktion auf 0°C aufwärmen und rührte 30 min bei dieser Temperatur. Die Reaktionsmischung wurde auf 170 ml gesättigte Ammoniumchlorid-Lösung gegossen und mit Ethylacetat extrahiert. Die organische Phase wurde mit Wasser gewaschen, über Natriumsulfat getrocknet und im Vakuum eingeengt. Das Rohrprodukt enthält das (1S)- und (1R)-Diastereomere im Verhältnis 3/1. Der Rückstand wurde an Kieselgel chromatographiert (Laufmittel: Toluol/Ethylacetat = 4/1) und das Produkt aus 120 ml n-Heptan kristallisiert. Ausbeute: 1.28 g (32%), weiße Kristalle.- $^1$H-NMR (270 MHz, $CDCl_3$); δ = 0.09 (s, 6H, $Si(CH_3)_2$; 0.88 (s, 9H, $SiC(CH_3)_3$); 1.27 (d, 3H, $CH-CH_3$); 1.95-2.12 und 2.21-2.33 (2 x m, 2x1H, $CH-CH_2-CH_2$); 2.71 (m, 1H, $CH-CH_2-CH_2$); 2.98-3.08 (m, 2H, $CH-CH_2-CH_2$); 3.10 (dd, 1H, H-3); 3.84 (s, 3H, $OCH_3$); 4.26 (m, 1H, CH-

$CH_3$); 4.44 (m, 1H, H-4); 5.75 (br., 1H, NH); 7.09 (dd, 1H, Aromaten-H); 7.17 (d, 1H, Aromaten-H); 7.49 (d, 1H, Aromaten-H).

Stufe 2:

[(3S,4R)-3-[(1R)-1-tert.-Butyldimethylsilyloxyethyl]-4-[(2R)-7-methoxy-1-oxo-1,2,3,4-tetrahydronaphth-2-yl)]-2-oxoazetidin-1-yl]-2-oxoessigsäureallylester

Analog zu Stufe 2 in Beispiel 1 wurden 1.1 g (2.27 mmol) des Azetidinons acyliert. Das Rohprodukt wurde durch Chromatographie an Kieselgel (Laufmittel: Heptan/Ethylacetat = 4/1) gereinigt. Ausbeute: 1.15 g (82 %).- $^1$H-NMR (270 MHz, $CDCl_3$): $\delta$ = 0.07 und 0.09 (2 x s, 2x3H, $SiC(CH_3)_2$); 0.87 (s, 9H, $SiC(CH_3)_3$; 1.20 (d, 3H, $CH-CH_3$); 1.90-2.30 (m, 2H, $CH-CH_2-CH_2$); 3.00-3.09 (m, 2H, $CH-CH_2-CH_2$); 3.21 (m, 1H, $CH-CH_2-CH_2$); 3.29 (m, 1H, H-3); 3.83 (s, 3H, $OCH_3$); 4.33 (m, 1H, $CH-CH_3$); 4.67 (m, 1H, H-4); 4.81 (d, 2H, $CH_2-CH=CH_2$); 5.30 und 5.41 (2 x d, 2 x 1H, $CH_2-CH=CH_2$); 5.98 (m, 1H, $CH_2-CH=CH_2$); 7.08 (dd, 1H, Aromaten-H); 7.17 (d, 1H, Aromaten-H); 7.51 (d, 1H, Aromaten-H).

Stufe 3:

(1S,5R,6S)-6-[(1R)-1-tert.-Butyldimethylsilyloxyethyl)-(7-methoxy-1,2,3,4-tetrahydronaphtho)[2,1-a]carbapen-2-em-3-carbonsäureallylester

Analog zu Stufe 3 in Beispiel 1 wurden 1.13 g (2.2 mmol) Produkt aus Stufe 2 in 25 ml Xylol bei 140°C zyklisiert. Ausbeute: 370 mg (35 %).- $^1$H-NMR (270 MHz, $CDCl_3$): $\delta$ = 0.10 (s, 6H, $Si(CH_3)_2$); 0.90 (s, 9H, $SiC(CH_3)_3$); 1.27 (d, 3H, $CH-CH_3$); 1.80-2.13 (m, 2H, $CH-CH_2-CH_2$); 2.95-3.04 (m, 2H, $CH-CH_2-CH_2$); 3.17 (m, 1H, $CH-CH_2$); 3.28 (dd, 1H, H-6); 3.78 (s, 3H, $OCH_3$); 4.20-4.34 (m, 2H, H-5 und $CH-CH_3$); 4.77 (m, 2H, $CH_2-CH=CH_2$); 5.25 und 5.41 (2 x d, 2 x 1H, $CH_2-CH=CH_2$); 5.99 (m, 1H, $CH_2-CH=CH_2$); 6.81 (dd, 1H, Aromaten-H); 7.03 (d, 1H, Aromaten-H); 7.40 (d, 1H, Aromaten-H).

Stufe 4:

(1S,5R,6S)-6-[(1R)-1-Hydroxyethyl]-(7-methoxy-1,2,3,4-tetrahydronaphtho)[2,1-a]carbapen-2-em-3-carbonsäureallylester

Wie für die Stufe 4 in Beispiel 1 beschrieben wurden 369 mg (0.76 mmol) umgesetzt. Das Rohprodukt wurde an Kieselgel chromatographiert (Laufmittel: Heptan/ Ethylacetat = 1/1). Ausbeute: 119 mg (43 %).- $^1$H-NMR (270 MHz, $CDCl_3$): $\delta$ = 1.37 (d, 3H, $CH-CH_3$); 1.81-2.18 (m, 2H, $CH-CH_2-CH_2$); 2.92-3.08 (m, 2H, $CH-CH_2-CH_2$); 3.21 (m, 1H, $CH-CH_2-CH_2$); 3.33 (dd, 1H, H-6); 3.78 (s, 3H, $OCH_3$); 4.20-4.37 (m, 2H, H-5 und $CH-CH_3$); 4.80 (m, 2H, $CH_2-CH=CH_2$); 5.26 und 5.41 (2 x d, 2 x 1H, $CH_2-CH=CH_2$); 6.00 (m, 1H, $CH_2-CH=CH_2$); 6.82 (dd, 1H, Aromaten-H); 7.03 (d, 1H, Aromaten-H); 7.41 (d, 1H, Aromaten-H).

Stufe 5:

Kalium-(1S,5R,6S)-6-[(1R)-1-hydroxyethyl]-(7-methoxy-1,2,3,4-tetrahydronaphtho)[2,1-a]carbapen-2-em-carboxylat

Analog zu Stufe 5 in Beispiel 1 wurden 119 mg (0.33 mmol) umgesetzt. Das Rohprodukt über (R)LiChroprep RP 18 chromatographiert. Dabei wurden anorganische Salze mit Wasser entfernt. Das Produkt wurde anschließend mit 20 % Acetonitril in Wasser eluiert. Ausbeute: 45 mg (37 %).- $^1$H-NMR (200 MHz, D20); $\delta$ = 1.28 (d, 3H, $CH-CH_3$); 1.80 und 2.05 (2 x mc, 2 x 1H, $CH_2-CH_2-CH$); 2.85-3.05 (m, 2H, $CH_2-CH_2-CH$); 3.21 (mc, 1H, $CH_2-CH_2-CH$); 3.55 (dd, 1H, H-6); 3.79 (s, 3H, $OCH_3$);4.2-4.4 (m, 2H, H-5 und $CH-CH_3$); 6.7-7.2 (m, 3H, Aromaten-H).

Beispiel 17

Natrium-(1S,5R,6S)-6-[(1R)-1-hydroxyethyl]-(7-methoxycarbonyl-1,2,3,4-tetrahydronaphtho[2,1-a]carbapen-2-em-3-carboxylat

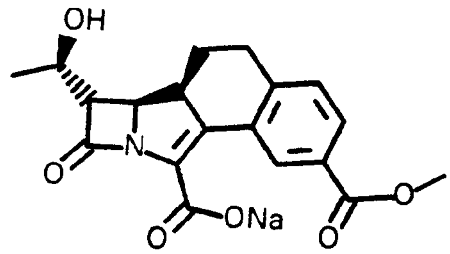

Stufe 1:

(3S,4R)-3-[(1R)-1-tert.-Butyldimethylsilyloxyethyl]-4-[(2R)-7-methoxycarbonyl-1-oxo-1,2,3,4-tetrahydronaphth-2-yl]-azetidin-2-on.

Wie für die Stufe 1 in Beispiel 16 beschrieben wurde, setzte man 500 mg (2.45 mmol)1-Tetralon-7-carbonsäuremethylester um. Nach Chromatographie an Kieselgel (Laufmittel: Toluol/Essigester = 5/1 erhielt man 220 mg (23 %) Produkt.- $^1$H-NMR (200 MHz,$CDCl_3$): δ = 0.09 (s, 6H, $SiCH_3$); 0.85 (s, 9H, $SiC(CH_3)_3$); 1.26 (d, 3H, $CH\text{-}CH_3$); 1.94-2.20 (m, 1H, $CH\text{-}CH_2\text{-}CH_2$); 2.22-2.40 (m, 1H, $CH\text{-}CH_2\text{-}CH_2$); 2.68-2.82 (m, 1H, $CH\text{-}CH_2\text{-}CH_2$); 3.05-3.22 (m, 3H, $CH\text{-}CH_2\text{-}CH_2$ und H-4); 3.95 (s, 3H, $COOCH_3$); 4.26 (mc, 1H, $CH\text{-}CH_3$); 4.24 (dd, 1H, H-3); 5.72 (bs, 1H, NH); 7.38 (d, 1H, Aromaten-H); 8.18 (dd, 1H, Aromaten-H); 8.65 (d, 1H, Aromaten-H).

Stufe 2:

[(3S,4R)-3-[(1R)-1-tert.-Butyldimethylsilyloxyethyl]-4-[(2R)-7-methoxycarbonyl-1-oxo-1,2,3,4-tetrahydronaphth-2-yl-]-2-oxoazetidin-1-yl]-2-oxoessigsäureallylester

Analog zur Stufe 2 im Beispiel 1 synthetisierte man aus 210 mg (0.49 mmol) Azetidin-2-on 100 mg (38 %) Allylester.- $^1$H-NMR (270 MHz, $CDCl_3$): δ = 0.08 (2 x s, 6H, $Si(CH_3)_2$; 0.84 (s, 9H, $SiC(CH_3)_3$); 1.20 (d, 3H, $CH\text{-}CH_3$); 1.90-2.15 (m, 1H, $CH\text{-}CH_2\text{-}CH_2$); 2.20-2.36 (m, 1H, $CH\text{-}CH_2\text{-}CH_2$); 3.05-3.28 (m, 3H, $CH\text{-}CH_2\text{-}CH_2$); 3.32 (mc, 1H, H-4); 3.92 (s, 3H, $COOCH_3$); 4.34 (mc, 1H, $CH\text{-}CH_3$); 4.68 (mc, 1H, H-3); 4.81 (mc, 2H, $CH_2\text{-}CH{=}CH_2$); 5.25-5.50 (m, 2H, $CH_2\text{-}CH{=}CH_2$); 5.88-6.10 (m, 1H, $CH_2\text{-}CH{=}CH_2$); 7.36 (d, 1H, Aromaten-H); 8.16 (dd, 1H, Aromaten-H); 8.70 (d, 1H, Aromaten-H).

Stufe 3:

(1S,5R,6S)-6-[(1R)-1-tert.-Butyldimethylsilyloxyethyl]-(-7-methoxycarbonyl-1,2,3,4-tetrahydronaphtho)[2,1-a-]-carbapen-2-em-carbonsäureallylester.

Die Zyklisierung von 880 mg (1.62 mmol) Allylester wurde wie im Beispiel 1 beschrieben durchgeführt. Nach 15 Minuten bei 160°C wurde aufgearbeitet. Nach der Chromatographie erhielt man 550 mg (66 %) Produkt.- $^1$H-NMR (200 MHz, $CDCl_3$): δ = 0.10 (s, 6H, $Si(CH_3)_2$); 0.89 (s, 9H, $SiC(CH_3)_3$); 1.24 (d, 3H, $CH\text{-}CH_3$); 1.80-2.20 (m, 2H, $CH\text{-}CH_2\text{-}CH_2$); 3.30-3.40 (m, 4H, $CH\text{-}CH_2\text{-}CH_2$ und H-5); 3,88 (s, 3H, $COOCH_3$); 4.25 (mc, 1H, $CH\text{-}CH_3$); 4.33 (dd, 1H, H-6); 4.78 (mc, 2H, $CH_2\text{-}CH{=}CH_2$); 5.18-5.50 (m, 2H, $CH_2\text{-}CH{=}CH_2$); 5.80-6.20 (m, 1H, $CH_2\text{-}CH{=}CH_2$); 7.20 (d, 1H, Aromaten-H); 7.86 (dd, 1H, Aromaten-H); 8.40 (d, 1H, Aromaten-H).

Stufe 4:

(1S,5R,6S)-6-[(1R)-1-Hydroxyethyl]-7-methoxycarbonyl-(1,2,3,4-tetrahydronaphtho-[2,1-a]carbapen-2-em-3-carbonsäureallylester.

Aus 550 m (1.08 mmol) Silylether stellte man analog zur Stufe 4 im Beispiel 1 die Hydroxyethylverbindung dar. Nach Säulenchromatographie an Kieselgel erhielt man 200 mg (47 %).- $^1$H-NMR (200 MHz, $CDCl_3$): δ = 1.38 (d, 3H, $CH\text{-}CH_3$); 1.80-2.20 (m, 2H, $CH\text{-}CH_2\text{-}CH_2$); 3.00-3.30 (m, 3H, $CH\text{-}CH_2\text{-}CH_2$); 3.36 (mc, 1H, H-5);

3.90 (s, 3H, $COOH_3$); 4.28 (mc, 1H, $CH-CH_3$); 4.38 (dd, 1H, H-6); 4.80 (mc, 2H, $CH_2-CH=CH_2$); 5.20-5.50 (m, 2H, $CH_2-CH=CH_2$); 5.80-6.10 (m, 1H, $CH_2-CH=CH_2$); 7.20 (d, 1H, Aromaten-H); 7.88 (dd, 1H, Aromaten-H); 8.41 (d, 1H, Aromaten-H).

Stufe 5:

Natrium-(1S,5R,6S)-6-[(1R)-1-hydroxyethyl]-(7-methoxycarbonyl-1,2,3,4-tetrahydronaphtho[2,1-a]carbapen-2-em-3-carboxylat.

Aus 190 mg (0.48 mmol) erhielt man 45 mg (25 %) Natriumsalz. $^1$H-NMR (200 MHz,$D_2O$); δ = 1.36 (d, 3H, $CH-CH_3$); 1.60-2.00 (m, 1H, $CH-CH_2-CH_2$); 2.10-2.20 (m, 1H, $CH-CH_2-CH_2$); 2.95-3.16 (m, 2H, $CH-CH_2-CH_2$); 3.25 (mc, 1H, $CH-CH_2-CH_2$); 3.59 (dd, 1H, H-6); 3.92 (s, 3H, $COOCH_3$); 4.30 (mc, 1H, $CH-CH_3$), 4.41 (dd, 1H, H-5); 7.25 (d, 1H, Aromaten-H); 7.73 (dd, 1H, Aromaten-H); 7.98 (d, 1H, Aromaten-H).

Beispiel 18

Natrium(1S,5R,6S)-6-[(1R)-1-hydroxyethyl]-(5,6-dimethoxy-1,2,3,4-tetrahydronaphtho)[2,1-a]carbapen-2-em-3-carboxylat.

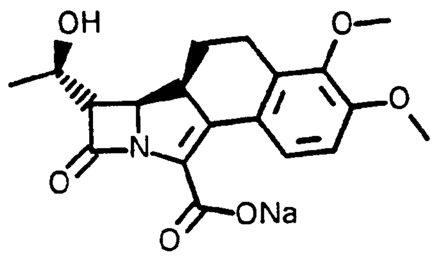

Stufe 1:

(3S,4R)-3-[(1R)-1-tert.-Butyldimethylsilyloxyethyl]-4-[(2R)-5,6-dimethoxy-1-oxo-1,2,3,4-tetrahydronaphth-2-yl)]-azetidin-2-on.

Wie für die Stufe 1 im Beispiel 16 beschrieben wurde, setzte man 500 mg (2,42 mmol) 5,6-Dimethoxy-1-tetralon um. Nach Chromatographie an Kieselgel (Laufmittel Toluol/ Essigester = 5/1) erhielt man 270 mg (29 %) Produkt.- $^1$H-NMR (200 MHz, $CDCl_3$): δ = 0.08 (s, 6H, $Si(CH_3)_2$); 0.85 (s, 9H, $Si(CH_3)_3$); 1.25 (d, 3H, $CH-CH_3$); 1.80-2.12 (m, 1H, $CH-CH_2-CH_2$); 2.20-2.12 (m, 1H, $CH-CH_2-CH_2$); 2.60-2.95 (m, 2H, $CH-CH_2-CH_2$); 3.08 (dd, 1H, H-3); 3.28 (mc, 1H, $CH-CH_2-CH_2$); 4.84 (s, 3H, $O-CH_3$); 4.94 (s, 3H, $O-CH_3$); 4.27 (mc, 1H, $CH-CH_3$); 4.42 (dd, 1H, H-4); 5,69 (bs, 1H, NH); 6.91 (d, 1H, Aromaten-H); 7.82 (d, 1H, Aromaten-H).

Stufe 2:

[(3S,4R)-3-[(1R)-1-tert.-Butyldimethylsilyloxyethyl]-4-[(2R)-5,6-dimethoxy-1-oxo-1,2,3,4-tetrahydronaphth-2-yl)]-2-oxoazetidin-1-yl]-2-oxoessigsäureallylester.

Analog zur Stufe 2 im Beispiel 1 synthetisierte man aus 1,46 g (3,37 mmol) Azetidin-2-on 910 mg (49 %) Allylester.- $^1$H-NMR (200 MHz, $CDCl_3$): δ = 0.05 (s, 3H, $Si(CH_3)$; 0.06 (s, 3H, $Si(CH_3)$; 0.84 (s, 9H, $SiC(CH_3)_3$); 1.18 (d, 3H, $CH-CH_3$); 1.80-2.10 (m, 1H, $CH-CH_2-CH_2$); 2.17-2.38 (m, 1H, $CH-CH_2-CH_2$); 2.70-3.00 (m, 1H, $CH-CH_2-CH_2$); 3.10-3.40 (m, 3H, $CH-CH_2-CH_2$ und H-3); 3.84 (s, 3H, $O-CH_3$); 3.93 (s, 3H, $O-CH_3$); 4.32 (mc, 1H, $CH-CH_3$); 4.64 (mc, 1H, H-4); 4.81 (mc, 2H, $CH_2-CH=CH_2$); 5.20-5.50 (m, 2H, $CH_2-CH=CH_2$); 5.80-6.10 (m, 1H, $CH_2-CH=CH_2$); 6.89 (d, 1H, Aromaten-H); 7.84 (d, 1H, Aromaten-H).

Stufe 3:

(1S,5R,6S)-6-[(1R)-1-tert.-Butyldimethylsilyloxyethyl]-(5,6-dimethoxy-1,2,3,4-tetrahydronaphtho)[2,1-a]carbapen-2-em-3-carbonsäureallylester.

Die Zyklisierung von 850 mg (1.56 mmol) Allylester wurde wie in Beispiel 1 beschrieben durchgeführt. Nach 30 Minuten bei 160°C wurde aufgearbeitet. Nach der Chromatographie erhielt man 190 mg (24 %) Produkt.- $^1$H-NMR (200 MHz, $CDCl_3$): δ = 0.09 (s, 6H, $Si(CH_3)_2$); 0.88 (s, 9H, $SiC(CH_3)_3$); 1.28 (d, 3H, $CH$-$CH_3$); 1.65-2.00 (m, 1H, $CH$-$CH_2$-$CH_2$); 2.02-2.20 (m, 1H, $CH_2$-$CH_2$); 2.70-3.18 (m, 3H, $CH$-$CH_2$-$CH_2$); 3.26 (mc, 1H, H-6); 3.80 (s, 3H, O-$CH_3$); 3.88 (s, 3H, O-$CH_3$); 4.18-4.35 (m, 2H, $CH$-$CH_3$ und H-5); 4.78 (mc, 2H, $CH_2$-$CH$=$CH_2$); 5.20-5.50 (m, 2H, $CH_2$-$CH$=$CH_2$); 5.86-6.18 (m, 1H, $CH_2$-$CH$=$CH_2$); 6.78 (d, 1H, Aromaten-H); 7.64 (d, 1H, Aromaten-H).

Stufe 4:

(1S,5R,6S)-6-[(1R)-1-Hydroxyethyl]-(5,6-dimethoxy-1,2,3,4-tetrahydronaphtho)[2,1-a]carbapen-2-em-3-carbonsäureallylester.

Aus 180 mg (0.35 mmol) Silylether stellte man analog zur Stufe 4 im Beispiel 1 die Hydroxyethylverbindung dar. Nach Säulenchromatographie an Kieselgel erhielt man 55 mg (39 %).- $^1$H-NMR (200 MHz,$CDCl_3$): δ = 1.39 (d, 3H, $CH$-$CH_3$); 1.80-2.00 (m, 1H, $CH$-$CH_2$-$CH_2$); 2.08-2.30 (m, 1H, $CH$-$CH_2$-$CH_2$); 2.75-3.00 (m, 1H, $CH$-$CH_2$-$CH_2$); 3.05-3.28 (m, 2H, $CH$-$CH_2$-$CH_2$); 3.34 (dd, 1H, H-6); 3.80 (s, 3H, $OCH_3$); 3.85 (s, 3H, $OCH_3$); 4.20-4.40 (m, 2H, $CH$-$CH_3$ und H-5); 4.60-4.92 (m, 2H, $CH_2$-$CH$=$CH_2$); 5.20-5.50 (m, 2H, $CH_2$-$CH$=$CH_2$); 5.90-6.15 (m, 1H, $CH_2$-$CH$=$CH_2$); 6.80 (d, 1H, Aromaten-H); 7.65 (d, 1H, Aromaten-H).

Stufe 5:

Natrium-(1S,5R,6S)-6-[(1R)-1-hydroxyethyl]-(5,6-dimethoxy-1,2,3,4-tetrahydronaphtho)[2,1-a]carbapen-2-em-3-carboxylat.

Aus 50 mg (0.125 mmol) erhielt man 40 mg (84 %) Natriumsalz.
$^1$H-NMR (200 MHz,$D_2O$): δ = 1.34 (d, 3H, $CH$-$CH_3$); 1.40-1.70 (m, 1H, $CH$-$CH_2$-$CH_2$); 2.10-2.30 (m, 1H, $CH$-$CH_2$-$CH_2$); 2.82 (mc, 1H, $CH$-$CH_2$-$CH_2$); 3.00-3.24 (m, 2H, $CH$-$CH_2$-$CH_2$); 3.56 (dd, 1H, H-6); 3.80 (s, 3H, $OCH_3$); 3.90 (s, 3H, $OCH_3$); 4.28 (mc, 1H, $CH$-$CH_3$); 4.36 (dd, 1H, H-5); 6.94 (d, 1H, Aromaten-H); 7.32 (d, 1H, Aromaten-H).

Beispiel 19

Pivaloyloxymethyl-(1S,5R,6S)-6-[(1R)-1-hydroxyethyl]-(1,2,3,4-tetrahydronaphtho)[2,1-a]carbapen-2-em-3-carboxylat

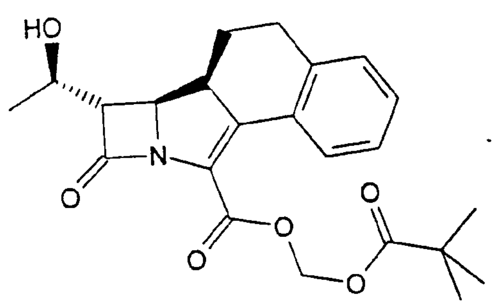

18 mg (0.12 mmol) Chlormethylpivaloat wurden in 2 ml trockenem DMF gelöst und mit 12 mg (0.12 mmol) Natriumbromid versetzt. Nach 48 Stunden bei Raumtemperatur filtrierte man über Glaswatte und versetzte das Filtrat mit 20 mg (0.06 mmol) Kalium-(1S,5R,6S)-6-[(1R)-1-hydroxyethyl]-(1,2,3,4-tetrahydronaphtho)[2,1-a-]carbapen-2-em-3-carboxylat (Beispiel 1) und rührte weitere 4 Stunden bei Raumtemperatur. Der Ansatz wurde in 10 ml Wasser und 70 ml Diethylether aufgenommen. Die organische Phase wurde mit je 10 ml 9 %iger Natriumbicarbonat-Lösung und 25 %iger Ammoniumchloridlösung und mit 3 x 10 ml Wasser gewaschen. Es wurde

über Magnesiumsulfat getrocknet, eingeengt und an der Ölpumpe bis zur Gewichtskonstanz getrocknet. Man erhält 20 mg (81 %) der Titelverbindung.- $^1$H-NMR (200 MHz,$CDCl_3$): δ = 1.24 (s, 9H, $C(CH_3)_3$); 1.36 (d, 3H, $CH$-$CH_3$); 1.80-2.20 und 2.80-3.30 (2 x m, 5H, $CH_2$-$CH_2$-CH); 3.34 (dd, 1H, H-6); 4.32 (mc, 1H, CH-$CH_3$); 4.36 (dd, 1H, H-5); 5.91 (mc, 2H, O-$CH_2$-O); 7.00-7.30 (m, 3H, Aromaten-H); 7.72 (d, 1H, Aromaten-H).

Beispiel 20

[1-(Pivaloyloxy)ethyl]-(1S,5R,6S)-6-[(1R)-1-hydroxyethyl]-(1,2,3,4-tetrahydronaphtho)[2,1-a]carbapen-2-em-3-carboxylat

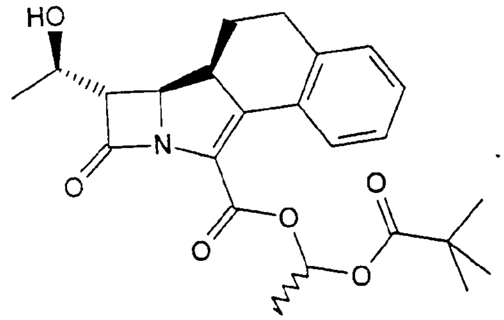

Wie in Beispiel 19 beschrieben wurden 100 mg (0.3 mmol) Kaliumsalz (Beispiel 1) mit 125 mg (0.6 mmol) 1-Bromethylpivaloat umgesetzt. Nach Chromatographie an Kieselgel (Laufmittel: Ethylacetat/n-Heptan = 3/1) konnte das Produkt als Öl erhalten werden. Ausbeute: 50 mg (39 %).- $^1$H-NMR (200 MHz, $CDCl_3$): δ = 1.21 und 1.23 (2 x s, 9H, $C(CH_3)_3$); 1.36 (d, 3H, CH-$CH_3$); 1.53 und 1.57 (2 x d, 3H, $OCH(CH_3)O$; 1.75-2.25 (m, 2H, CH-$CH_2$-$CH_2$); 3.02-3.15 (m, 2H, CH-$CH_2$); 3.25 (mc, 1H, CH-$CH_2$-$CH_2$); 3.34 (dd, 1H, H-6); 4.20-4.40 (m, 2H, H-6 und $CHCH_3$); 6.80-7.35 und 7.73-7.82 (2 x m, 5H, $OCH(CH_3)O$ und Aromaten-H).

Beispiel 21

(1S,5R,6S)-6-[(1R)-1-Hydroxyethyl]-[7-[(4,4-dimethylpiperazinium-1-yl)-carbonyl]-1,2,3,4-tetrahydronaphthol][2.1-a]carbapen-2-em-3-carboxylat

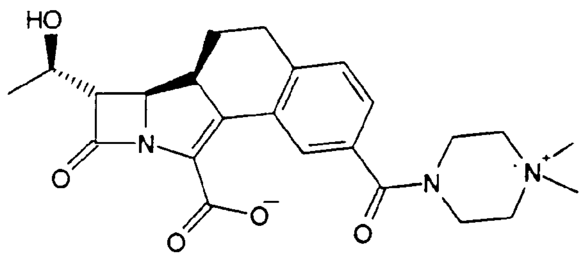

Ausgehend von 475 mg (0.82 mmol) Stufe 4 in Beispiel 14 stellte man dort wie in Stufe 5 beschrieben die Hydroxyethylverbindung her. Das Rohprodukt wurde mit 215 µl (3.2mmol) lodmethan in 10 ml Dichlormethan alkyliert. Nach 24 h bei Zimmertemperatur engte man in Vakuum ein und setzte das Rohprodukt wie für Stufe 5 in Beispiel 1 beschrieben weiter um. Nach der Chromatographie über (R)LiChroprep RP 18 und Gefriertrocknen erhielt man 37.9 mg (11%) des gewünschten Produktes.-$^1$H-NMR (270 MHz,$D_2O$): δ=1.30 (d, 3H, CH-$CH_3$); 1.75-1.95 und 2.1-2.25 (2 x m, 2 x 1H, CH-$CH_2$-$CH_2$); 3.03-3.15 (m, 2H, CH-$CH_2$-$CH_2$); 3.28 (s, 6H, $N(CH_3)_2$); 3.3-4.2 (m, 10H, Piperazin-$CH_2$, H-6, CH-$CH_2CH_2$); 4.27 (mc, 1H, CH-$CH_3$); 4.39 (dd, 1H, H-5); 7.33 (mc, 2H, Aromaten-H; 7.49 (d, 1H, Aromaten-H).

Beispiel 22

Kalium-(1S,5R,6S)-6-[(1R)-1-hydroxyethyl]-[7-[(morpholin-4-yl)-carbonyl]-1,2,3,4-tetrahydronaphtho][2.1-a] carbapen-2-em-3-carboxylat

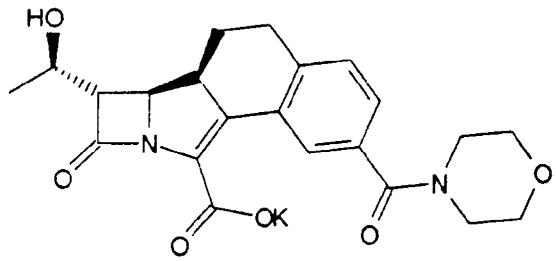

Stufe 1

(1S,5R,6S)-6-[(1R)-1-tert.-Butyldimethylsilyloxyethyl]-[7-[(morpholin-4-yl)-carbonyl]-1,2,3,4tetrahydronaphtho][2.1-a]carbapen-2-em-3-carbonsäureallylester.

Analog zu Stufe 4 im Beispiel 14 setzt man 500 mg (0.75 mmol) Pentafluorphenylester mit 215 µl (2.48 mmol) Morpholin um. Nach Chromatographie (Laufmittel: Toluol/Ethylacetat = 1/1) erhielt man 251 mg (59%) des gewünschten Produktes.- $^1$H-NMR (200 MHz, $CDCl_3$) δ=0.09 (s, 6H, $SiCH_3$); 0.89 (s, 9H, $SiC(CH_3)_3$); 1.27 (d, 3H, $CH\text{-}CH_3$); 1.8-2.15 (m, 2H, $CH_2\text{-}CH_2\text{-}CH$); 3.0-3.3 (m, 4H, $CH_2\text{-}CH_2\text{-}CH$ und H-6); 3.4-3.9 (m, 8H, Morpholin-$CH_2$); 4.18-4.38 (m, 2H, $CH\text{-}CH_3$ und H-5); 4.76 (mc, 2H, $CH_2\text{-}CH{=}CH_2$); 5.34 (mc, 2H, $CH_2\text{-}CH{=}CH_2$); 5.85-6.10 (m, 1H, $CH_2\text{-}CH{=}CH_2$); 7.10-7.40 (m, 2H, Aromaten-H); 7.78 (d, 1H, Aromaten-H).

Stufe 2

(1S,5R,6S)-6-[(1R)-1-Hydroxyethyl]-[7-[(morpholin-4-yl)-carbonyl)-1,2,3,4-tertahydronaphtho][2.1-a]carbapen-2-em-3-carbonsäureallylester

237 mg (0.42 mmol) Silylether wurden analog zu Stufe 5 im Beispiel 14 umgesetzt. Nach Chromatographie an (R)LiChroprep RP 18 (Laufmittel: Wasser/Acetonitril-Gradient) erhielt man 77 mg (41%) des Produktes.- $^1$H-NMR (200 MHz, $CDCl_3$) δ=1.37 (d, 3H, $CH\text{-}CH_3$); 1.75-2.20 (m, 2H, $CH_2\text{-}CH_2\text{-}CH$); 2.90-3.15 (m, 3H, $CH_2\text{-}CH_2\text{-}CH$); 3.29 (dd, 1H, H-6); 3.4-3.9 (m, 8H, Morpholin-$CH_2$); 4.18-4.35 (m, 2H, $CH\text{-}CH_3$ und H-5); 5.77 (mc, 2H, $CH_2\text{-}CH\text{-}CH_2$); 5.22-5.50 (m, 2H, $CH_2\text{-}CH{=}CH_2$); 5.88-6.10 (m, 1H, $CH_2\text{-}CH{=}CH_2$); 7.15-7.40 (m, 2H, Aromaten-H); 7.82 (d, 1H, Aromaten-H).

Stufe 3

Kalium-(1S,5R,6S)-6-[(1R)-1-hydroxyethyl]-[7-[(morpholin-4-yl)-carbonyl]-1,2,3,4-tetrahydronaphtho][2.1-a] carbapen-2-em-3-carboxylat

Analog zu Stufe 5 im Beispiel 1 wurden 73 mg (0.16 mmol) Amid mit 35.7 mg (0.194 mmol) Kalium-2-ethylhexanoat und 15 µl ( =.097 mmol) 2-Ethylhexansäure umgesetzt. Nach der Chromatographie an (R)LiChroprep RP 18 (Laufmittel: Wasser/Acetonitril, Gradient von Wasser zu Wasser/Acetonitril = 9/1) erhielt man 35 mg (48 %) Kaliumsalz.- $^1$H-NMR (200 MHz, $D_2O$): δ=1.35 (d, 3H, $CH\text{-}CH_3$); 1.75-2.05 und 2.10-2.30 (2 x m, 2 x 1H, $CH_2\text{-}CH_2\text{-}CH$); 3.00-3.18 (m, 2H, $CH_2\text{-}CH_2\text{-}CH$); 3.32 (mc, 1H, $CH_2CH_2\text{-}CH$); 3.45-4.02 (m, 9H, Morpholin-$CH_2$ und H-6); 4.33 (mc, 1H, $CH\text{-}CH_3$); 4.45 (dd, 1H, H-5); 7.32 (mc, 2H, Aromaten-H); 7.50 (d, 1H, Aromaten-H).

Beispiel 23

Natrium-(1S,5R,6S)-6-[(1R)-1-hydroxyethyl]-(6,7-dimethoxy-1,2,3,4-tetrahydronaphtho)[2,1-a]carbapen-2-em-carboxylat

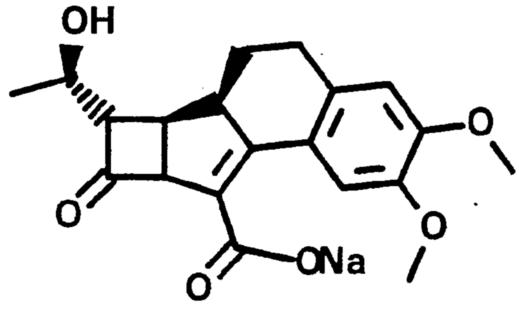

Stufe 1

(3S,4R)-2-[(1R)-1-tert.-Butyldimethylsilyloxyethyl]-4-[(2R)-6,7-dimethoxy-1-oxo-1,2,3,4-tetrahydronaphth-2-yl]-azetidin-2-on.

Wie für die Stufe 1 im Beispiel 16 beschrieben wurde, setzte man 4.0 g (19.4 mmol) 6,7-Dimethoxy-1-tetralon um. Nach Chromatographie an Kieselgel (Laufmittel Toluol/ Essigester= 3:1) erhielt man 1.3 g (32 %) Produkt.- $^1$H-NMR (200 MHz, $CDCl_3$): $\delta$ = 0.09 (s, 6H, $Si(CH_3)_2$); 0.85 (s, 9H, $SiC(CH_3)_3$); 1.24 (d, 3H, $CH\text{-}CH_3$); 1.90-2.10 (m, 1H, $CH\text{-}CH_2\text{-}CH_2$); 2.20-2.40 (m, 1H, $CH\text{-}CH_2\text{-}C_2$)); 2.60-2.78 (m, 1H, $CH\text{-}CH_2\text{-}CH_2$); 2.90-3.20 (m, 3H, $CH\text{-}CH_2\text{-}CH_2$ und H-3); 3.90 (s, 3H, $OCH_3$); 3.94 (s, 3H, $OCH_3$); 2.23 (mc, 1H, $CH\text{-}CH_3$); 4.44 (dd, 1H, H-4); 5.72 (bs, 1H, NH); 6.65 (s, 1H, Aromaten-H).

Stufe 2

[(3S,4R)-3-[(1R)-1-tert.-Butyldimethylsilyloxyethyl]-4-[(2R)-6,7-dimethoxy-1-oxo-1,2,3,4-tetrahydronaphth-2-yl]-2-oxoazetidin-1-yl]-2-oxoessigsäureallylester

Analog zur Stufe 2 im Beispiel 1 synthetisierte man aus 2.43 g (5.6 mmol) Azetidin-2-on 2.35 g (77 %) Allylester.- $^1$H-NMR (200 MHz, $CDCl_3$): $\delta$ = 0.05 (s, 3H, $Si(CH_3)$); 0.07 (s, 3H, $Si(CH_3)$); 0.83 (s, 9H, $SiC(CH_3)_3$); 1.18 (d, 3H, $CH\text{-}CH_3$); 1.90-2.14 (m, 1H, $CH\text{-}CH_2\text{-}CH_2$); 2.15-2.38 (m, 1H, $CH\text{-}CH_2\text{-}CH_2$); 2.90-3.40 (m, 4H, $CH\text{-}CH_2\text{-}CH_2$ und H-3); 3.89 (s, 3H, $OCH_3$); 3.92 (s, 3H, $OCH_3$); 4.32 (mc, 1H, $CH\text{-}CH_3$); 4.64 (mc, 1H, H-4); 4.81 (mc, 2H, $CH_2\text{-}CH{=}CH_2$); 5.25-5.52 (m, 2H, $CH_2\text{-}CH{=}CH_2$); 5.80-6.20 (m, 1H, $CH_2\text{-}CH{=}CH_2$); 6.65 (s, 1H, Aromäten-H); 7.50 (s, 1H, Aromaten-H).

Stufe 3

(1S,5R,6S)-6-[(1R)-1-tert.-Butyldimethylsilyloxyethyl]-(6,7-dimethoxy-1,2,3,4-tetrahydronaphtho)[2,1-a]carbapen-2-em-3-carbonsäureallylester

Die Zyklisierung von 2.35 g (4.6 mmol) Allylester wurde wie in Beispiel 1 beschrieben durchgeführt. Nach 20 Minuten bei 160°C wurde aufgearbeitet. Nach der Chromatographie erhielt man 230 mg (16 %) Produkt.- $^1$H-NMR (200 MHz, $CDCl_3$): $\delta$ = 0.09 (s, 6H, $Si((CH_3)_2)$; 0.91 (s, 9H, $SiC(CH_3)_3$); 1.23 (d, 3H, $CH\text{-}CH_3$); 1.80-2.20 (m, 2H, $CH\text{-}CH_2\text{-}CH_2$); 2.90-3.20 (m, 3H, $CH\text{-}CH_2\text{-}CH_2$); 3.26 (dd, 1H, H-3); 3.83 (s, 3H, $OCH_3$); 3.84 (s, 3H, $OCH_3$); 4.20-4.36 (m, 2H, $CH\text{-}CH_3$ und H-4); 4.78 (m, 2H, $CH_2\text{-}CH{=}CH_2$); 5.20-5.53 (m, 2H, $CH_2\text{-}CH{=}CH_2$); 5.83-6.13 (m, 1H, $CH_2\text{-}CH{=}CH_2$); 6.59 (s, 1H, Aromaten-H); 7.58 (s, 1H, Aromaten-H).

Stufe 4

(1S,5R,6S)-6-[(1R)-1-Hydroxyethyl](6,7-dimethoxy-1,2,3,4-tetrahydronaphtho)[2,1-a]-carbapen-2-em-3-carbonsäureallylester

Aus 375 mg (0.73 mmol) Silylether stellte man analog zur Stufe 4 im Beispiel 1 die Hydroxyethylverbindung

dar. Nach Säulenchromatographie an Kieselgel erhielt man 100 mg (34 %). $^1$H-NMR (200 MHz, $CDCl_3$): δ = 1.38 (d, 3H, $CH-CH_3$); 1.60-2.20 (m, 2H, $CH-CH_2-CH_2$); 2.30-3.22 (m, 3H, $CH-CH_2-CH_2$); 3.32 (dd, 1H, H-6); 3,86 (s, 6H, $OCH_3$); 4.20-4.40 (m, 2H, $CH-CH_3$ und H-5); 4.60-4.95 (m, 2H, $CH_2-CH=CH_2$); 5.20-5.58 (m, 2H, $CH_2-CH=CH_2$); 5.88-6.18 (m, 1H, $CH_2-CH=CH_2$); 6.60 (s, 1H, Aromaten-H); 7.59 (s, 1H, Aromaten-H).

Stufe 5

Natrium-(1S,5R,6S)-[(1R)-1-hydroxyethyl](6,7-dimethoxy-1,2,3,4-tetrahydronaphtho)[2,1-a]carbapen-2-em-3-carboxylat

Aus 90 mg (0.225 mmol) Allylester erhielt man 12 mg (14 %) Natriumsalz.- $^1$H-NMR (200 MHz, $D_2O$): δ = 1.34 (d, 3H, $CH-CH_3$); 1.60-1.90 (m, 1H, $CH-CH_2-CH_2$); 2.02-2.20 (m, 1H, $CH-CH_2-CH_2$); 2.90-3.10 (m, 2H, $CH-CH_2-CH_2$); 3.10-3.30 (m, 1H, $CH-CH_2-CH_2$); 3.58 (dd, 1H, H-6); 3.82 (s, 3H, $OCH_3$); 3.86 (s, 3H, $OCH_3$); 4.20-4.40 (m, 2H, $CH-CH_3$ und H-5); 6.86 (s, 1H, Aromaten-H); 7.24 (s, 1H, Aromaten-H).

Beispiel 24

Kalium-(1S,5R,6S)-6-[(1R)-1-hydroxyethyl]-(5-fluor-1,2,3,4-tetrahydronaphtho)[2,1-a]carbapen-2-em-3-carboxylat

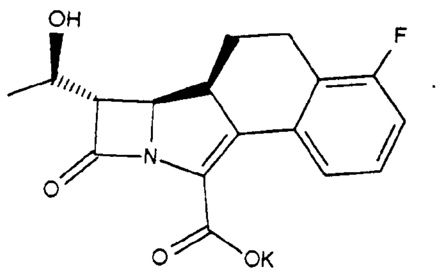

Stufe 1

(3S,4R)-3-[(1R)-1-tert.-Butyldimethylsilyloxyethyl]-4-[(2R)-5-fluor-1-oxo-1,2,3,4-tetrahydronaphth-2-yl]-azetidin-2-on

Wie für die Stufe 1 im Beispiel 16 beschrieben wurde, versetzte man bei-78°C eine Lösung von 3.28 g (20.0 mmol) 5-Fluor-1-tetralon in 100 ml THF mit 15 ml einer 1.5-molaren Lösung von Lithiumdiisopropylamid Tetrahydrofuran Komplex (22.5 mmol in Cyclohexan). Man rührte 10 min bei-78°C und 30 min bei 0°C. Bei-78°C wurden dann 24 ml einer 1-molaren Lösung von Chlortriisopropoxytitanat (24 mmol) in Hexan zugetropft und es wurde 70 min bei dieser Temperatur gerührt. Nach Zugabe von 5.75 g (20 mmol) (3S,4R)-4-Acetoxy-3-[(1R)-1 -tert.-butyldimethylsilyloxyethyl]-azetidin-2-on, gelöst in 10 ml THF, ließ man die Reaktion auf 0°C aufwärmen und rührte 30 min bei dieser Temperatur. Die Reaktionsmischung wurde auf 170 ml gesättigte Ammoniumchlorid-Lösung gegossen und mit Ethylacetat extrahiert. Die organische Phase wurde mit Wasser gewaschen, über Natriumsulfat getrocknet und im Vakuum eingeengt. Das Rohprodukt enthielt das (1S)- und (1R)-Diastereomere im Verhältnis 2/1. DerRückstand wurde an Kieselgel chromatographiert (Laufmittel: Toluol/Ethylacetat = 6/1) und das Produkt zur Trennung der Diastereomeren an (R)LiChroprep RP 18 (Laufmittel: Acetonitril/ Wasser = 3/1) chromatographier. Ausbeute: 1.05 g (13 %), weiße Kristalle.- $^1$H-NMR (270 MHz, $CDCl_3$): δ = 0.09 (s, 6H, $Si(CH_3)_2$); 0.88 (s, 9H, $SiC(CH_3)_3$); 1.24 (d, 3H, $CH-CH_3$); 1.99-2.17 (2 x m, 2H, $CH-CH_2-CH_2$); 2.70-3.01 (m, 3H, $CH-CH_2-CH_2$); 3.08 (dd, 1H, H-3); 4.21 (m, 1H, $CH-CH_3$); 4.42 (m, 1H, H-4); 5.78 (bs., 1H, NH); 7.20-7.41 (m, 2H, Aromaten-H); 7.82 (m, 1H, Aromaten-H).

Stufe 2

[(3S,4R)-3-[(1R)-1-tert.-Butyldimethylsilyloxyethyl]-4-[(2R)-5-fluor-1-oxo-1,2,3,4-tetrahydropahth-2-yl]-2-oxoazetidin-1-yl]-2-oxoessigsäureallylester

Analog zu Stufe 2 in Beispiel 1 wurden 0.95 g (2.42 mmol) des Azetidinons acyliert. Das Rohprodukt wurde durch Ausrühren mit Pentan (15 ml) gereinigt. Ausbeute: 0.55 g (46 %).- $^1$H-NMR (270 MHz, $CDCl_3$): δ = 0.06

und 0.08 (2 x s, 2 x 3H, $Si(CH_3)_2$); 0.84 (s, 9H, $SiC(CH_3)_3$); 1.19 (d, 3H, $CH-CH_3$); 1.90-2.40 (m, 2H, $CH-CH_2-CH_2$); 2.80-3.00 (m, 1H, $CH-CH_2-CH_2$); 3.18-3.39 (m, 3H, $CH-CH_2-CH_2$ und H-3); 4.36 (m, 1H, H-4); 4.80 (d, 2H, $CH_2-CH=CH_2$); 5.32 und 5.40 (2 x d, 2 x 1H, $CH_2-CH=CH_2$); 5.98 (m, 1H, $CH_2-CH=CH_2$); 7.20-7.40 (m, 2H, Aromaten-H); 7.84 (dd, 1H, Aromaten-H).

Stufe 3

(1S,5R,6S)-6-[(1R)-1-tert.-Butyldimethylsilyloxyethyl)-(5-fluor-1,2,3,4-tetrahydronaphtho)[2,1-a]carbapen-2-em-3-carbonsäureallylester

Analog zu Stufe 3 in Beispiel 1 wurden 0.55 g (1.09 mmol) Produkt aus Stufe 2 in 20 ml Mesitylen bei 160°C zyklisiert. Nach 45 min bei dieser Temperatur wurde aufgearbeitet und das Rohprodukt chromatographiert (Laufmittel: Toluol/Essigester = 50/1). Ausbeute: 460 mg (89 %).- $^1$H-NMR (270 MHz, $CDCl_3$): δ = 0.10 (s, 6H, $Si(CH_3)_2$); 0.90 (s, 9H, $SiC(CH_3)_3$); 1.27 (d, 3H, $CH-CH_3$); 1.80-2.13 (m, 2H, $CH-CH_2-CH_2$); 2.95-3.04 (m, 2H, $CH-CH_2-CH_2$); 3.17 (m, 1H, $CH-CH_2-CH_2$); 3.28 (dd, 1H, H-6); 4.20-4.34 (m, 2H, H-5 und $CH-CH_3$); 4.77 (m, 2H, $CH_2-CH=CH_2$); 5.25 und 5.41 (2 x s, 2 x 1H, $CH_2-CH=CH_2$); 5.99 (m, 1H, $CH_2-CH=CH_2$); 6.81 (dd, 1H, Aromaten-H); 7.03 (d, 1H, Aromaten-H); 7.40 (d, 1H, Aromaten-H).

Stufe 4

(1S,5R,6S)-6-[(1R)-1-Hydroxyethyl]-(5-fluor-1,2,3,4-tetrahydronaphtho)[2,1-a]-carbapen-2-em -3-carbonsäureallylester

Wie für die Stufe 4 in Beispiel 1 beschrieben wurden 450 mg (0.95 mmol) umgesetzt. Das Rohprodukt wurde an Kieselgel chromatographiert (Laufmittel: Toluol/ Ethylenacetat = 1/1). Ausbeute: 240 mg (71 %). Die Substanz wurde sofort weiter umgesetzt.

Stufe 5

Kalium-(1S,5R,6S)-6-[(1R-)-1-hydroxyethyl]-(5-fluor-1,2,3,4-tetrahydronaphtho)[2,1-a]carbapen-2-em-3-carboxylat

Analog zu Stufe 5 in Beispiel 1 wurden 240 mg (0.67 mmol) umgesetzt. Das Rohprodukt wurde über (R-)LiChroprep RP 18 chromatographiert (Laufmittel: Wasser). Ausbeute: 57 mg (24 %).- $^1$H-NMR (200 MHz, $D_2O$); δ = 1.28 (d, 3H, $CH-CH_3$); 1.71 -1.96 und 2.17-2.30 (2 x m, 2 x 1H, $CH_2-CH_2-CH$); 2.85-2.98 (m, 1H, $CH_2-CH_2-CH$); 3.07-3.38 (m, 2H, $CH_2-CH_2-CH$); 3.61 (dd, 1H, H-6); 4.23-4.38 (m, 1H, $CH-CH_3$); 4.42 (dd, 1H, H-5); 6.97-7.37 (m, 3H, Aromaten-H).

Beispiel 25

Pivaloyloxymethyl-(1S,5R,6S)-6-[(1R)-1-hydroxyethyl]-(thiochromano)-[1,2-c]carbapen-2-em-3-carboxylat

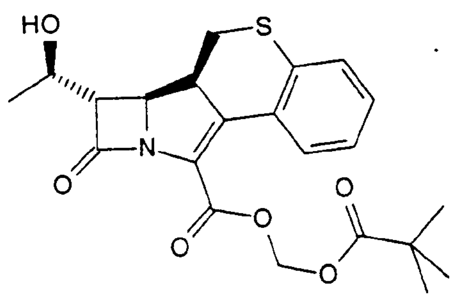

Wie in Beispiel 19 beschrieben wurden 18 mg (0.12 mmol) Chlormethylpivaloat mit 12 mg (0.12 mmol) Natriumbromid versetzt und anschließend mit 18 mg (0.05 mmol) Kalium-(1S,5R,6S)-6-[(1R)-1-hydroxyethyl]-(thiochromano)[1,2-c]-carbapen-2-em-3-carboxylat (Beispiel 8) umgesetzt. Man erhält 15 mg (81 %) der Titelverbindung.- $^1$H-NMR (200 MHz, $CDCl_3$): δ = 1.25 (s, 9H, $C(CH_3)_3$); 1.36 (d, 3H, $CH-CH_3$); 3.00-3.45 (m, 4H, $S-CH_2-CH$ und H-6); 4.36 (mc, 1H, $CH-CH_3$); 4.41 (dd, 1H, H-5); 5.91 (mc, 2H, $OCH_2$); 6.98-7.27 (m, 3H, Aromaten-H); 7.50 (d, 1H, Aromanten-H).

Beispiel 26

Kalium-(1S,5R,6S)-6-[(1R)-1-hydroxyethyl]-(1,2,3,4-tetrahydronaphtho)[2,1-a]-carbapen-2-em-3-carboxylat

Stufe 1

(3S,4R)-3-[(1R)-1-Hydroxyethyl]-4-[(2R)-1-oxo-1,2,3,4-tetrahydronaphth-2-yl]-azetidin-2-on

1.6 g (4.3 mmol) der Stufe 1 aus dem Beispiel 1 wurden in 8.5 ml Acetonitril gelöst und bei 0°C mit 0.96 ml Bortrifluorid-Etherat versetzt. Nach 15 Minuten bei 0°C war die Umsetzung vollständig und es wurden je 10 ml Ethylacetat und Wasser zugesetzt. Der pH-Wert wurde auf 7.0 eingestellt und die wäßrige Phase nochmals mit Ethylacetat extrahiert. Nach dem Trocknen über Magnesiumsulfat wurde im Vakuum zur Trockne eingeengt. Ausbeute: 805 mg (73 %) eines Öls, das beim Stehen im Kühlschrankt fest wurde.- $^1$H-NMR (200 MHz, $CDCl_3$): $\delta$ = 1.40 (d, 3H, $CH\text{-}CH_3$); 1.85-2.08 und 2.19-2.36 (2 x m, 2H, $CH\text{-}CH_2\text{-}CH_2$); 2.73 (mc, 1H, $CH\text{-}CH_2\text{-}CH_2$); 3.03-3.18 (m, 3H, H-3 und $CH\text{-}CH_2CH_2$); 3.85 (dd, 1H, H-4); 4.20 (mc, 1H, $CH\text{-}CH_3$); 4.45 (bs, 1H, OH); 6.17 (bs, 1H, NH); 7.25-7.38 und 7.48-7.59 (2xm, 3H, Aromaten-H); 8.02 (dd, 1H, Aromaten-H).

Stufe 2

(3S,4R)-3-[(1R)-1-Triethylsilyloxyethyl]-4-[(2R)-1-oxo-1,2,3,4-tetrahydronaphth-2-yl]azetidin-2-on

Zu 100 mg (0.39 mmol) der Hydroxyethylverbindung aus Stufe 1 in 3 ml wasserfreiem Methylenchlorid gab man bei 0°C nacheinander 0.06 ml Pyridin und 0.1 ml Triethylchlorsilan und rührte anschließend 3 h bei Zimmertemperatur. Die Reaktionsmischung wurde mit 10 ml Methylenchlorid verdünnt und nacheinander mit verdünnter Salzsäure, gesättigter $NaHCO_3$-Lösung und Wasser gewaschen. Nach dem Trocknen über Natriumsulfat und dem Einengen im Vakuum wurde der Rückstand an Kieselgel chromatographiert (Laufmittel: Toluol/Ethylacetat = 1/2). Ausbeute: 122 mg (85 %).- $^1$H-NMR (200 MHz, $CDCl_3$); $\delta$ = 0.50-0.70 (2 x m, 6H und 9H, $SiCH_2CH_3$); 1.28 (d, 3H, $CH\text{-}CH_3$); 1.95-2.38 (m, 2H, $CH\text{-}CH_2\text{-}CH_2$); 2.76 (dt, 1H, $CH\text{-}CH_2\text{-}CH_2$); 3.02-3.16 (m, 3H, $CH\text{-}CH_2\text{-}CH_2$ und H-3); 4.23 (mc, 1H, $CH\text{-}CH_3$); 4.43 (dd, 1H, H-4); 5.8 (bs, 1H, NH); 7.22-7.38 und 7.45-7.57 (2 x m, 3H, Aromaten-H); 8.02 (dd, 1H, Aromaten-H).

Die weiteren Umsetzungen wurden, wie im Beispiel 3 beschrieben, durchgeführt.

Beispiel 27

[1-(Ethoxycarbonyloxy)ethyl]-(1S,5R,6S)-6-[(1R)-1-hydroxyethyl]-(1,2,3,4-tetrahydronaphtho)[2,1-a]carbapen-2-em-3-carboxylat

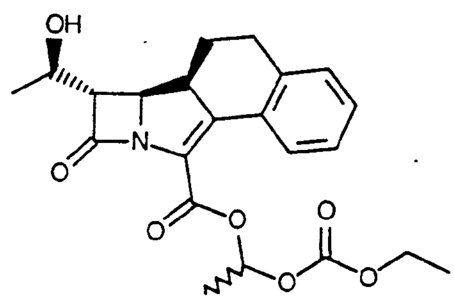

100 mg (0.30 mmol) Kalium-(1S,5R,6S)-6-[(1R)-1-hydroxyethyl]-(1,2,3,4-tetrahydronaphtho)[2,1-a]carbapen-2-em-3-carboxylat (Beispiel 1) wurden in 2.5 ml wasserfreiem DMF gelöst und bei 0°C mit 145 mg (0.59 mmol) 1-(Ethyloxycarbonyloxy)ethyliodid versetzt. Nach 30 min wurde der Reaktionsansatz in 10 ml Wasser aufgenommen und zweimal mit je 20 ml Ethylacetat extrahiert. Die organischen Phasen wurden über Magnesiumsulfat getrocknet, im Vakuum eingeengt und der Rückstnd über (R)LiChroprep RP 18 chromatographiert (Laufmittel: Wasser/Acetonitril-Gradient). Ausbeute: 60 mg (49 %).- $^1$H-NMR (200 MHz, DMSO): $\delta$ = 1.17 (d, 3H, $CH\text{-}CH_3$); 1.24 (t, 3H, $OCH_2\text{-}CH_3$); 1.47 (d, 3H, $O_2CH\text{-}CH_3$); 1.75-2.12 (m, 2H, $CH\text{-}CH_2\text{-}CH_2$); 3.02 (mc, 2H, $CH\text{-}CH_2\text{-}CH_2$); 3.18-3.33 (m, 1H, H-6); 3.40 (mc, 1H, $CH\text{-}CH_2\text{-}CH_2$); 4.03 (mc, 1H, $CH\text{-}CH_3$); 4.19 (q, 2H,

$OCH_2$-$CH_3$); 4.30 und 4.35 (2 x dd, 1H, H-5); 5.12 (d, 1H, OH); 6.93 (mc, 1H; OCH ($CH_3$)O; 7.04-7.34 und 7.48-7.70 (2 x m, 4H, Aromaten-H).

Beispiel 28

[1-(Isobutyloxycarbonyloxy)ethyl]-(1S,5R,6S)-6-[(1R)-1-hydroxyethyl]-(1,2,3,4-tetrahydronaphtho)[2,1-a]-carbapen-2-em-3-carboxylat

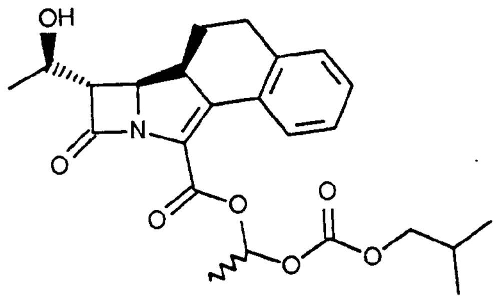

Wie in Beispiel 27 beschrieben wurden 100 mg (0.30 mmol) Kaliumsalz und 157 mg (0.58 mmol) 1-(Isobutyloxycarbonyloxy)ethyliodid umgesetzt. Nach Chromatographie an (R)LiChroprep RP 18 erhielt man 52 mg (40 %) des Prodrugesters.- $^1$H-NMR (200 MHz, DMSO): $\delta$ = 0.93 (2 x d, 6H, CH($CH_3$)$_2$); 1.06 (d, 3H, CH-$CH_3$); 1.27-1.41 (m, 4H, OCH($CH_3$)O und CH-($CH_3$)$_2$); 1.77-2.10 (m, 2H, CH-$CH_2$-$CH_2$); 3.02 (mc, 2H, CH-$CH_2$-$CH_2$); 3.20-3.32 (m, 1H, H-6); 3.36-3.45 (mc, 1H, CH-$CH_2$-$CH_2$); 3.80-4.08 (m, 3H, CH-$CH_3$ und $OCH_2$-CH); 4.30 und 4.36 (2 x dd, 1H, H-5); 5.08 und 5.13 (2 x d, 1H, OH); 6.82 (mc, 1H, OCH($CH_3$)O; 7.04-7.33 (m, 3H, Aromaten-H); 7.60 (d, 1H, Aromaten-H).

Beispiel 29

[1-(sec.-Butyloxycarbonyloxy)ethyl]-(1S,5R,6S)-6-[(1R)-1-hydroxyethyl]-(1,2,3,4-tetrahydronaphtho)[2,1-a] carbapen-2-em-3-carboxylat

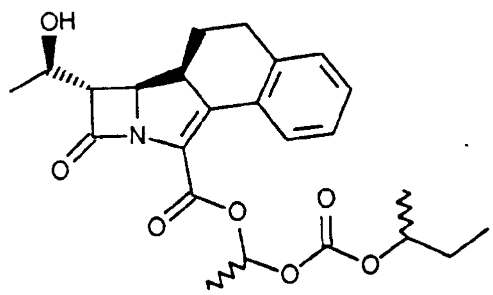

Analog Beispiel 27 wurden 100 mg (0.30 mmol) Kaliumsalz bei 0°C mit 157 mg (0.58 mmol) 1-(sec.-Butyloxycarbonyloxy)ethyliodid umgesetzt. Nach Reinigung über (R)LiChroprep RP 18 konnte das Produkte als Öl erhalten werden. Ausbeute: 72 mg (55 %).- $^1$H-NMR (200 MHz, DMSO): $\delta$ = 0.94 (t, 3H, $CH_2$-$CH_3$); 1.10-1.28 (m, 6H, CH-$CH_3$ und CH($CH_3$)$CH_2$); 1.47 (d, 3H, OCH($CH_3$)O; 1.58 (mc, 2H, $CH_2$-$CH_3$); 1.86-2.10 (m, 2H, CH-$CH_2$-$CH_2$); 3.02 (mc, 2H, CH-$CH_2$-$CH_2$); 3.19-3.30 (m, 1H, H-6); 3.40 (mc, 1H, CH-$CH_2$-$CH_2$); 4,03 (mc, 1H, CH-$CH_3$); 4.27 und 4.35 (2 x dd, 1H, H-5); 4.65 (mc, 1H, CH($CH_3$)$CH_2$); 5.08 und 5.12 (2xd, 1H, OH); 6.38 (mc, 1H, OCH($CH_3$)O; 7.07-7.32 (m, 3H, Aromaten-H); 7.60 (d, 1H, Aromaten-H).

Beispiel 30

[1-(Butyloxycarbonyloxy)ethyl]-(1S,5R,6S)-6-[(1R)-1-hydroxyethyl]-(1,2,3,4-tetrahydronaphtho)[2,1-a]carbapen-2-em-3-carboxylat

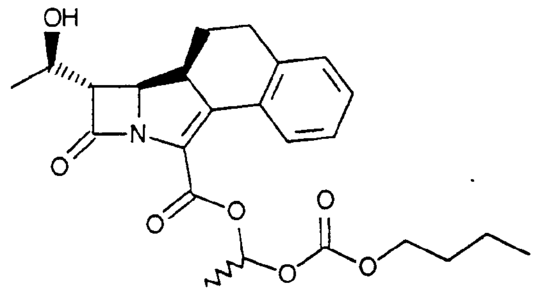

Wie in Beispiel 27 beschrieben wurden 100 mg (0.30 mmol) Kaliumsalz mit 157 mg (0.58 mmol) 1-(Butyloxycarbonyloxy)ethyliodid umgesetzt. Nach Chromatographie über (R)LiChroprep RP 18 erhielt man 44 mg (34 %) Produkt.- $^1$H-NMR (200 MHz, DMSO): δ = 0.90 (t, 3H, $CH_2$-$CH_3$); 1.15 (d, 3H, CH-$CH_3$); 1.20-1.66 (m, 7H, OCH($CH_3$)O und $CH_2$-$CH_2$-$CH_3$); 1.75-2.13 (m 2H,, CH-$CH_2$-$CH_2$); 3.03 (mc, 2H, CH-$CH_2$-$CH_2$); 3.23-3.42 (m, 2H, H-6 und CH-$CH_2$-$CH_2$); 4.02 (mc, 1H, CH-$CH_3$); 4.15 (t, 2H, $OCH_2$); 4.28 und 4.33 (2 x dd, 1H, H-5); 5.08 und 5.11 (2 x s, 1H, OH); 6.83 (mc, 1H, OCH($CH_3$)O; 7.07-7.34 (m, 3H, Aromaten-H); 7.60 (d, 1H, Aromaten-H).

Beispiel 31

Kalium-(1S,5R,6S)-6-[(1R)-1-hydroxyethyl]-(6-methoxy-1,2,3,4-tetrahydronaphtho)[2,1-a]carbapen-2-em-3-carboxylat

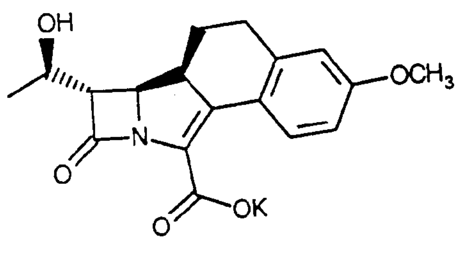

Stufe 1

(3S,4R)-3-[(1R)-1-tert.-Butyldimethylsilyloxyethyl]-4-[(2R)-6-methoxy-1-oxo-1,2,3,4-tetrahydronaphth-1-yl]-azetidin-2-on

Analog zu Stufe 1 in Beispiel 16 wurden 50 g (284 mmol) 6-Methoxytetralon mit 71 g (247 mmol) 4-Acetoxy-3[(1R)-1-tert.-butyldimethylsilyloxyethyl]-azetidin-2-on umgesetzt. Nach Chromatographie über Kieselgel (Laufmittel: Toluol/Ethylacetat = 2/1) und anschließender Kristallisation aus n-Heptan wurden 24.5 g (25 %) des β-Isomeren isoliert. - $^1$H-NMR (270 MHz, $CDCl_3$): δ = 0.09 (s, 6H, $SiCH_3$); 0.89 (s, 9H, $SiC(CH_3)_3$); 1.27 (d, 3H, CH-$CH_3$); 1.95-2.06 und 2.20-2.33 (2 x m, 2H, CH-$CH_2$-$CH_2$); 2.69 (mc, 1H, CH-$CH_2$-$CH_2$); 3.01-3.12 (m, 3H, CH-$CH_2$-$CH_2$ und H-3); 3.87 (s, 3H, $OCH_3$); 4.26 (mc, 1H, CH-$CH_3$); 4.45 (dd, 1H, H-4); 5.72 (bs, 1H, NH); 6.71 (d, 1H, Aromaten-H); 6.84 (mc, 1H, Aromaten-H); 7.99 (d, 1H, Aromaten-H).

Stufe 2

[(3S,4R)-3-[(1R)-1-tert.-Butyldimethylsilyloxyethyl]-4-[(2R)-6-methoxy-1-oxo-1,2,3,4-tetrahydronaphth-2-yl]-2-oxoazetidin-1-yl]-2-oxoessigsäureallylester.

Analog zu Stufe 2 in Beispiel 1 wurden 2.4 g (5.95 mmol) des Azetidinons mit Oxalsäureallylesterchlorid umgesetzt. Nach Chromatographie an Kieselgel (Laufmittel: Petrolether/Ethylacetat = 4/1) erhielt man 2.4 g

(78 %) des Produktes als hellgelben Feststoff.- $^1$H-NMR ($CDCl_3$, 200 MHz): δ = 0.07 und 0.08 (2 x s, 6H, $SiCH_3$); 0.84 (s, 9H, $Si(CH_3)_3$); 1.18 (d, 3H, $CH-CH_3$); 1.90-2.32 (m, 2H, $CH-CH_2-CH_2$); 3.02-3.23 (m, 3H, $CH-CH_2-CH_2$); 3.34 (dd, 1H, H-3); 3.85 (s, 3H, $OCH_3$); 4.35 (mc, 1H, $CH-CH_3$); 4.65 (dd, 1H, H-4); 4.80 (mc, 2H, $CH_2-CH=CH_2$); 5.36 (mc, 2H, $CH_2-CH=CH_2$); 5.97 (mc, 1H, $CH_2-CH=CH_2$); 6.70 (d, 1H, Aromaten-H); 6.94 (mc, 1H, Aromaten-H); 8.00 (d, 1H, Aromaten-H).

Stufe 3

(1S,5R,6S)-6-[(1R)-1-tert.-Butyldimethylsilyloxyethyl]-(6-methoxy-1,2,3,4-tetrahydronaphtho)[2.1-a]carbapen-2-em-3-carbonsäureallylester.

Wie in Stufe 3 von Beispiel 1 beschrieben wurden 2.4 g (4.6 mmol) Allylester in wasserfreiem Mesitylen zyklisiert. Nach Chromatographie an Kieselgel (Laufmittel: Toluol/ Ethylacetat = 30/1) wurden 750 mg (33 %) Produkt isoliert.- $^1$H-NMR (200 MHz, $CDCl_3$): δ = 0.07 (s, 6H, $SiCH_3$); 0.90 (s, 9H, $Si(CH_3)_3$); 1.27 (d, 3H, $CH-CH_3$); 1.80-2.14 (m, 2H, $CH-CH_2-CH_2$); 3.03 (mc, 2H, $CH-CH_2-CH_2$); 3.10 (mc, 1H, $CH-CH_2-CH_2$); 3.25 (dd, 1H, H-6); 3.80 (s, 3H, $OCH_3$); 4.15-4.34 (m, 2H, $CH-CH_3$ und H-5); 4.78 (mc, 2H, $CH_2-CH=CH_2$); 5.34 (mc, 2H, $CH_2-CH=CH_2$); 5.97 (mc, 1H, $CH_2-CH=CH_2$); 6.60-6.77 (m, 2H, Aromaten-H); 7.83 (d, 1H, Aromaten-H).

Stufe 4

(1S,5R,6S)-6-[(1R)-1-Hydroxyethyl]-(6-methoxy-1,2,3,4-tetrahydronaphtho)[2,1-a]carbapen-2-em-3-carbonsäureallylester.

Aus 500 mg (1.03 mmol) Silylether stellte man analog zu Stufe 4 in Beispiel 1 die Hydroxyethylverbindung her. Ausbeute: 130 mg (34 %).- $^1$H-NMR (200 MHz, DMSO): δ = 1.16 (d, 3H, $CH-CH_3$), 1.72-2.08 (m, 2H, $CH-CH_2-CH_2$); 2.98 (mc, 2H, $CH-CH_2-CH_2$); 3.09-3.27 (m, 1H, $CH-CH_2-CH_2$); 3.34 (d, 1H, H-6); 3.75 (s, 3H, $OCH_3$); 4.00 (mc, 1H, $CH-CH_3$); 4.28 (dd, 1H, H-5); 4.69 (mc, 2H, $CH_2-CH=CH_2$); 5.07 (d, 1H, OH); 5.19-5.43 (mc, 2H, $CH_2-CH=CH_2$); 5.93 (mc, 1H, $CH_2-H=CH_2$); 6.63-6.76 (m, 2H, Aromaten-H); 7.60 (d, 1H, Aromaten-H).

Stufe 5

Kalium-(1S,5R,6S)-6-[(1R)-1-hydroxyethyl-]-(6-methoxy-1,2,3,4-tetrahydronaphtho)[2,1-a]carbapen-2-em-3-carboxalat

Analog zu Stufe 5 in Beispiel 1 wurden 114 mg (0.308 mmol) Allylester umgesetzt. Nach Chromatographie an $^{(R)}$LiChroprep RP 18 (Laufmittel: Wasser, Gradient Acetonitril 0-15 %) und Gefriertrocknung erhielt man 48 mg (42 %) des Kaliumsalzes.- $^1$H-NMR (200 MHz, DMSO): δ = 1.15 (d, 3H, $CH-CH_3$); 1.62 und 1.94 (2 x mc, 2H, $CH-CH_2-CH_2$); 2.75-3.00 (m, 3H, $CH-CH_2-CH_2$); 3.16 (dd, 1H, H-6); 3.69 (s, 3H, $OCH_3$); 3.95 (mc, 1H, $CH-CH_3$); 4.07 (dd, 1H, H-5); 4.96 (bs, 1H, OH); 6.52-6.73 (m, 2H, Aromaten-H); 7.68 (d, 1H, Aromaten-H).

## Patentansprüche

1. β-Lactam-Antibiotika der Formel I und deren pharmazeutisch verträgliche Salze,

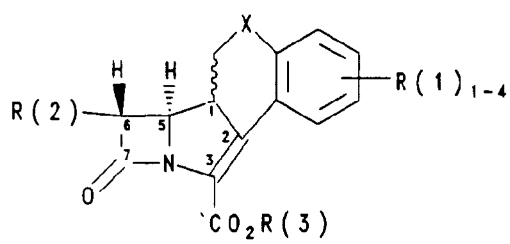

I

worin bedeuten

X: $(CH_2)_n$ mit n = 0, 1, 2;

CR(a)R(b), wobei R(a) und R(b) voneinander unabhängig aus den folgenden Gruppen ausgewählt werden können: H; $(C_1-C_6)$-Alkyl; Aryl, ausgewählt aus der Gruppe unsubstituiertes und durch $(C_1-C_4)$-Alkyl, F,

Cl, Br, $O(C_1-C_4)$-Alkyl, OH, $OCO(C_1-C_4)$-Alkyl, $NH_2$, $NH(C_1-C_4)$-Alkyl, $OCOC_6H_5$, $NHC_6H_5$ substituiertes Phenyl und Naphthyl; Heteroaryl, ausgewählt aus der Gruppe unsubstituierter oder durch $(C_1-C_4)$-Alkyl, F, Cl, Br, $O(C_1-C_4)$-Alkyl, OH, $OCO(C_1-C_4)$-Alkyl, $NH_2$, $NH(C_1-C_4)$-Alkyl, $OCOC_6H_5$, $NHC_6H_5$ substituierter 5- bis 6-gliedriger Ring mit 1 bis 4 N-, O-, S-Atomen;

O;

$SO_n$ mit n = 0, 1, 2;

NR(c), wobei R(c) aus der Gruppe bestehend aus H, $(C_1-C_6)$-Alkyl, Aryl, $CO(C_1-C_6)$-Alkyl, CO-Aryl, CO-Heteroaryl, $(C_1-C_6)$-Alkoxycarbonyl, $(C_1-C_6)$-Alkylsulfonyl und Arylsulfonyl ausgewählt ist,

R(1) : bis zu vier Substituenten, die gleich oder verschieden sind, ausgewählt aus der Gruppe, bestehend aus H, $(C_1-C_6)$-Alkyl,

ein Substituent aus der Gruppe bestehend aus Aryl, Heteroaryl, OH, SH, $SO_n(C_1-C_6)$-Alkyl (mit n = 0, 1, 2), NR(b)R(c), (wobei R(b) und R(c) wie oben definiert sind), CN, $NO_2$, C(R(a)) = NOR(b), (wobei R(a) und R(b) wie oben definiert sind),

bis zu zwei Substituenten aus der Gruppe $CF_3$, F, Cl, Br, I, $O(C_1-C_6)$-Alkyl, $OCO(C_1-C_6)$-Alkyl, OCONR(d)R(e), (wobei R(d) und R(e) voneinander unabhängig aus den folgenden Gruppen ausgewählt werden: Wasserstoff, $(C_1-C_6)$-Alkyl, NR(d)R(e) kann auch einem 5- oder 6-gliedrigen Ringsystem entsprechen), $SO_2NR(d)R(e)$, (wobei R(d) und R(e) wie oben definiert sind), $CO(C_1-C_6)$-Alkyl, COAryl, $CO_2H$, $CO_2(C_1-C_6)$-Alkyl, CONR(d)R(e), (wobei R(d) und R(e) wie oben definiert sind), $CH_2R(f)$, (wobei R(f) aus den folgenden Gruppen ausgewählt ist: Hydroxy, $(C_1-C_6)$-Alkoxy, Acyloxy, Aryloxy, Heteroaryloxy, $(C_1-C_6)$-Alkylthio, Arylthio, Heteroarylthio und die daraus ableitbaren Sulfinyl- und Sulfonylverbindungen, NR(b)R(c), wobei R(b) und R(c) wie oben definiert sind. NR(b)R(c) kann zusätzlich Teil eines zyklischen und heterozyklischen Systems sein);

$NHCO(C_1-C_6)$-Alkyl; $NHCOC_6H_5$, NHCO-Napthyl;

R(2): H, $(C_1-C_4)$-Alkyl, $CH_2OH$, $CH_2OCOR(a)$, $CH(OH)CH_3$, $CH(OCOR(a))CH_3$, $CH_2NR(b)R(c)$, $CH(NR(b)R(c))CH_3$, $C(CH_3)=NR(a)$, $CH[^{\oplus}NR(g)R(h)R(i)]CH_3$, wobei R(a), R(b) und R(c) wie unter R(1) definiert sind und R(g), R(h) und R(i) voneinander unabhängig sind und $(C_1-C_6)$-Alkylgruppen darstellen; $NH_2$; NHR(a)R(b); $NHCO(C_1-C_6)$-Alkyl; $NHCOC_6H_5$, NHCO-Napthyl;

R(3): H, $(C_1-C_3)$-Alkyl-$OCO(C_1-C_6)$-Alkyl, $(C_1-C_3)$-Alkyl-$OCO_2(C_1-C_6)$-Alkyl, 5-Methyl-1,3-dioxolen-2-on-4-yl-methyl,

und in denen die bevorzugte Stereochemie in Position 5 gleich R und in Position 6 gleich S ist.

**2.** β-Lactam-Antibiotika der Formel I und deren pharmazeutisch verträgliche Salze,

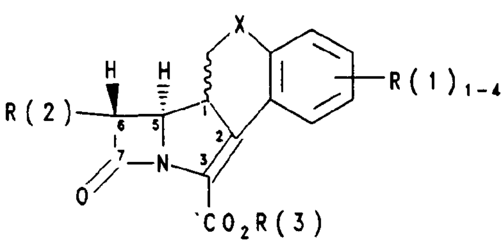

I

worin bedeuten:

X: $(CH_2)_n$ mit n = 0, 1, 2;

CR(a)R(b), wobei R(a) und R(b) voneinander unabhängig aus den folgenden Gruppen ausgewählt werden können: H; $(C_1-C_6)$-Alkyl; Aryl, ausgewählt aus der Gruppe unsubstituiertes und durch $(C_1-C_4)$-Alkyl, F, Cl, Br, $O(C_1-C_4)$-Alkyl, OH, $OCO(C_1-C_44)$-Alkyl, $NH_2$, $NH(C_1-C_4)$-Alkyl, $OCOC_6H_5$, $NHC_6H_5$ substituiertes Phenyl und Naphthyl; Heteroaryl, ausgewählt aus der Gruppe unsubstituierter oder durch $(C_1-C_4)$-Alkyl, F, Cl, Br, $O(C_1-C_4)$-Alkyl, OH, $OCO(C_1-C_4)$-Alkyl, $NH_2$, $NH(C_1-C_4)$-Alkyl, $OCOC_6H_5$, $NHC_6H_5$ substituierter 5- bis 6-gliedriger Ring mit 1 bis 4 N-, O-, S-Atomen;

O;

$SO_n$ mit n = 0, 1, 2;

NR(c), wobei R(c) aus der Gruppe bestehend aus H, $(C_1-C_6)$-Alkyl, Aryl, $CO(C_1-C_6)$-Alkyl, CO-Aryl, CO-Heteroaryl, $(C_1-C_6)$-Alkoxycarbonyl, $(C_1-C_6)$-Alkylsulfonyl und Arylsulfonyl ausgewählt ist,

R(1) : bis zu vier Substituenten, die gleich oder verschieden sind, ausgewählt aus der Gruppe, bestehend aus H, $(C_1-C_6)$-Alkyl,

ein Substituent aus der Gruppe bestehend aus Aryl, Heteroaryl, OH, SH, $SO_n(C_1-C_6)$-Alkyl (mit n = 0, 1, 2), NR(b)R(c), (wobei R(b) und R(c) wie oben definiert sind), CN, $NO_2$, C(R(a)) = NOR(b), (wobei

R(a) und R(b) wie oben definiert sind),

bis zu zwei Substituenten aus der Gruppe $CF_3$, F, Cl, Br, I, O($C_1$-$C_6$)-Alkyl, OCO($C_1$-$C_6$)-Alkyl, OCONR(d)R(e), (wobei R(d) und R(e) voneinander unabhängig aus den folgenden Gruppen ausgewählt werden: Wasserstoff, ($C_1$-$C_6$)-Alkyl, NR(d)R(e) kann auch einem 5- oder 6-gliedrigen Ringsystem entsprechen), $SO_2$NR(d)R(e), (wobei R(d) und R(e) wie oben definiert sind), CO($C_1$-$C_6$)-Alkyl, COAryl, $CO_2H$, $CO_2$($C_1$-$C_6$)-Alkyl, CONR(d)R(e), (wobei R(d) und R(e) wie oben definiert sind), $CH_2$R(f), (wobei R(f) aus den folgenden Gruppen ausgewählt ist: Hydroxy, ($C_1$-$C_6$)-Alkoxy, Acyloxy, Aryloxy, Heteroaryloxy, ($C_1$-$C_6$)-Alkylthio, Arylthio, Heteroarylthio und die daraus ableitbaren Sulfinyl- und Sulfonylverbindungen, NR(b)R(c), wobei R(b) und R(c) wie oben definiert sind. NR(b)R(c) kann zusätzlich Teil eines zyklischen und heterozyklischen Systems sein);

NHCO($C_1$-$C_6$)-Alkyl; $NHCOC_6H_5$, NHCO-Napthyl;

R(2): H, ($C_1$-$C_4$)-Alkyl, $CH_2OH$, $CH_2$OCOR(a), CH $(OH)CH_3$, CH (OCOR(a))$CH_3$, $CH_2$NR(b)R(c), CH(NR(b)R(c))$CH_3$, C($CH_3$) =NR(a), CH[$^{\oplus}$NR(g)R(h)R(i)]$CH_3$, wobei R(a), R(b) und R(c) wie unter R(1) definiert sind und R(g), R(h) und R(i) voneinander unabhängig sind und ($C_1$-$C_6$)-Alkylgruppen darstellen; $NH_2$; NHR(a)R(b); NHCO($C_1$-$C_6$)-Alkyl; $NHCOC_6H_5$, NHCO-Napthyl;

R(3): H, ($C_1$-$C_3$)-Alkyl-OCO($C_1$-$C_6$)-Alkyl, ($C_1$-$C_3$)-Alkyl-$OCO_2$($C_1$-$C_6$)-Alkyl, 5-Methyl-1,3-dioxolen-2-on-4-yl-methyl,

und in denen die bevorzugte Stereochemie in Position 5 gleich R und in Position 6 gleich S ist, wobei jedoch Verbindungen ausgenommen sind, in denen R(1) viermal Wasserstoff bedeutet.

3. Verbindungen I nach Anspruch 1, in denen die Substituenten die folgenden Bedeutungen haben:

X: $CH_2$; $C(CH_3)_2$; CH-Phenyl; O; $SO_n$ mit n = 0, 1, 2; $NSO_2CH_3$; $NSO_2$-$C_6H_4$-$CH_3$; wovon $CH_2$ und $SO_n$ mit n = 0, 1, 2 besonders bevorzugt sind.

R(1): H, $CH_3$, $C_6H_5$,

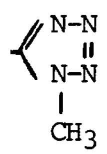 ,

F, Cl, Br, $OCH_3$, OH, $OCOCH_3$,

OCONH-$C_6H_5$, $NH_2$, $NHCOCH_3$, $NHSO_2CH_3$, $NHSO_2$-$C_6H_4$-$CH_3$, $NHCOCH_2NH_2$, CN, $COCH_3$, $C(CH_3)$=NOH, $CO_2H$, $CO_2CH_3$, $CONH_2$,

$CON(CH_3)_2$,

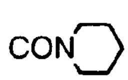 , 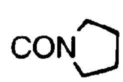 , 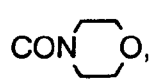, 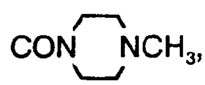,

$CONHCH_2CO_2H$, $CH_2OCOCH_3$, $CH_2SCH_2CH_3$, $CH_2SCH_2CH_2NH_2$, $CH_2SCH_2CH_2NHCH(=NH)$, $CH_2SC_6H_5$,

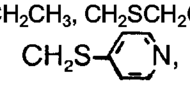,

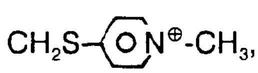,

$CH_2N(CH_3)_2$, $CH_2NHCOCH_2NH_2$,

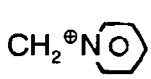 ,

$CH_2OH$,

R(2): H, $CH_3$, $CH_2CH_3$, $CH_2OH$, CH $(OH)CH_3$, $CH(OCOCH_3)CH_3$, CH($OCOCH_2$-$C_6H_5$)$CH_3$, CH-($OCOCH_2O$-$C_6H_5$)$CH_3$, $CH(NH_2)CH_3$, CH[$N^{\oplus}(CH_3)_3$]$CH_3$, $CH(NHCOCH_3)CH_3$, $CH(NHSO_2CH_3)CH_3$, CH-($NHSO_2C_6H_5$)$CH_3$, $CH(NHCOCH_2NH_2)CH_3$, wovon $CH(OH)CH_3$ mit der R-Konfiguration besonders bevorzugt ist,

R(3): H, $CH_2OCOCH_3$, $CH_2OCOCH_2CH_3$, $CH_2OCOCH_2CH_2CH_3$, $CH_2OCOCH(CH_3)_2$, $CH_2OCOC(CH_3)_3$, $CH_2OCOC(CH_3)_2CH_2CH_3$, $CH(CH_3)OCOCH_3$, $CH(CH_2CH_3)OCOCH_3$, $CH(CH_3)OCOC(CH_3)_3$, $CH(CH_3)OCO_2CH_3$, $CH(CH_3)OCO_2CH_2CH_3$, $CH(CH_3)OCO_2CH(CH_3)_2$, $CH_2OCO_2CH_3$.

**4.** Verbindung I nach Anspruch 1, dadurch gekennzeichnet, daß R(2) die Bedeutung von $NH_2$, NHR(a)R(b), $NHCO(C_1-C_4)$-Alkyl, $NHCOC_6H_5$, NHCO-Naphthyl hat.

**5.** Verbindung I nach Anspruch 1, dadurch gekennzeichnet, daß die Stereochemie 5R,6S ist.

**6.** Verfahren zur Herstellung von Verbindungen der Formel I nach Anspruch 1 und ihrer pharmazeutisch verträglichen Salze,

dadurch gekennzeichnet, daß man

a) eine Verbindung der Formel II herstellt, wobei R(1), R(2) und X wie in Anspruch 1 definiert sind, falls erforderlich, NH- und OH-Gruppen mit Schutzgruppen substituiert sind und R(5) eine Carboxyschutzgruppe darstellt,

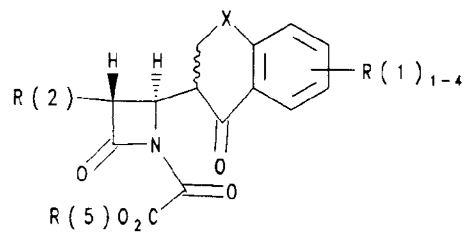

II

die Verbindungen der Formel II mit Alkanphosphonigsäureestern zyklisiert, die Schutzgruppen abspaltet und,

falls erforderlich, die erhaltenen Produkte in pharmazeutisch verträgliche Salze überführt,

oder daß man

b) eine Verbindung der Formel III herstellt, wobei R(1), R(2) und X wie in Anspruch 1 definiert sind, falls erforderlich, NH- und OH-Gruppen mit Schutzgruppen substituiert sind und R(5) eine Carboxyschutzgruppe oder eine der unter R(3) aufgeführten Gruppen darstellt,

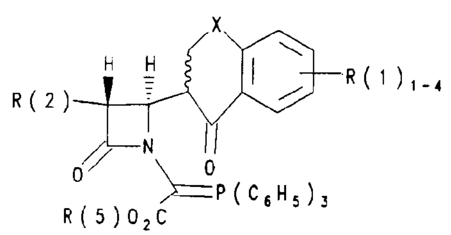

III

die Verbindungen der Formel III durch Erhitzen zyklisiert,

falls erforderlich, die Schutzgruppen abspaltet und,

falls erforderlich, die erhaltenen Produkte I in pharmazeutisch verträgliche Salze überführt,

oder daß man

c) die nach Verfahrensvariante a) und b) erhaltenen Verbindungen der Formel I, in der R(3) für ein Wasserstoffatom steht, in die Ester mit den unter R(3) angegebenen Gruppen überführt.

**7.** Verwendung einer Verbindung I nach Anspruch 1 zum Herstellen eines Medikaments zum Behandeln von Infektionskrankheiten.

**8.** Verfahren zum Behandeln von Infektionskrankheiten, dadurch gekennzeichnet, daß man eine wirksame Menge einer Verbindung I nach Anspruch 1 mit pharmazeutisch üblichen Zuschlagstoffen versieht und der zu behandelnden Person appliziert.

**9.** Mittel zum Behandeln einer Infektionskrankheit, dadurch gekennzeichnet, daß es eine wirksame Menge einer Verbindung I nach Anspruch 1 enthält.

**Patentanprüche für folgende Vertragsstaaten: ES, GR**

1. Verfahren zur Herstellung von Verbindungen der Formel I

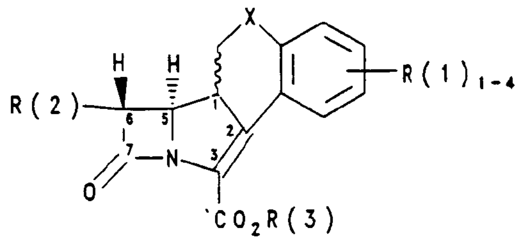

I

worin bedeuten:

X: $(CH_2)_n$ mit n = 0, 1, 2;

CR(a)R(b), wobei R(a) und R(b) voneinander unabhängig aus den folgenden Gruppen ausgewählt werden können: H; $(C_1\text{-}C_6)$-Alkyl; Aryl, ausgewählt aus der Gruppe unsubstituiertes und durch $(C_1\text{-}C_4)$-Alkyl, F, Cl, Br, $O(C_1\text{-}C_4)$-Alkyl, OH, $OCO(C_1\text{-}C_4)$-Alkyl, $NH_2$, $NH(C_1\text{-}C_4)$-Alkyl, $OCOC_6H_5$, $NHC_6H_5$ substituiertes Phenyl und Naphthyl; Heteroaryl, ausgewählt aus der Gruppe unsubstituierter oder durch $(C_1\text{-}C_4)$-Alkyl, F, Cl, Br, $O(C_1\text{-}C_4)$-Alkyl, OH, $OCO(C_1\text{-}C_4)$-Alkyl, $NH_2$, $NH(C_1\text{-}C_4)$-Alkyl, $OCOC_6H_5$, $NHC_6H_5$ substituierter 5- bis 6-gliedriger Ring mit 1 bis 4 N-, O-, S-Atomen;

O;

$SO_n$ mit n = 0, 1, 2;

NR(c), wobei R(c) aus der Gruppe bestehend aus H, $(C_1\text{-}C_6)$-Alkyl, Aryl, $CO(C_1\text{-}C_6)$-Alkyl, CO-Aryl, CO-Heteroaryl, $(C_1\text{-}C_6)$-Alkoxycarbonyl, $(C_1\text{-}C_6)$-Alkylsulfonyl und Arylsulfonyl ausgewählt ist,

R(1) : bis zu vier Substituenten, die gleich oder verschieden sind, ausgewählt aus der Gruppe, bestehend aus H, $(C_1\text{-}C_6)$-Alkyl,

ein Substituent aus der Gruppe bestehend aus Aryl, Heteroaryl, OH, SH, $SO_n(C_1\text{-}C_6)$-Alkyl (mit n = 0, 1, 2), NR(b)R(c), (wobei R(b) und R(c) wie oben definiert sind), CN, $NO_2$, C(R(a)) = NOR(b), (wobei R(a) und R(b) wie oben definiert sind),

bis zu zwei Substituenten aus der Gruppe $CF_3$, F, Cl, Br, I, $O(C_1\text{-}C_6)$-Alkyl, $OCO(C_1\text{-}C_6)$-Alkyl, OCONR(d)R(e), (wobei R(d) und R(e) voneinander unabhängig aus den folgenden Gruppen ausgewählt werden: Wasserstoff, $(C_1\text{-}C_6)$-Alkyl, NR(d)R(e) kann auch einem 5- oder 6-gliedrigen Ringsystem entsprechen), $SO_2NR(d)R(e)$, (wobei R(d) und R(e) wie oben definiert sind), $CO(C_1\text{-}C_6)$-Alkyl, COAryl, $CO_2H$, $CO_2(C_1\text{-}C_6)$-Alkyl, CONR(d)R(e), (wobei R(d) und R(e) wie oben definiert sind), $CH_2R(f)$, (wobei R(f) aus den folgenden Gruppen ausgewählt ist: Hydroxy, $(C_1\text{-}C_6)$-Alkoxy, Acyloxy, Aryloxy, Heteroaryloxy, $(C_1\text{-}C_6)$-Alkylthio, Arylthio, Heteroarylthio und die daraus ableitbaren Sulfinyl- und Sulfonylverbindungen, NR(b)R(c), wobei R(b) und R(c) wie oben definiert sind. NR(b)R(c) kann zusätzlich Teil eines zyklischen und heterozyklischen Systems sein);

$NHCO(C_1\text{-}C_6)$-Alkyl; $NHCOC_6H_5$, NHCO-Napthyl;

R(2): H, $(C_1\text{-}C_4)$-Alkyl, $CH_2OH$, $CH_2OCOR(a)$, $CH(OH)CH_3$, $CH(OCOR(a))CH_3$, $CH_2NR(b)R(c)$, $CH(NR(b)R(c))CH_3$, $C(CH_3)$=NR(a), $CH[^{\oplus}NR(g)R(h)R(i)]CH_3$, wobei R(a), R(b) und R(c) wie unter R(1) definiert sind und R(g), R(h) und R(i) voneinander unabhängig sind und $(C_1\text{-}C_6)$-Alkylgruppen darstellen; $NH_2$; NHR(a)R(b); $NHCO(C_1\text{-}C_6)$-Alkyl; $NHCOC_6H_5$, NHCO-Napthyl;

R (3): H, $(C_1\text{-}C_3)$-Alkyl-$OCO(C_1\text{-}C_6)$-Alkyl, $(C_1\text{-}C_3)$-Alkyl-$OCO_2(C_1\text{-}C_6)$-Alkyl, 5-Methyl-1,3-dioxolen-2-on-4-yl-methyl,

und ihrer pharmazeutisch verträglichen Salze, dadurch gekennzeichnet, daß man

a) eine Verbindung der Formel II herstellt, wobei R(1), R(2) und X wie in Anspruch 1 definiert sind, falls erforderlich, NH- und OH-Gruppen mit Schutzgruppen substituiert sind und R(5) eine Carboxyschutzgruppe darstellt,

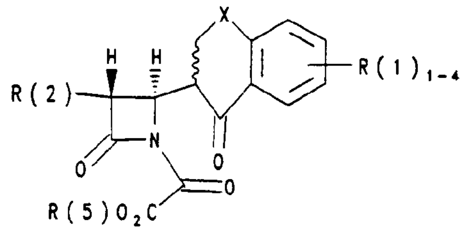

II

die Verbindungen der Formel II mit Alkanphosphonigsäureestern zyklisiert, die Schutzgruppen abspaltet und,

falls erforderlich, die erhaltenen Produkte in pharmazeutisch verträgliche Salze überführt,

oder daß man

b) eine Verbindung der Formel III herstellt, wobei R(1), R(2) und X wie in Anspruch 1 definiert sind, falls erforderlich, NH- und OH-Gruppen mit Schutzgruppen substituiert sind und R(5) eine Carboxyschutzgruppe oder eine der unter R(3) aufgeführten Gruppen darstellt,

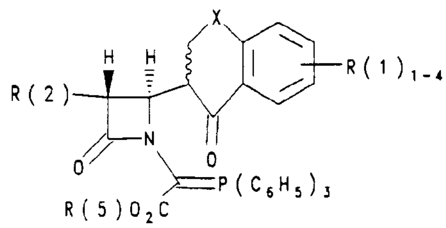

III

die Verbindungen der Formel III durch Erhitzen zyklisiert,

falls erforderlich, die Schutzgruppen abspälten und,

falls erforderlich, die erhaltenen Produkte I in pharmazeutisch verträgliche Salze überführt,

oder daß man

c) die nach Verfahrensvariante a) und b) erhaltenen Verbindungen der Formel I, in der R(3) für ein Wasserstoff steht, in die Ester mit den unter R(3) angegebenen Gruppen überführt.

2. Verfahren nach Anspruch 1, dadurch gekennzeichnet, daß dabei die Substituenten folgende Bedeutung haben:

X: $(CH_2)_n$ mit n = 0, 1, 2;

CR(a)R(b), wobei R(a) und R(b) voneinander unabhängig aus den folgenden Gruppen ausgewählt werden können: H; ($C_1$-$C_6$)-Alkyl; Aryl, ausgewählt aus der Gruppe unsubstituiertes und durch ($C_1$-$C_4$)-Alkyl, F, Cl, Br, O($C_1$-$C_4$)-Alkyl, OH, OCO($C_1$-$C_4$)-Alkyl, $NH_2$, NH($C_1$-$C_4$)-Alkyl, $OCOC_6H_5$, $NHC_6H_5$ substituiertes Phenyl und Naphthyl; Heteroaryl, ausgewählt aus der Gruppe unsubstituierter oder durch ($C_1$-$C_4$)-Alkyl, F, Cl, Br, O($C_1$-$C_4$)-Alkyl, OH, OCO($C_1$-$C_4$)-Alkyl, $NH_2$, NH($C_1$-$C_4$)-Alkyl, $OCOC_6H_5$, $NHC_6H_5$ substituierter 5- bis 6-gliedriger Ring mit 1 bis 4 N-, O-, S-Atomen;

O;

$SO_n$ mit n = 0, 1, 2;

NR(c), wobei R(c) aus der Gruppe bestehend aus H, ($C_1$-$C_6$)-Alkyl, Aryl, CO($C_1$-$C_6$)-Alkyl, CO-Aryl, CO-Heteroaryl, ($C_1$-$C_6$)-Alkoxycarbonyl, ($C_1$-$C_6$)-Alkylsulfonyl und Arylsulfonyl ausgewählt ist,

R(1) : bis zu vier Substituenten, die gleich oder verschieden sind, ausgewählt aus der Gruppe, bestehend aus H, ($C_1$-$C_6$)-Alkyl,

ein Substituent aus der Gruppe bestehend aus Aryl, Heteroaryl, OH, SH, $SO_n$($C_1$-$C_6$)-Alkyl (mit n = 0, 1, 2), NR(b)R(c), (wobei R(b) und R(c) wie oben definiert sind), CN, $NO_2$, C(R(a)) = NOR(b), (wobei R(a) und R(b) wie oben definiert sind),

bis zu zwei Substituenten aus der Gruppe $CF_3$, F, Cl, Br, I, O($C_1$-$C_6$)-Alkyl, OCO($C_1$-$C_6$)-Alkyl, OCONR(d)R(e), (wobei R(d) und R(e) voneinander unabhängig aus den folgenden Gruppen ausgewählt werden: Wasserstoff, ($C_1$-$C_6$)-Alkyl, NR(d)R(e) kann auch einem 5- oder 6-gliedrigen Ringsystem entsprechen), $SO_2$NR(d)R(e), (wobei R(d) und R(e) wie oben definiert sind), CO($C_1$-$C_6$)-Alkyl, COAryl, $CO_2H$, $CO_2$($C_1$-$C_6$)-Alkyl, CONR(d)R(e), (wobei R(d) und R(e) wie oben definiert sind), $CH_2$R(f), (wobei R(f) aus den folgenden Gruppen ausgewählt ist: Hydroxy, ($C_1$-$C_6$)-Alkoxy, Acyloxy, Aryloxy, Heteroaryloxy, ($C_1$-$C_6$)-Alkylthio, Arylthio, Heteroarylthio und die daraus ableitbaren Sulfinyl- und Sulfonylverbindungen,

NR(b)R(c), wobei R(b) und R(c) wie oben definiert sind. NR(b)R(c) kann zusätzlich Teil eines zyklischen und heterozyklischen Systems sein);
NHCO($C_1$-$C_6$)-Alkyl; $NHCOC_6H_5$, NHCO-Napthyl;
R(2): H, ($C_1$-$C_4$)-Alkyl, $CH_2OH$, $CH_2OCOR(a)$, $CH(OH)CH_3$, $CH(OCOR(a))CH_3$, $CH_2NR(b)R(c)$, $CH(NR(b)R(c))CH_3$, $C(CH_3) = NR(a)$, $CH[^{\oplus}NR(g)R(h)R(i)]CH_3$, wobei R(a), R(b) und R(c) wie unter R(1) definiert sind und R(g), R(h) und R(i) voneinander unabhängig sind und ($C_1$-$C_6$)-Alkylgruppen darstellen; $NH_2$; NHR(a)R(b); NHCO($C_1$-$C_6$)-Alkyl; $NHCOC_6H_5$, NHCO-Napthyl;
R(3): H, ($C_1$-$C_3$)-Alkyl-OCO ($C_1$-$C_6$)-Alkyl, ($C_1$-$C_3$)-Alkyl-$OCO_2$($C_1$-$C_6$)-Alkyl, 5-Methyl-1,3-dioxolen-2-on-4-yl-methyl,
wobei jedoch R(1) nicht vier Mal Wasserstoff ist.

3. Verfahren nach Anspruch 1, dadurch gekennzeichnet, daß die Substituenten folgende Bedeutungen haben:
X: $CH_2$; $C(CH_3)_2$; CH-Phenyl; O; $SO_n$ mit n = 0, 1, 2; $NSO_2CH_3$; $NSO_2$-$C_6H_4$-$CH_3$; wovon $CH_2$ und $SO_n$ mit n = 0, 1, 2 besonders bevorzugt sind.
R(1): H, $CH_3$, $C_6H_5$,

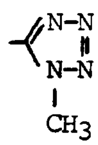 ,

F, Cl, Br, $OCH_3$, OH, $OCOCH_3$,
OCONH-$C_6H_5$, $NH_2$, $NHCOCH_3$, $NHSO_2CH_3$, $NHSO_2$-$C_6H_4$-$CH_3$, $NHCOCH_2NH_2$, CN, $COCH_3$, $C(CH_3)$=NOH, $CO_2H$, $CO_2CH_3$, $CONH_2$,
$CON(CH_3)_2$,

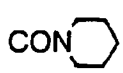 , 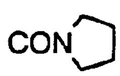, 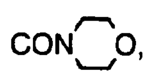, 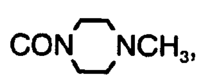,

$CONHCH_2CO_2H$, $CH_2OCOCH_3$, $CH_2SCH_2CH_3$, $CH_2SCH_2CH_2NH_2$, $CH_2SCH_2CH_2NHCH(=NH)$, $CH_2SC_6H_5$,
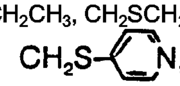,

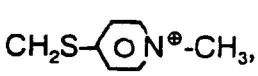,

$CH_2N(CH_3)_2$, $CH_2NHCOCH_2NH_2$,

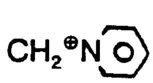 ,

$CH_2OH$,
R(2): H, $CH_3$, $CH_2CH_3$, $CH_2OH$, $CH(OH)CH_3$, $CH(OCOCH_3)CH_3$, $CH(OCOCH_2$-$C_6H_5)CH_3$, CH-($OCOCH_2O$-$C_6H_5$)$CH_3$, $CH(NH_2)CH_3$, $CH[N^{\oplus}(CH_3)_3]CH_3$, $CH(NHCOCH_3)CH_3$, $CH(NHSO_2CH_3)CH_3$, CH-($NHSO_2C_6H_5$)$CH_3$, $CH(NHCOCH_2NH_2)CH_3$, wovon $CH(OH)CH_3$ mit der R-Konfiguration besonders bevorzugt ist,
R(3): H, $CH_2OCOCH_3$, $CH_2OCOCH_2CH_3$, $CH_2OCOCH_2CH_2CH_3$, $CH_2OCOCH(CH_3)_2$, $CH_2OCOC(CH_3)_3$, $CH_2OCOC(CH_3)_2CH_2CH_3$, $CH(CH_3)OCOCH_3$, $CH(CH_2CH_3)OCOCH_3$, $CH(CH_3)OCOC(CH_3)_3$, $CH(CH_3)OCO_2CH_3$, $CH(CH_3)OCO_2CH_2CH_3$, $CH(CH_3)OCO_2CH(CH_3)_2$, $CH_2OCO_2CH_3$.

4. Verfahren nach Anspruch 1, dadurch gekennzeichnet, daß der Substituent R(2) $NH_2$, NHR(a)R(b), NHCO($C_1$-$C_4$)-Alkyl, $NHCOC_6H_5$, NHCO-Naphthyl bedeutet.

5. Verfahren nach Anspruch 1, dadurch gekennzeichnet, daß eine Verbindung der Stereochemie 5R,6S hergestellt wird.

6. Verwendung einer Verbindung I nach Anspruch 1 zum Herstellen eines Medikaments zum Behandeln von Infektionskrankheiten.

Europäisches Patentamt

# EUROPÄISCHER RECHERCHENBERICHT

Nummer der Anmeldung

EP 92 10 8792

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| X | EP-A-0 422 596 (TAKEDA CHEMICAL INDUSTRIES, LTD.)<br>* Seiten 33-35, Beispiele 8,9; Seiten 49-51, Beispiele 26-28; Ansprüche *<br>--- | 1-9 | C 07 D 477/00<br>C 07 D 491/14<br>C 07 D 495/14<br>C 07 D 471/14 //<br>A 61 K 31/40<br>(C 07 D 491/14<br>C 07 D 311:00<br>C 07 D 209:00<br>C 07 D 205:00 )<br>(C 07 D 495/14<br>C 07 D 335:00<br>C 07 D 209:00<br>C 07 D 205:00 )<br>(C 07 D 471/14<br>C 07 D 221:00<br>C 07 D 209:00<br>C 07 D 205:00 ) |
| A | EP-A-0 416 953 (GLAXO S.P.A.)<br>* Ansprüche *<br>----- | 1-9 | |
| | | | RECHERCHIERTE SACHGEBIETE (Int. Cl.5) |
| | | | C 07 D<br>A 61 K |

Der vorliegende Recherchenbericht wurde für alle Patentansprüche erstellt

| Recherchenort | Abschlußdatum der Recherche | Prüfer |
|---|---|---|
| DEN HAAG | 03-09-1992 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)